# EUROPEAN PATENT APPLICATION

(11) **EP 4 435 009 A2**
(43) Date of publication of application: **25.09.2024**
(21) Application number: 24169481.9
(22) Date of filing: 18.09.2018
(51) Int. Cl.: C07K 16/30

(54) **CLAUDIN6 ANTIBODIES AND METHODS OF TREATING CANCER**

(30) Priority: 18.09.2017 US 201762560143 P
(62) Divisional of application: 18857242.4
(71) Applicant: The Regents of the University of California, Oakland, CA 94607-5200 (US)
(72) Inventor: CONKLIN, Dylan, Woodland Hills, CA 91364 (US); SLAMON, Dennis, Woodland Hills, CA 91367 (US); O'BRIEN, Neil, Santa Monica, CA 90405 (US); PALAZZOLO, Mike, Thousand Oaks, CA 91362 (US); VON EUW, Erika, Los Angeles, CA 90066 (US)
(74) Representative: Vossius & Partner Patentanwälte Rechtsanwälte mbB

(57) **Abstract**

The present invention relates to an antigen-binding protein that binds to a human Claudin6 (CLDN6) protein (SEQ ID NO: 200) that comprises (i) HC CDR1 comprising GFTFSNYW(SEQ ID NO: 23), (ii) HC CDR2 comprising IRLKSDNYAT (SEQ ID NO: 24), (iii) HC CDR3 comprising XDGPPSGX (SEQ ID NO: 457), wherein X at position 1 is N and X at position 8 is S, T, A, C, or Y, (iv) LC CDR1 comprising ENIYSY (SEQ ID NO: 20), (v) LC CDR2 comprising NAK (SEQ ID NO: 21), and(vi) LC CDR3 comprising QHHYTVPWT (SEQ ID NO: 22).

## Description

### INCORPORATION BY REFERENCE OF MATERIAL SUBMITTED ELECTRONICALLY

Incorporated by reference in its entirety is a computer-readable nucleotide/amino acid sequence listing submitted concurrently herewith and identified as follows: 315,776 byte ASCII (Text) file named "51836_SeqListing.txt"; created on September 18, 2018.

### BACKGROUND

Antibodies constitute powerful therapeutic agents characterized by limited side effects due to their ability to specifically target a distinct antigen on a cell, bacteria, virus, or toxin. In 1986, the first therapeutic monoclonal antibody, Orthoclone OKT3, was introduced into the market. Since then, this class of biopharmaceutical products has significantly grown. In late 2014, forty-seven monoclonal antibody products had received approval in the U.S. or Europe for the treatment of a variety of diseases, including cancer and inflammatory, cardiovascular, respiratory, and infectious diseases.

More than a dozen monoclonal antibodies are currently approved by the U.S. Food and Drug Administration to treat cancers. Among these agents are alemtuzumab (Campath^{®}), which is indicated for chronic lymphocytic leukemia (CLL), and trastuzumab (Herceptin^{®}), which is used for treating breast cancer. Some antibodies are labeled with chemotherapeutic drugs, including, for example, brentuximab vedotin (Adcetris^{®}) and Ado-trastuzumab emtansine (Kadcyla^{®}). Other antibody products, such as blinatumomab (Blincyto) are designed to recognize and bind to two different antigens. Despite the commercial availability of such antibody products, the current cancer incidence and cancer deaths remain high. It has been reported that cancer incidence is greater than 450 per 100,000 men and women per year, and cancer mortality is just over 170 per 100,000 men and women per year.

### SUMMARY

Provided herein are antigen-binding proteins which bind to Claudin-6 (CLDN6). In various aspects, the antigen-binding protein of the present disclosure binds to a human CLDN6 and optionally binds to a mouse CLDN6. In various aspects, the antigen-binding protein binds to the extracellular domain (ECD) of CLDN6. In various instances, the antigen-binding protein binds to Extracellular Loop 2 (EL2) of the ECD of CLDN6. In various aspects, the antigen-binding protein binds to EL2 and does not bind to Extracellular Loop 1 (EL1) of the ECD of CLDN6. In various instances, the antigen binding protein binds to additional members of the human Claudin family, including, for example, Claudin-3 (CLDN3), Claudin-4 (CLDN4), and Claudin-9 (CLDN9). In various instances, the antigen binding protein binds to CLDN6 and at least one of CLDN4 and CLDN9. In various instances, the antigen binding protein binds to CLDN6 and does not bind to any other member of the Claudin family. In various aspects, the antigen binding protein binds to CLDN6 endogenously expressed by human ovarian cancer cells, e.g., OVCA429 cells, and exhibits an IC50 less than about 1200 nM in a FACS affinity assay with OVCA429 cells. In various instances, the antigen-binding proteins of the present disclosure inhibit tumor growth in a subject, e.g., a human, without any other moiety attached to the antigen-binding protein. In various instances, the antigen-binding proteins unconjugated to a heterologous moiety (e.g., unconjugated to any chemotherapeutic agent, drug or toxic moiety) inhibit tumor growth in a subject, e.g., a human.

In various aspects, the antigen-binding protein binds to CLDN6 expressed by human cancer cells. In various aspects, the antigen-binding protein inhibits a binding interaction between human CLDN6 and a reference anti-CLDN6 antibody. Without being bound to a particular theory, the inhibiting action of the antigen-binding proteins provided herein allow such entities to be useful in methods of reducing tumor growth and treating a subject with a tumor or cancer. As further discussed herein, in various aspects, the antigen-binding protein is an antibody, antigen-binding antibody fragment thereof, or antibody protein product.

The present disclosure also provides antigen-binding proteins comprising at least 3, 4, 5, or all amino acid sequences of a specified group of amino acid sequences. In various aspects, the antigen-binding proteins comprise at least 3, 4, 5, or 6 complementary determining region (CDR) amino acid sequences of CLDN6 antibodies disclosed herein.

The present disclosure further provides antigen-binding proteins comprising amino acid sequences as detailed herein. In various aspects, the antigen-binding protein comprises an amino acid sequence of a SEQ ID NO: listed in Table A, A1, B, B1, C, or D, or a combination thereof, as further described herein.

Related polypeptides, nucleic acids, vectors, host cells, and conjugates are further provided herein. Kits and pharmaceutical compositions comprising such entities are moreover contemplated.

Also provided are methods of making an antigen-binding protein. In various embodiments, the method comprises culturing a host cell comprising a nucleic acid encoding a antigen-binding protein or a polypeptide as described herein so as to express the antigen-binding protein or polypeptide.

Methods of treating a subject having cancer are additionally provided herein. In various embodiments, the method comprises administering to the subject the pharmaceutical composition of the present disclosure in an amount effective for treating the cancer in the subject.

Also provided are methods of treating a subject with a CLDN6-expressing cancer comprising administering to the subject a pharmaceutical composition described herein. Further contemplated is a method of inhibiting tumor growth in a subject, comprising administering to the subject a pharmaceutical composition described herein.

A method of reducing tumor size in a subject, or preventing the recurrence of cancer in a subject comprising administering to the subject a pharmaceutical composition described herein.

Also provided herein is a method of treating cancer in a subject diagnosed to be a low over-expresser of CLDN6, comprising administering to the subject a pharmaceutical composition described herein.

In various embodiments, the administering induces apoptosis in tumor cells, for example in cells expressing CLDN6. In various embodiments, the administration induces antibody-dependent cell-mediated cytotoxicity (ADCC) or Complement-dependent cytotoxicity (CDC), tumor necrosis and death or depletion of cells, and/or disruption of tumor cell adherence, each of which result tumor regression or slowing of tumor growth.

### BRIEF DESCRIPTION OF THE DRAWINGS

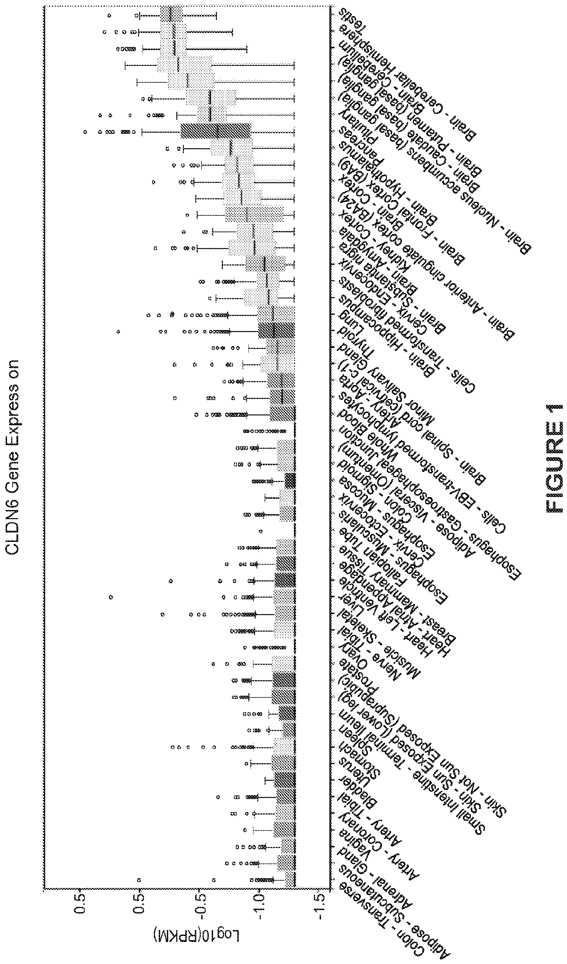

Figure 1 represents a graph of CLDN6 expression in normal (non-cancerous) tissues.

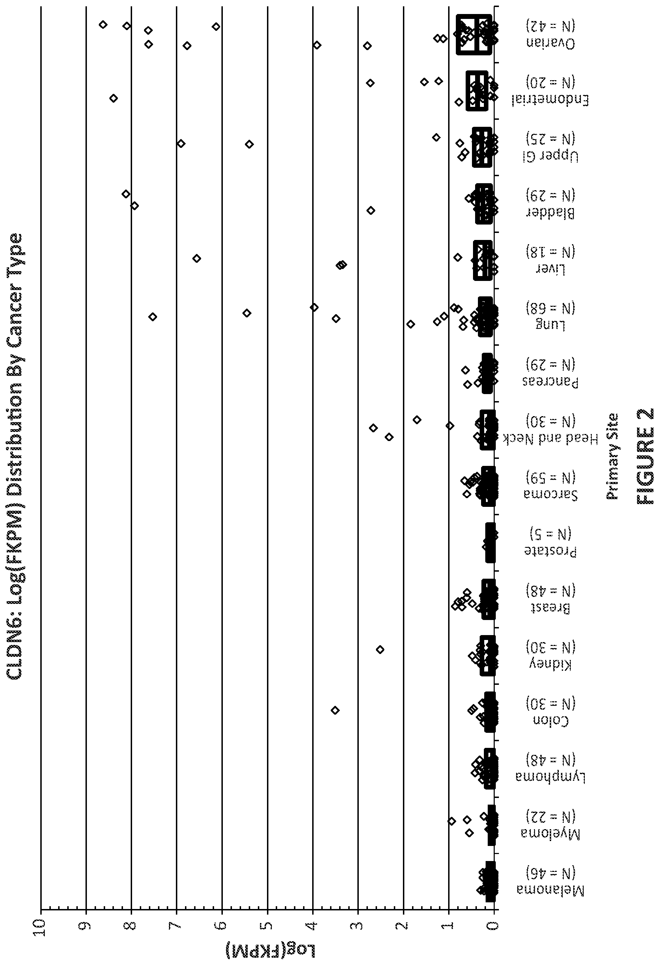

Figure 2 represents a graph of CLDN6 expression in cancer cell lines as determined by Agilent44K methodology.

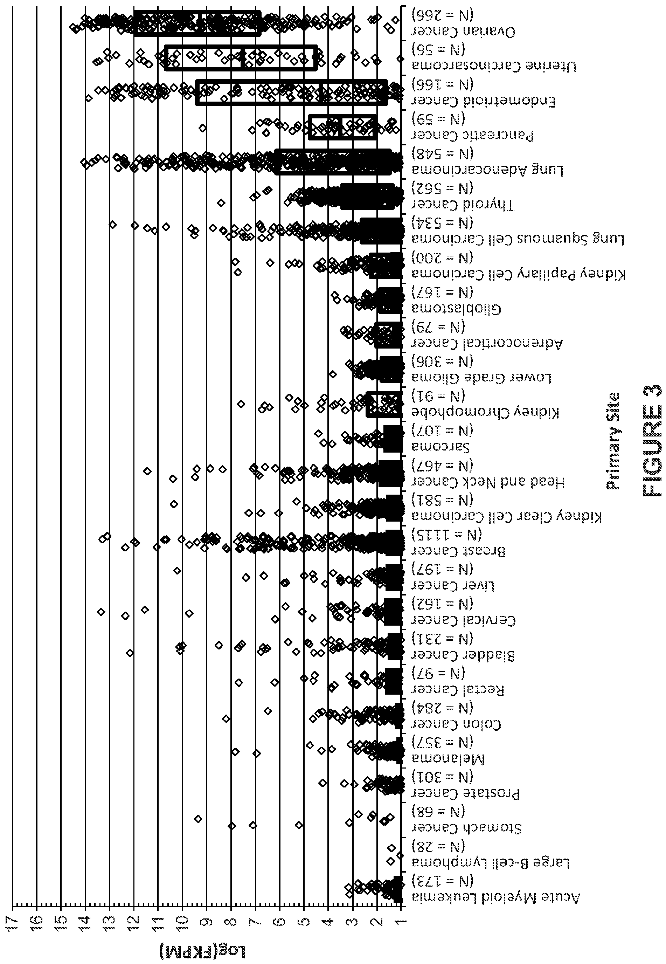

Figure 3 represents a graph of CLDN6 expression in cancer cell lines as determined by RNASeq.

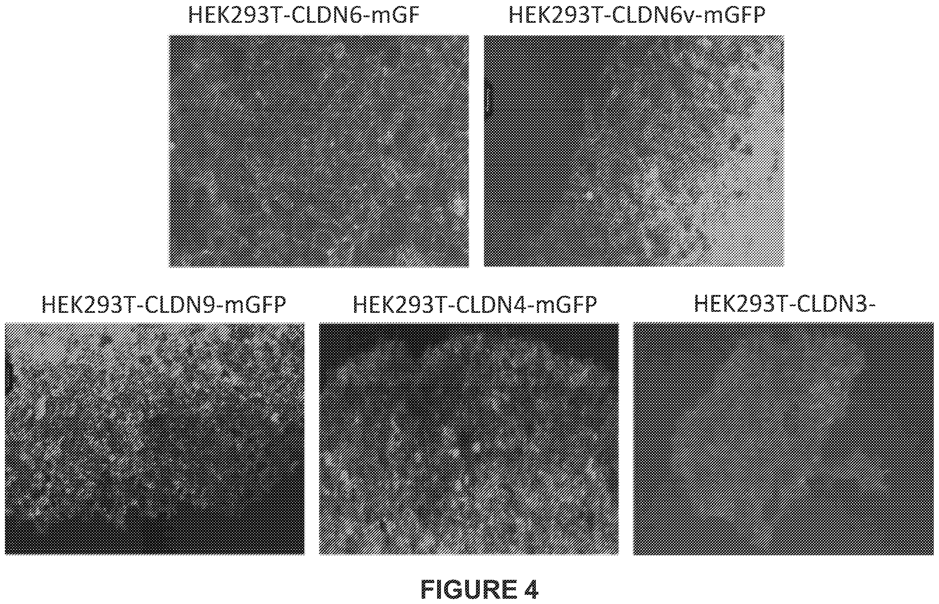

Figure 4 represents a set of fluorescent images depicting CLDN6-GFP localization in different cell models.

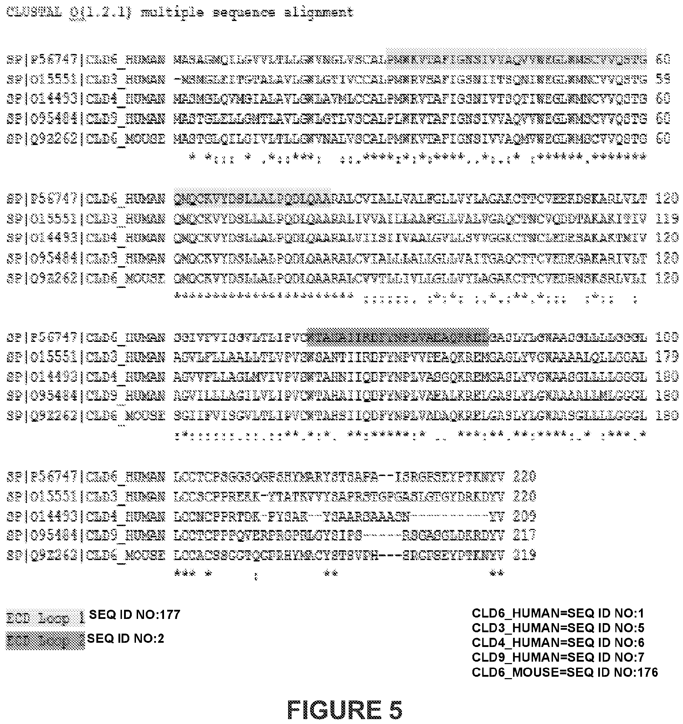

Figure 5 represents a sequence alignment of human CLDN6, human CLDN3, human CLDN4, human CLDN9, and mouse CLDN6. The sequences of the EL1 and EL2 are shown.

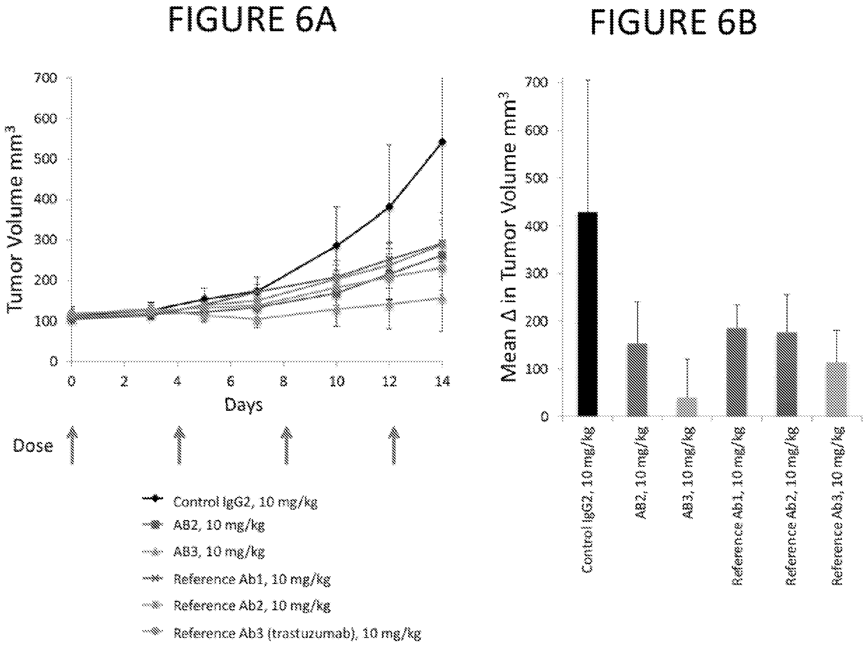

Figure 6A represents a graph of tumor volume (mm³) of tumors in mice bearing endometrial tumors as a function of time (days) after treatment with control IgG2 antibody, AB3, Reference Ab1, Reference Ab2, Reference Ab3, AB2, and AB3. Figure 6B represents a graph of the mean change in tumor volume (mm³) at Day 14 of tumors in mice bearing endometrial tumors treated with control IgG2 antibody, AB3, Reference Ab1, Reference Ab2, Reference Ab3, AB2, or AB3.

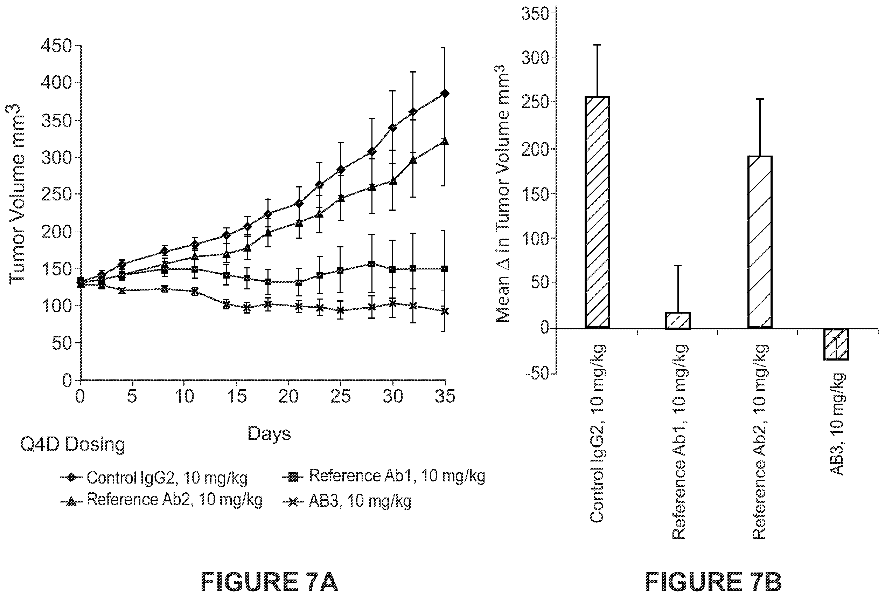

Figure 7A represents a graph of tumor volume (mm³) of tumors in mice bearing bladder tumors as a function of time (days) after treatment with control IgG2 antibody, AB3, Reference Ab1, Reference Ab2, and AB3. Figure 7B represents a graph of the mean change in tumor volume (mm³) at Day 35 of tumors in mice bearing bladder tumors treated with control IgG2 antibody, AB3, Reference Ab1, Reference Ab2, or AB3.

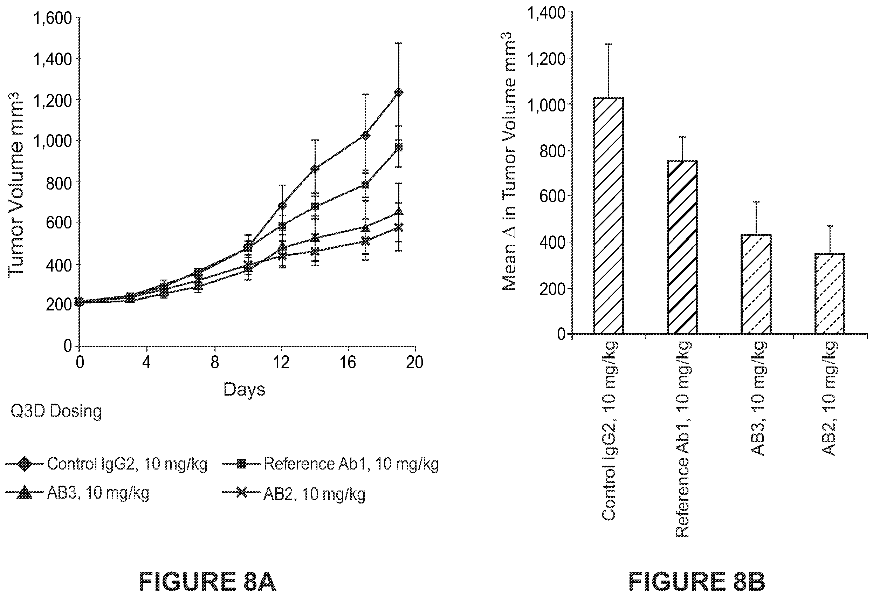

Figure 8A represents a graph of tumor volume (mm³) of tumors in mice bearing ovarian tumors as a function of time (days) after treatment with control IgG2 antibody, AB3, Reference Ab1, AB2, and AB3. Figure 8B represents a graph of the mean change in tumor volume (mm³) at Day 20 of tumors in mice bearing ovarian tumors treated with control IgG2 antibody, AB3, Reference Ab1, AB2, or AB3.

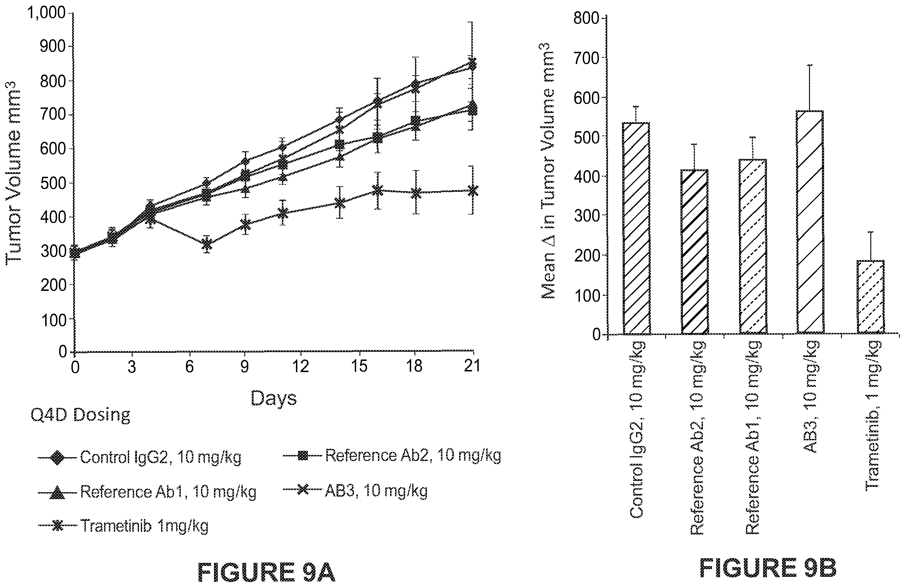

Figure 9A represents a graph of tumor volume (mm³) of tumors in mice bearing melanoma tumors as a function of time (days) after treatment with control IgG2 antibody, AB3, Reference Ab1, Reference Ab2, Reference Ab3, and AB3. Figure 9B represents a graph of the mean change in tumor volume (mm³) at Day 21 of tumors in mice bearing melanoma tumors treated with control IgG2 antibody, AB3, Reference Ab1, Reference Ab2, Reference Ab3, or AB3.

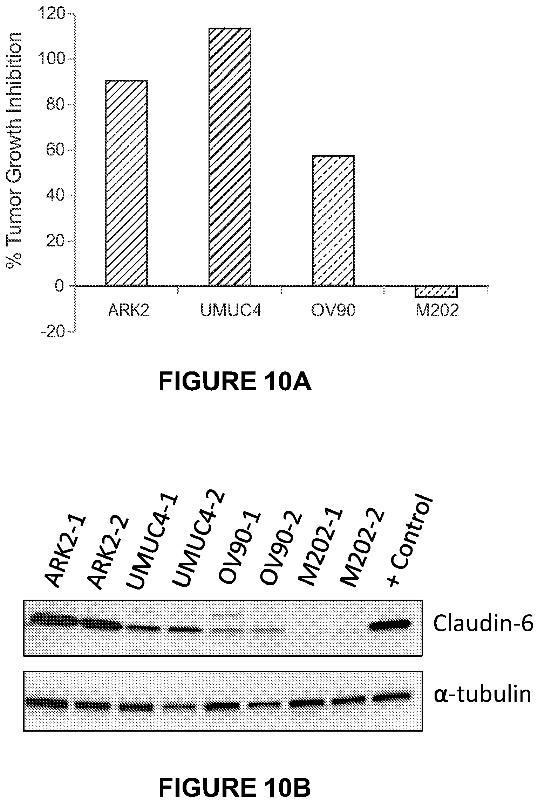

Figure 10A represents a graph of the % tumor growth inhibition achieved in tumor-bearing mice treated with AB3, relative to mice treated with control antibody. Figure 10B represents an image of a Western blot demonstrating the different levels of CLDN6 id endometrial cancer cell lines (ARK2), bladder cancer cell lines (UMUC4), ovarian cancer cell lines (OV90) and melanoma cell lines (M202) and control cells. The levels of α-tubulin were approximately the same, demonstrating equal protein loading.

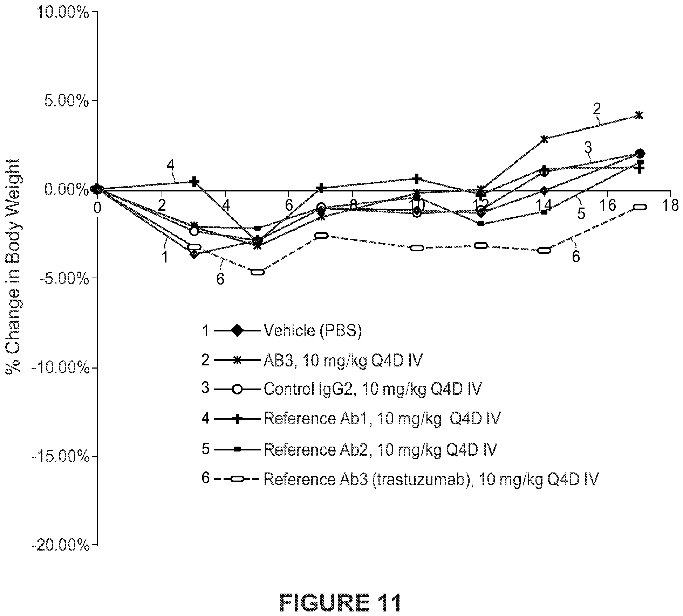

Figure 11 represents a graph of the % change in body weight of tumor-bearing mice treated with vehicle control, control antibody, Reference Ab1, Reference Ab2, Reference Ab3, and AB3 as a function of time (days).

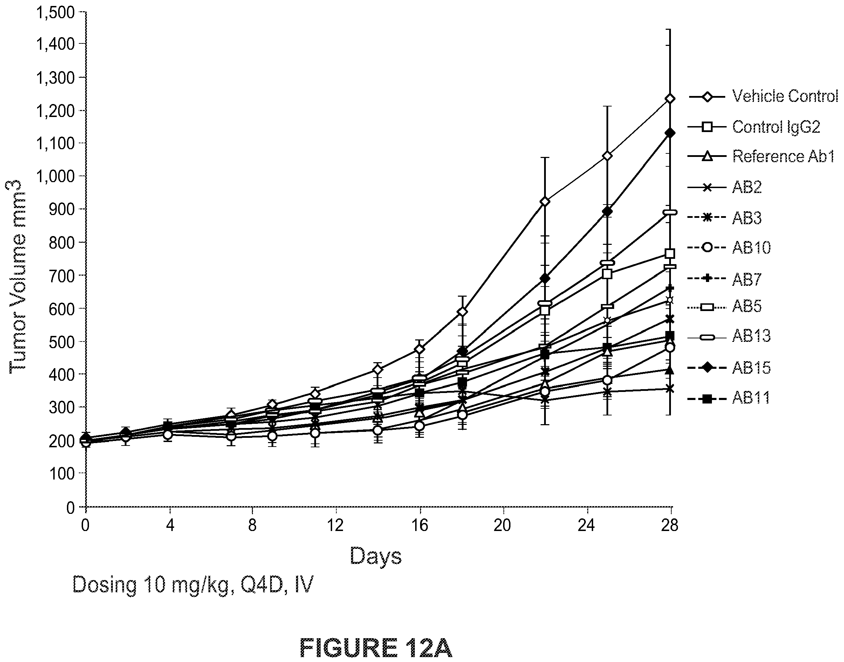

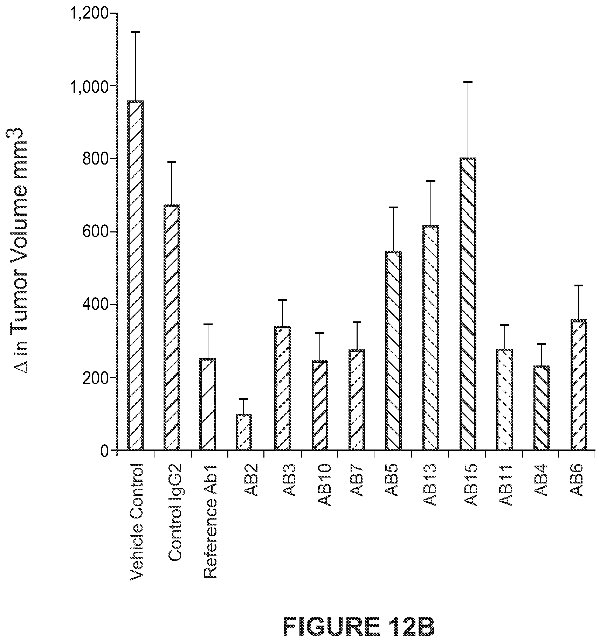

Figure 12A represents a graph of tumor volume (mm³) of tumors in mice bearing ovarian tumors as a function of time (days) after treatment with vehicle control, control IgG2 antibody, AB3, Reference Ab1, or one of the indicated anti-CLDN6 antibodies. Figure 12B represents a graph of the mean change in tumor volume (mm³) at Day 28 of tumors in mice bearing ovarian tumors treated with vehicle control, control IgG2 antibody, AB3, Reference Ab1, or one of the indicated anti-CLDN6 antibodies.

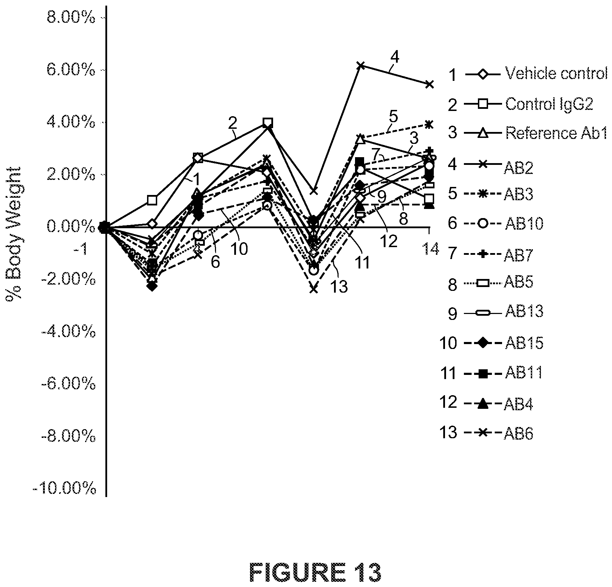

Figure 13 represents a graph of the % change in body weight of tumor-bearing mice treated with vehicle control, control antibody, Reference Ab1, and the indicated anti-CLDN6 antibodies as a function of time (days).

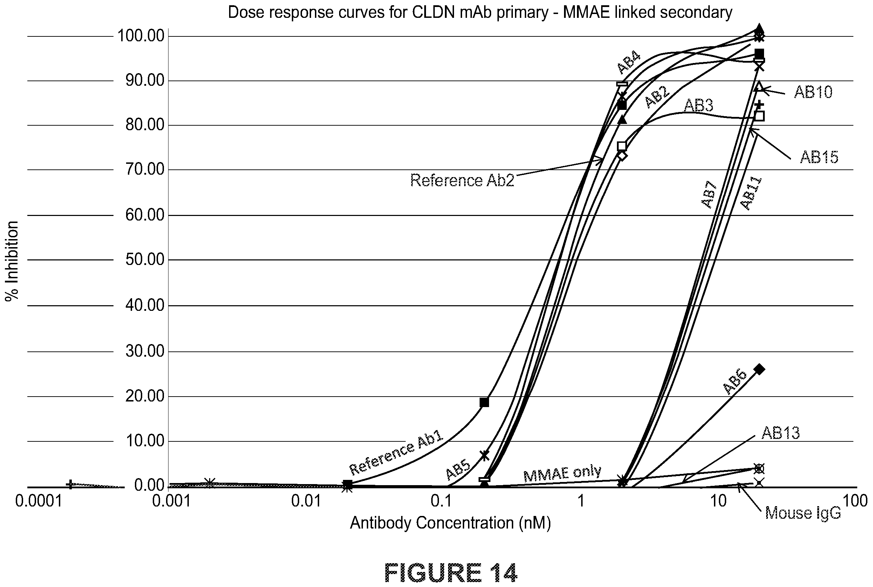

Figure 14 represents a series of dose response curves for several anti-CLDN6 antibodies of the invention and Reference Ab1 and Reference 2. Mouse IgG was used as a control.

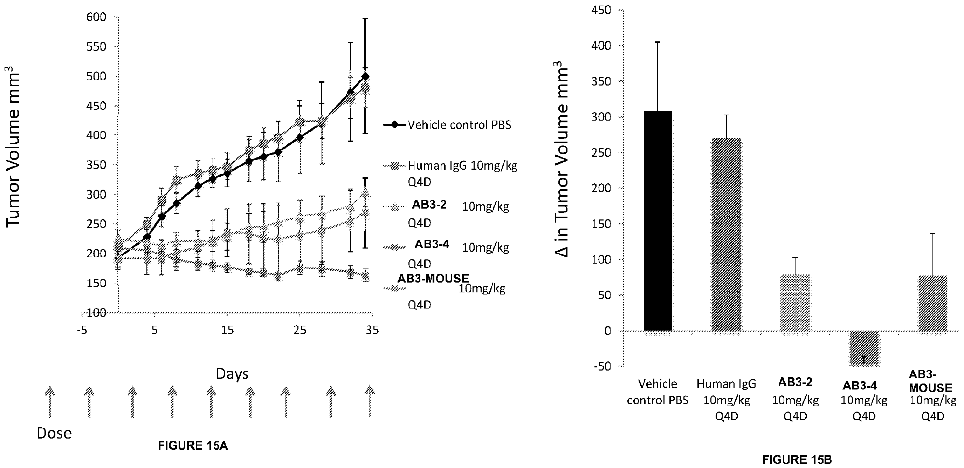

Figure 15A represents a graph of the mean change in tumor volume (mm³) at Day 35 of tumors in mice bearing bladder tumors treated with vehicle control, control IgG antibody, a mouse form of AB3, a first humanized form of AB3, and a second humanized form of AB3. Figure 15B represents a graph of the changes in tumor volume(mm³) for each of the groups in Figure 15A.

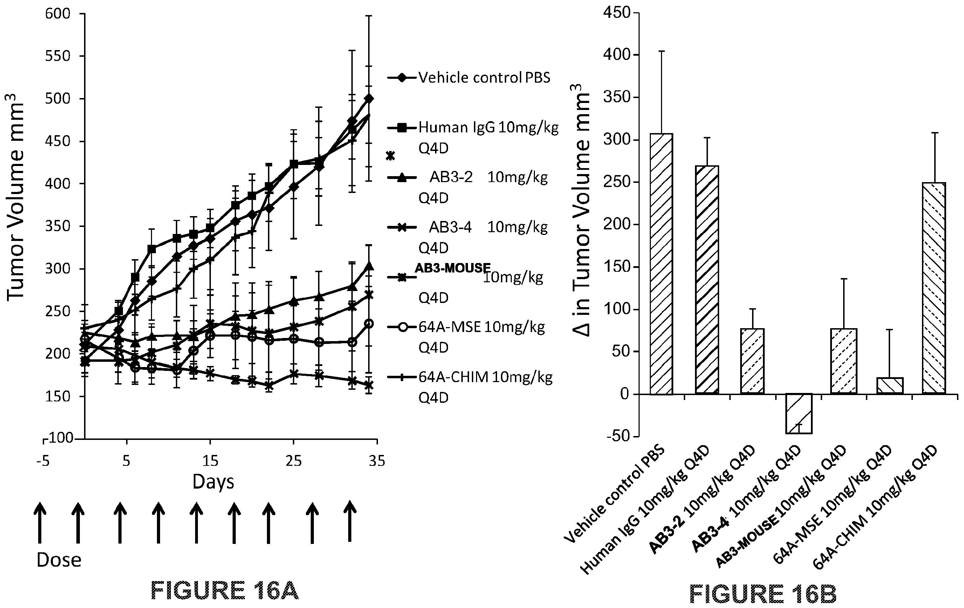

Figure 16A represents a graph of the mean change in tumor volume (mm³) at Day 35 of tumors in mice bearing bladder tumors treated with vehicle control, control IgG antibody, a mouse form of AB3, a first humanized form of AB3, and a second humanized form of AB3. Two control antibodies (one mouse and one chimeric) are also tested in this experiment. Figure 16B represents a graph of the changes in tumor volume(mm³) for each of the groups in Figure 16A.

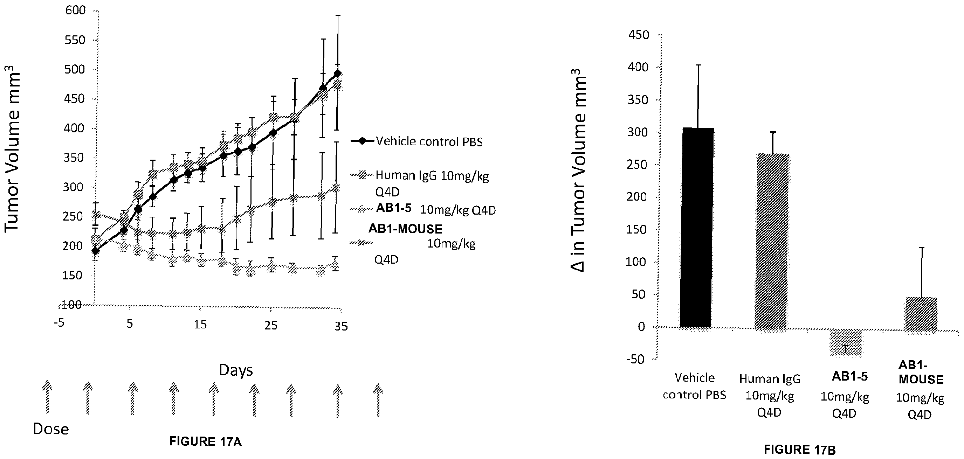

Figure 17A represents a graph of the mean change in tumor volume (mm³) at Day 35 of tumors in mice bearing bladder tumors treated with vehicle control, control IgG antibody, a mouse form of AB1, and a humanized form of AB1. Figure 17B represents a graph of the changes in tumor volume(mm³) for each of the groups in Figure 17A.

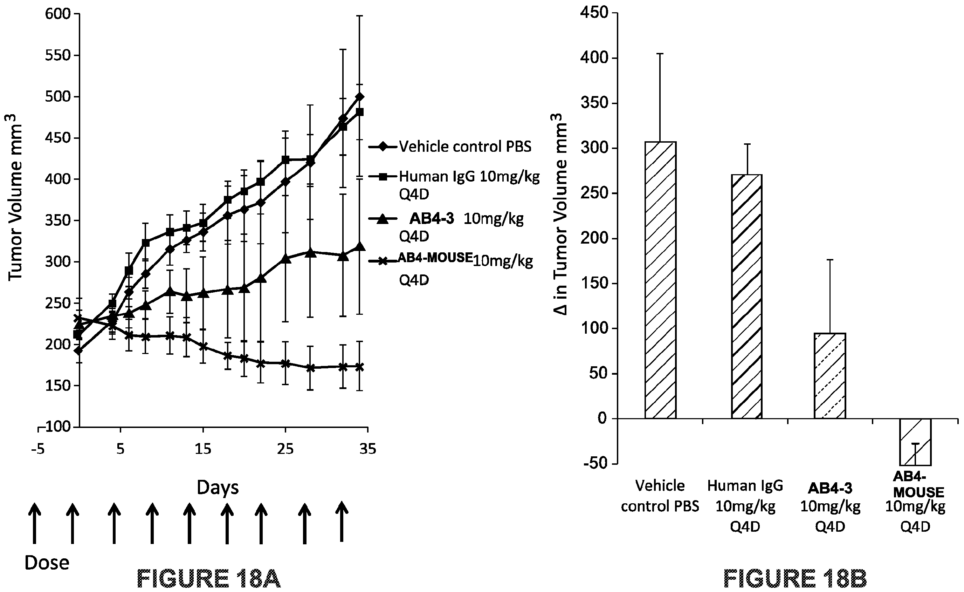

Figure 18A represents a graph of the mean change in tumor volume (mm³) at Day 35 of tumors in mice bearing bladder tumors treated with vehicle control, control IgG antibody, a mouse form of AB4, and a humanized form of AB4. Figure 18B represents a graph of the changes in tumor volume(mm³) for each of the groups in Figure 18A.

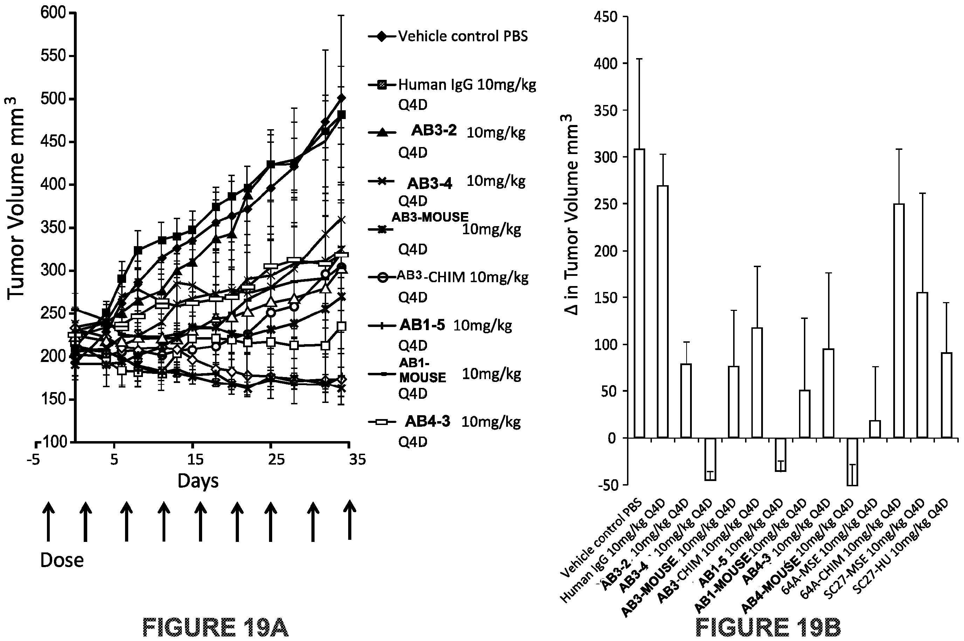

Figure 19A represents a graph of the mean change in tumor volume (mm³) at Day 35 of tumors in mice bearing bladder tumors treated with vehicle control, control IgG antibody, a mouse form of AB3, a chimeric form of AB3, a first humanized form of AB3, a second humanized form of AB3, a mouse form of Ab 1, a humanized form of AB1, a mouse form of AB4, a humanized form of AB4, and four control antibodies (one antibody have either a mouse form or a chimeric form and one antibody having a mouse or human form) are also tested in this experiment. Figure 19B represents a graph of the changes in tumor volume(mm³) for each of the groups in Figure 19A.

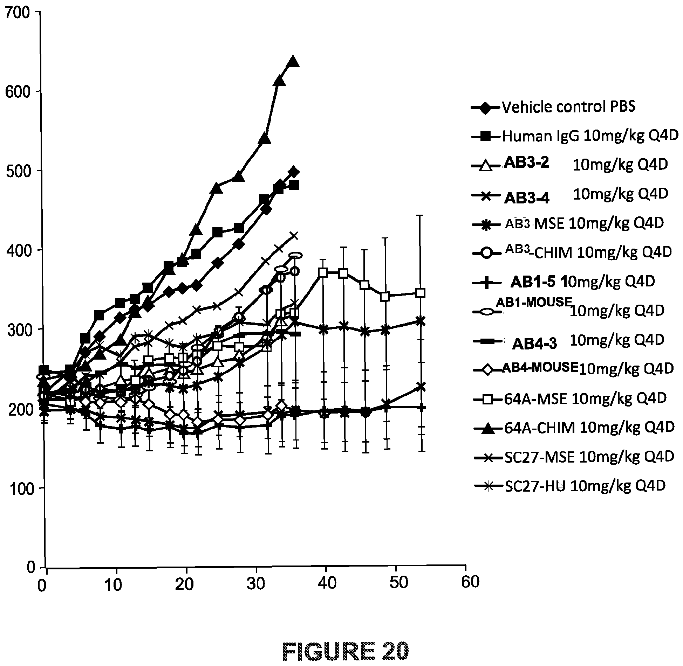

Figure 20 represents a graph of the mean change in tumor volume (mm³) at Day 55 of tumors in mice bearing bladder tumors treated as described in Figure 19A.

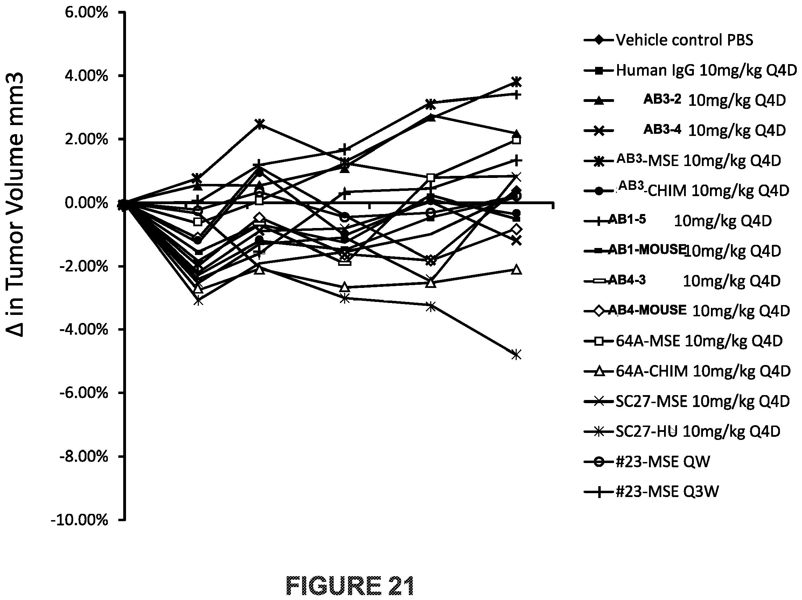

Figure 21 represents a graph of the % change in body weight of tumor-bearing mice treated at Day 32 treated as described in Figure 19A.

### DETAILED DESCRIPTION

### The Claudin Family

Tight junctions, also known as occluding junctions or zonulae occludentes, are vertebrate structures located between two adjacent cells that regulate paracellular permeability and maintain cell polarity in epithelial and endothelial cell sheets. The claudin (CLDN) family of genes encodes membrane proteins that are important components of tight junctions. CLDN proteins comprise four transmembrane (TM) helices (TM1, TM2, TM3, and TM4) and two extracellular loops (EL1 and EL2). The extracellular loops of the CLDN proteins of adjacent cells interact with one another to seal the cellular sheet and regulate paracellular transport between the luminal and basolateral spaces.

CLDN proteins play a role in various human diseases and pathologies. For example, mutations in the *CLDN1* gene have been shown to result in progressive scaling of the skin along with obstruction of bile ducts. Mutants of the *CLDN16* gene cause a magnesium wasting disorder. *CLDN19* mutations lead to ocular conditions, such as macular colobomata and myopia, while *CLDN14* mutations can lead to nonsyndromic recessive deafness. CLDN3 and CLDN4 are known to be surface receptors for the *Clostridium perfringens* enterotoxin in the gut, and CLDN1, CLDN6, and CLDN9 are co-receptors for hepatitis C virus (HCV) entry. Several CLDN proteins have been shown to be abnormally expressed in cancers. For instance, CLDN1 is downregulated in breast and colon cancer, whereas CLDN3 and CLDN4 are highly upregulated in multiple cancers.

Claudin-6 (CLDN6) is a member of the CLDN family. The gene encoding the human CLDN6 protein is located on the p arm of human chromosome 16 at 16p13.3 and is conserved in chimpanzee, Rhesus monkey, dog, cow, mouse, rat, zebrafish, and frog. CLDN6 is generally expressed in humans as a 220-amino acid precursor protein; the first 21 amino acids of which constitute the signal peptide. The amino acid sequence of the CLDN6 precursor protein is publically available at the National Center for Biotechnology Information (NCBI) website as NCBI Reference Sequence NP_067018.2 and is provided herein as SEQ ID NO: 1. The amino acid at position 143 of SEQ ID NO: 1 is Ile. In some instances, due to a single-nucleotide polymorphism (SNP) in the DNA sequence encoding CLDN6, the amino acid at position 143 is a Val. The amino acid sequence of human CLDN6 having a Val at position 143 is provided herein as SEQ ID NO: 178.

### Antigen binding proteins

Provided herein are antigen-binding proteins that bind to Claudin-6 (CLDN6). The antigen-binding proteins of the present disclosure can take any one of many forms of antigen-binding proteins known in the art. In various embodiments, the antigen-binding proteins of the present disclosure take the form of an antibody, or antigen-binding antibody fragment, or an antibody protein product.

In various embodiments of the present disclosure, the antigen-binding protein comprises, consists essentially of, or consists of an antibody. As used herein, the term "antibody" refers to a protein having a conventional immunoglobulin format, comprising heavy and light chains, and comprising variable and constant regions. For example, an antibody may be an IgG which is a "Y-shaped" structure of two identical pairs of polypeptide chains, each pair having one "light" (typically having a molecular weight of about 25 kDa) and one "heavy" chain (typically having a molecular weight of about 50-70 kDa). An antibody has a variable region and a constant region. In IgG formats, the variable region is generally about 100-110 or more amino acids, comprises three complementarity determining regions (CDRs), is primarily responsible for antigen recognition, and substantially varies among other antibodies that bind to different antigens. The constant region allows the antibody to recruit cells and molecules of the immune system. The variable region is made of the N-terminal regions of each light chain and heavy chain, while the constant region is made of the C-terminal portions of each of the heavy and light chains. (Janeway et al., "Structure of the Antibody Molecule and the Immunoglobulin Genes", Immunobiology: The Immune System in Health and Disease, 4th ed. Elsevier Science Ltd./Garland Publishing, (1999)).

The general structure and properties of CDRs of antibodies have been described in the art. Briefly, in an antibody scaffold, the CDRs are embedded within a framework in the heavy and light chain variable region where they constitute the regions largely responsible for antigen binding and recognition. A variable region typically comprises at least three heavy or light chain CDRs (Kabat et al., 1991, Sequences of Proteins of Immunological Interest, Public Health Service N.I.H., Bethesda, Md.; see also Chothia and Lesk, 1987, J. Mol. Biol. 196:901-917; Chothia et al., 1989, Nature 342: 877-883), within a framework region (designated framework regions 1-4, FR1, FR2, FR3, and FR4, by Kabat et al., 1991; see also Chothia and Lesk, 1987, supra).

Antibodies can comprise any constant region known in the art. Human light chains are classified as kappa and lambda light chains. Heavy chains are classified as mu, delta, gamma, alpha, or epsilon, and define the antibody's isotype as IgM, IgD, IgG, IgA, and IgE, respectively. IgG has several subclasses, including, but not limited to IgG1, IgG2, IgG3, and IgG4. IgM has subclasses, including, but not limited to, IgM1 and IgM2. Embodiments of the present disclosure include all such classes or isotypes of antibodies. The light chain constant region can be, for example, a kappa- or lambda-type light chain constant region, e.g., a human kappa- or lambda-type light chain constant region. The heavy chain constant region can be, for example, an alpha-, delta-, epsilon-, gamma-, or mu-type heavy chain constant regions, e.g., a human alpha-, delta-, epsilon-, gamma-, or mu-type heavy chain constant region. Accordingly, in various embodiments, the antibody is an antibody of isotype IgA, IgD, IgE, IgG, or IgM, including any one of IgG1, IgG2, IgG3 or IgG4. In various aspects, the antibody comprises a constant region comprising one or more amino acid modifications, relative to the naturally-occurring counterpart, in order to improve half-life/stability or to render the antibody more suitable for expression/manufacturability. In various instances, the antibody comprises a constant region wherein the C-terminal Lys residue that is present in the naturally-occurring counterpart is removed or clipped.

The antibody can be a monoclonal antibody. In some embodiments, the antibody comprises a sequence that is substantially similar to a naturally-occurring antibody produced by a mammal, e.g., mouse, rabbit, goat, horse, chicken, hamster, human, and the like. In this regard, the antibody can be considered as a mammalian antibody, e.g., a mouse antibody, rabbit antibody, goat antibody, horse antibody, chicken antibody, hamster antibody, human antibody, and the like. In certain aspects, the antigen-binding protein is an antibody, such as a human antibody. In certain aspects, the antigen-binding protein is a chimeric antibody or a humanized antibody. The term "chimeric antibody" refers to an antibody containing domains from two or more different antibodies. A chimeric antibody can, for example, contain the constant domains from one species and the variable domains from a second, or more generally, can contain stretches of amino acid sequence from at least two species. A chimeric antibody also can contain domains of two or more different antibodies within the same species. The term "humanized" when used in relation to antibodies refers to antibodies having at least CDR regions from a non-human source which are engineered to have a structure and immunological function more similar to true human antibodies than the original source antibodies. For example, humanizing can involve grafting a CDR from a non-human antibody, such as a mouse antibody, into a human antibody. Humanizing also can involve select amino acid substitutions to make a non-human sequence more similar to a human sequence. Information, including sequence information for human antibody heavy and light chain constant regions is publicly available through the Uniprot database as well as other databases well-known to those in the field of antibody engineering and production. For example, the IgG2 constant region is available from the Uniprot database as Uniprot number P01859, incorporated herein by reference.

An antibody can be cleaved into fragments by enzymes, such as, e.g., papain and pepsin. Papain cleaves an antibody to produce two Fab fragments and a single Fc fragment. Pepsin cleaves an antibody to produce a F(ab')₂ fragment and a pFc' fragment. In various aspects of the present disclosure, the antigen-binding protein of the present disclosure is an antigen-binding fragment of an antibody (a.k.a., antigen-binding antibody fragment, antigen-binding fragment, antigen-binding portion). In various instances, the antigen-binding antibody fragment is a Fab fragment or a F(ab')₂ fragment.

The architecture of antibodies has been exploited to create a growing range of alternative antibody formats that spans a molecular-weight range of at least about 12-150 kDa and has a valency (n) range from monomeric (n = 1), to dimeric (n = 2), to trimeric (n = 3), to tetrameric (n = 4), and potentially higher; such alternative antibody formats are referred to herein as "antibody protein products". Antibody protein products include those based on the full antibody structure and those that mimic antibody fragments which retain full antigen-binding capacity, e.g., scFvs, Fabs and VHH/VH (discussed below). The smallest antigen-binding fragment that retains its complete antigen binding site is the Fv fragment, which consists entirely of variable (V) regions. A soluble, flexible amino acid peptide linker is used to connect the V regions to a scFv (single chain fragment variable) fragment for stabilization of the molecule, or the constant (C) domains are added to the V regions to generate a Fab fragment [fragment, antigen-binding]. Both scFv and Fab fragments can be easily produced in host cells, e.g., prokaryotic host cells. Other antibody protein products include disulfide-bond stabilized scFv (ds-scFv), single chain Fab (scFab), as well as di- and multimeric antibody formats like dia-, tria- and tetra-bodies, or minibodies (miniAbs) that comprise different formats consisting of scFvs linked to oligomerization domains. The smallest fragments are VHH/VH of camelid heavy chain Abs as well as single domain Abs (sdAb). The building block that is most frequently used to create novel antibody formats is the single-chain variable (V)-domain antibody fragment (scFv), which comprises V domains from the heavy and light chain (VH and VL domain) linked by a peptide linker of ~15 amino acid residues. A peptibody or peptide-Fc fusion is yet another antibody protein product. The structure of a peptibody consists of a biologically active peptide grafted onto an Fc domain. Peptibodies are well-described in the art. See, e.g., Shimamoto et al., mAbs 4(5): 586-591 (2012).

Other antibody protein products include a single chain antibody (SCA); a diabody; a triabody; a tetrabody; bispecific or trispecific antibodies, and the like. Bispecific antibodies can be divided into five major classes: BsIgG, appended IgG, bispecific antibody (BsAb) fragments, bispecific fusion proteins, and BsAb conjugates. See, e.g., Spiess et al., Molecular Immunology 67(2) Part A: 97-106 (2015).

In various aspects, the antigen-binding protein of the present disclosure comprises, consists essentially of, or consists of any one of these antibody protein products. In various aspects, the antigen-binding protein of the present disclosure comprises, consists essentially of, or consists of any one of an scFv, Fab VHH/VH, Fv fragment, ds-scFv, scFab, dimeric antibody, multimeric antibody (e.g., a diabody,, triabody, tetrabody), miniAb, peptibody VHH/VH of camelid heavy chain antibody, sdAb, diabody; a triabody; a tetrabody; a bispecific or trispecific antibody, BsIgG, appended IgG, BsAb fragment, bispecific fusion protein, and BsAb conjugate.

In various instances, the antigen-binding protein of the present disclosure is an antibody protein product in monomeric form, or polymeric, oligomeric, or multimeric form. In certain embodiments in which the antibody comprises two or more distinct antigen binding regions fragments, the antibody is considered bispecific, trispecific, or multi-specific, or bivalent, trivalent, or multivalent, depending on the number of distinct epitopes that are recognized and bound by the antibody.

In various embodiments, an anti-CLDN6 antibody or antibody variant thereof is selected from the group consisting of a human antibody, a humanized antibody, a chimeric antibody, a monoclonal antibody, a recombinant antibody, an antigen-binding antibody fragment, a single chain antibody, a monomeric antibody, a diabody, a triabody, a tetrabody, a Fab fragment, an IgG1 antibody, an IgG2 antibody, an IgG3 antibody, and an IgG4 antibody.

In various aspects, the antigen-binding protein of the present disclosure is linked to a therapeutic agent. As described below, the therapeutic agent may be any known in the art, including, but not limited to, chemotherapeutic agents, cytokines and growth factors, cytotoxic agents, and the like. See *"Conjugates"* below.

### CLDN6 and Epitopes

The antigen-binding proteins of the present disclosure bind to CLDN6. In various aspects, the CLDN6 is a human CLDN6 having the amino acid sequence of:
MASAGMQILGVVLTLLGWVNGLVSCALPMWKVTAFIGNSIVVAQVVWEGLWM SCVVQSTGQMQCKVYDSLLALPQDLQAARALCVIALLVALFGLLVYLAGAKCTT CVEEKDSKARLVLTSGIVFVISGVLTLIPVCWTAHAXIRDFYNPLVAEAQKRELG ASLYLGWAASGLLLLGGGLLCCTCPSGGSQGPSHYMARYSTSAPAISRGPSEY PTKNYV, wherein X is Ile or Val (SEQ ID NO: 202).

In various aspects, the human CLDN6 comprises the amino acid sequence of any one of SEQ ID NOs: 1, 178, and 200-202.

In various aspects, the antigen-binding proteins of the present disclosure bind to an epitope within an amino acid sequence of CLDN6. In various aspects, CLDN6 is a human CLDN6 and the antigen-binding proteins of the present disclosure bind to an epitope within an amino acid sequence of human CLDN6, e.g., SEQ ID NOs: 1, 178, and 200-202. By "epitope" is meant the region of or within CLDN6 which is bound by the antigen-binding protein. In some embodiments, the epitope is a linear epitope. "Linear epitope" refers to the region of or within the CLDN6 which is bound by the antigen-binding protein and which region is composed of contiguous amino acids of the amino acid sequence of the CLDN6. The amino acids of a linear epitope are adjacent to each other in the primary structure of the CLDN6. Accordingly, a linear epitope is a fragment or portion of the amino acid sequence of the antigen, i.e., CLDN6. In other various embodiments, the epitope is a conformational or structural epitope. By "conformational epitope" or "structural epitope" is meant an epitope which is composed of amino acids which are located in close proximity to one another only when the CLDN6 is in its properly folded state. Unlike linear epitopes, the amino acids of a conformational or structural epitope are not adjacent to each other in the primary structure (i.e., amino acid sequence) of the CLDN6. A conformational or structural epitope is not made of contiguous amino acids of the amino acid sequence of the antigen (CLDN6).

In various aspects, the epitope is located within the extracellular domain (ECD) of CLDN6, e.g., human CLDN6. In various aspects, the antigen binding protein binds to Extracellular Loop 2 (EL2) of the ECD of CLDN6 having the amino acid sequence of WTAHAIIRDFYNPLVAEAQKREL (SEQ ID NO: 2). In various aspects, the epitope to which the antigen-binding protein binds is within SEQ ID NO: 2. In various aspects, the antigen-binding protein of the present disclosure binds to an N-terminal portion of SEQ ID NO: 2, e.g., TAHAIIRDFYNPL (SEQ ID NO: 3). In various aspects, the antigen-binding protein of the present disclosure binds to a C-terminal portion of SEQ ID NO: 2, e.g., LVAEAQKREL (SEQ ID NO: 4). In various instances, the antigen-binding protein of the present disclosure binds to EL2, but not to Extracellular Loop 1 (EL1) of CLDN6. In various aspects, the epitope(s) to which the antigen binding proteins of the present disclosure bind to is different from the epitope bound by an anti-CLDN6 antibody comprising a light chain variable region comprising the sequence of SEQ ID NO: 185 and a heavy chain variable region comprising the sequence of SEQ ID NO: 186. In various aspects, the epitope(s) to which the antigen binding proteins of the present disclosure bind to is different from the epitope bound by an anti-CLDN6 antibody comprising a light chain variable region comprising the sequence of SEQ ID NO: 181 and a heavy chain variable region comprising the sequence of SEQ ID NO: 182.

In various aspects, the antigen-binding proteins bind to human CLDN6 and a non-human CLDN6. In various instances, the non-human CLDN6 is a CLDN6 of chimpanzee, Rhesus monkey, dog, cow, mouse, rat, zebrafish, or frog. In various instances, the antigen-binding proteins bind to human CLDN6 and mouse CLDN6.

### Affinity and Avidity

The antigen-binding proteins provided herein bind to CLDN6 in a non-covalent and reversible manner. In various embodiments, the binding strength of the antigen-binding protein to CLDN6 may be described in terms of its affinity, a measure of the strength of interaction between the binding site of the antigen-binding protein and the epitope. In various aspects, the antigen-binding proteins provided herein have high-affinity for CLDN6 and thus will bind a greater amount of CLDN6 in a shorter period of time than low-affinity antigen-binding proteins. In various aspects, the antigen-binding protein has an equilibrium association constant, K_{A}, which is at least 10⁵ mol⁻¹, at least 10⁶ mol⁻¹, at least 10⁷ mol⁻¹, at least 10⁸ mol⁻¹, at least 10⁹ mol⁻¹, or at least 10¹⁰ mol⁻¹ or at least 10¹⁰ mol⁻¹ least 10¹⁰ mol⁻¹. As understood by the artisan of ordinary skill, K_{A} can be influenced by factors including pH, temperature and buffer composition.

In various embodiments, the binding strength of the antigen-binding protein to CLDN6 may be described in terms of its sensitivity. K_{D} is the equilibrium dissociation constant, a ratio of k_{off}/kₒₙ, between the antigen-binding protein and CLDN6. K_{D} and K_{A} are inversely related. The K_{D} value relates to the concentration of the antigen-binding protein (the amount of antigen-binding protein needed for a particular experiment) and so the lower the K_{D} value (lower concentration) the higher the affinity of the antigen-binding protein. In various aspects, the binding strength of the antigen-binding protein to CLDN6 may be described in terms of K_{D}. In various aspects, the K_{D} of the antigen-binding proteins provided herein is about 10⁻¹, about 10⁻², about 10⁻³, about 10⁴, about 10⁻⁵, about 10⁻⁶, or less. In various aspects, the K_{D} of the antigen-binding proteins provided herein is micromolar, nanomolar, picomolar or femtomolar. In various aspects, the K_{D} of the antigen-binding proteins provided herein is within a range of about 10⁻⁴ to 10⁻⁶ or 10⁻⁷ to 10⁻⁹ or 10⁻¹⁰ to 10⁻¹² or 10⁻¹³ to 10⁻¹⁵. In various aspects, the K_{D} of the antigen-binding proteins provided herein is within a range of about 1.0 x 10⁻¹² M to about 1.0 x 10⁻⁸ M. In various aspects, the K_{D} of the antigen-binding proteins is within a range of about 1.0 x 10⁻¹¹ M to about 1.0 x 10⁻⁹ M.

In various aspects, the affinity of the antigen-binding proteins are measured or ranked using a flow cytometry- or Fluorescence-Activated Cell Sorting (FACS)-based assay. Flow cytometry-based binding assays are known in the art. See, e.g., Cedeno-Arias et al., Sci Pharm 79(3): 569-581 (2011); Rathanaswami et al., Analytical Biochem 373: 52-60 (2008); and Geuijen et al., J Immunol Methods 302(1-2): 68-77 (2005). In various aspects, the affinity of the antigen-binding proteins are measured or ranked using a competition assay as described in Trikha et al., Int J Cancer 110: 326-335 (2004) and Tam et al., Circulation 98(11): 1085-1091 (1998), as well as below. See section titled *"Competition Assays"* below. In Trikh et al., cells that express the antigen were used in a radioassay. The binding of ¹²⁵ I-labeled antigen-binding protein (e.g., antibody) to the cell surface antigen is measured with the cells in suspension. In various aspects, the relative affinity of a CLDN6 antibody is determined via a FACS-based assay in which different concentrations of a CLDN6 antibody conjugated to a fluorophore are incubated with cells expressing CLDN6 and the fluorescence emitted (which is a direct measure of antibody-antigen binding) is determined. A curve plotting the fluorescence for each dose or concentration is made. The max value is the lowest concentration at which the fluorescence plateaus or reaches a maximum, which is when binding saturation occurs. Half of the max value is considered an EC50 or an IC50 and the antibody with the lowest EC50/IC50 is considered as having the highest affinity relative to other antibodies tested in the same manner. Such an assay is described herein at Example 5.

In various aspects, the IC₅₀ value, as determined in a competitive binding inhibition assay, approximates the K_{D} of the antigen-binding protein. In various instances, as discussed below, the competition assay is a FACS-based assay carried out with a reference antibody, fluorophore-conjugated secondary antibody, and cells which express CLDN6. In various aspects, the cells are genetically-engineered to overexpress CLDN6. In some aspects, the cells are HEK293T cells transduced with a viral vector to express CLDN6. In alternative aspects, the cells endogenously express CLDN6. Before the FACS-based assay is carried out, in some aspects, the cells which endogenously express CLDN6 are pre-determined as low CLDN6-expressing cells or high CLDN6-expressing cells. In some aspects, the cells are cancer or tumor cells. In various aspects, the cells are cells from a cell line, e.g., an ovarian cell line, endometrial cell line, bladder cell line, lung cell line, gastrointestinal (GI) cell line, liver cell line, lung cell line, and the like. In various aspects, the cells which endogenously express CLDN6 as selected from the group consisting of OVCA429 ovarian cells, ARK2 endometrial cells, OAW28 ovarian cells, UMUC-4 bladder cells, PEO14 ovarian cells, OV177 ovarian cells, H1693 lung cells, MKN7 upper GI cells, OV-90 ovarian cells, HUH-7 liver cells, JHOS-4 ovarian cells, H1435 lung cells, and NUGC3 upper Gl cells. In various aspects, the antigen-binding protein inhibits the binding interaction between human CLDN6 expressed by the cells and the reference antibody, which reference antibody is known to bind to CLDN6 but is not an antigen-binding protein of the present disclosure. In various instances, the antigen-binding proteins of the present disclosure compete with the reference antibody for binding to human CLDN6 and thereby reduce the amount of human CLDN6 bound to the reference antibody as determined by an *in vitro* competitive binding assay. In various aspects, the antigen-binding proteins of the present disclosure inhibit the binding interaction between human CLDN6 and the reference antibody and the inhibition is characterized by an IC₅₀. In various aspects, the antigen-binding proteins exhibit an IC₅₀ of less than about 2500 nM for inhibiting the binding interaction between human CLDN6 and the reference antibody. In various aspects, the antigen-binding proteins exhibit an IC₅₀ of less than about 2000 nM, less than about 1500 nM, less than about 1000 nM, less than about 900 nm, less than about 800 nm, less than about 700 nm, less than about 600 nm, less than about 500 nm, less than about 400 nm, less than about 300 nm, less than about 200 nm, or less than about 100 nm. In various aspects, the antigen-binding proteins exhibit an IC₅₀ of less than about 90 nM, less than about 80 nM, less than about 70 nM, less than about 60 nM, less than about 50 nM, less than about 40 nM, less than about 30 nM, less than about 20 nM, or less than about 10 nM. In various instances, the antigen binding proteins of the present disclosure compete against a reference antibody known to bind to CLDN6 (which reference antibody is different from any of the antigen-binding proteins of the present disclosure) for binding to CLDN6. See further description under *Competition assays.*

Avidity gives a measure of the overall strength of an antibody-antigen complex. It is dependent on three major parameters: affinity of the antigen-binding protein for the epitope, valency of both the antigen-binding protein and CLDN6, and structural arrangement of the parts that interact. The greater an antigen-binding protein's valency (number of antigen binding sites), the greater the amount of antigen (CLDN6) it can bind. In various aspects, the antigen-binding proteins have a strong avidity for CLDN6. In various aspects, the antigen-binding proteins are multivalent. In various aspects, the antigen-binding proteins are bivalent. In various instances, the antigen antigen-binding proteins are monovalent.

### Cross-reactivity

In various embodiments, the antigen-binding proteins of the present disclosure bind to CLDN6 and do not bind to any other member of the CLDN family, e.g., do not cross-react with any other member of the CLDN family. In various instances, the antigen-binding proteins of the present disclosure are CLDN-6 specific. In various embodiments, the antigen-binding proteins of the present disclosure have a selectivity for CLDN6 which is at least 10-fold, 5-fold, 4-fold, 3-fold, 2-fold greater than the selectivity of the antigen-binding protein for CLDN3, CLDN4, CLDN9, or a combination thereof. In various embodiments, the antigen-binding proteins of the present disclosure have a selectivity for CLDN6 which is at least 10-fold, 5-fold, 4-fold, 3-fold, 2-fold greater than the selectivity of the antigen-binding protein for each of CLDN3, CLDN4, and CLDN9. Selectivity may be based on the K_{D} exhibited by the antigen binding protein for CLDN6, or a CLDN family member, wherein the K_{D} may be determined by techniques known in the art, e.g., surface plasmon resonance, FACS-based affinity assays.

In various aspects, the antigen-binding proteins of the present disclosure bind to CLDN6 and do not bind to any of Claudin3 (CLDN3), Claudin4 (CLDN4), and Claudin9 (CLDN9). In various aspects, the antigen-binding proteins do not bind to any of CLDN3, CLDN4, and CLDN9 and exhibit an IC₅₀ of less than about 1200 nM (e.g., less than about 1000 nM, less than about 750 nM, less than about 500 nM, less than about 250 nM) in a FACS-based assay with OVCA429 cells endogenously expressing CLDN6. In various aspects, the antigen-binding proteins do not bind to any of CLDN3, CLDN4, and CLDN9 and the concentration at which 50% of binding saturation is achieved with OVCA429 cells endogenously expressing CLDN6 is less than about 1200 nM (e.g., less than about 1000 nM, less than about 750 nM, less than about 500 nM, less than about 250 nM). In various aspects, the antigen-binding proteins exhibit at least a 5-fold selectivity for CLDN 6 greater than that for CLDN3, CLDN4, and CLDN9 and the concentration at which 50% of binding saturation is achieved with OVCA429 cells endogenously expressing CLDN6 is less than about 1200 nM(e.g., less than about 1000 nM, less than about 750 nM, less than about 500 nM, less than about 250 nM). In various aspects, the antigen-binding proteins exhibit an IC50 of less than about 1200 nM (e.g., less than about 1000 nM, less than about 750 nM, less than about 500 nM, less than about 250 nM) for CLDN6 artificial and endogenous models and exhibit a greater than about 5-fold ratio separating CLDN6 IC50s from CLDN3, CLDN4 and/or CLDN9. In various instances, the antigen-binding proteins exhibit an IC50 of less than about 1200 nM (e.g., less than about 1000 nM, less than about 750 nM, less than about 500 nM, less than about 250 nM) for CLDN6 and exhibit an IC50 for any one of CLDN3, CLDN4, and CLDN9 at least 5-fold greater than the IC50.

In various embodiments, the antigen-binding proteins of the present disclosure bind to CLDN6 and cross-react with (e.g., bind to) at least one other member of the CLDN family. In various aspects, the antigen-binding proteins of the present disclosure bind to CLDN6 and one or more of CLDN3, CLDN4, and CLDN9. In various aspects, the antigen-binding proteins of the present disclosure bind to CLDN6 and CLDN4 or CLDN9, but do not bind to CLDN3. In various instances, the antigen-binding proteins of the present disclosure bind to CLDN6 and CLDN4 but binds to neither CLDN3 nor CLDN9. In various instances, the antigen-binding proteins of the present disclosure bind to CLDN6 and CLDN9 but do not bind to either CLDN3 or CLDN4.

### Competition assays

In various embodiments, the antigen-binding protein inhibits a binding interaction between human CLDN6 and a reference antibody, which reference antibody is known to bind to CLDN6 but is not an antigen-binding protein of the present disclosure. In various instances, the antigen-binding proteins of the present disclosure compete with the reference antibody for binding to human CLDN6 and thereby reduce the amount of human CLDN6 bound to the reference antibody as determined by an *in vitro* competitive binding assay. In various embodiments, the reference antibody binds to an epitope within the amino acid sequence of the extracellular domain of human CLDN6, optionally, within EL2 or EL1. In various aspects, the reference antibody comprises a light chain variable sequence encoded by SEQ ID NO: 179, and a heavy chain variable sequence encoded by SEQ ID NO: 180. In various aspects, the reference antibody comprises a light chain variable sequence of SEQ ID NO: 181, and a heavy chain variable sequence of SEQ ID NO: 182. In various aspects, the antigen-binding proteins of the present disclosure inhibit the binding interaction between human CLDN6 and the reference antibody and the inhibition is characterized by an IC₅₀. In various aspects, the antigen-binding proteins exhibit an IC₅₀ of less than about 2500 nM for inhibiting the binding interaction between human CLDN6 and the reference antibody. In various aspects, the antigen-binding proteins exhibit an IC₅₀ of less than about 2000 nM, less than about 1500 nM, less than about 1000 nM, less than about 900 nm, less than about 800 nm, less than about 700 nm, less than about 600 nm, less than about 500 nm, less than about 400 nm, less than about 300 nm, less than about 200 nm, or less than about 100 nm. In various aspects, the antigen-binding proteins exhibit an IC₅₀ of less than about 90 nM, less than about 80 nM, less than about 70 nM, less than about 60 nM, less than about 50 nM, less than about 40 nM, less than about 30 nM, less than about 20 nM, or less than about 10 nM.

In various instances, the antigen-binding proteins of the present disclosure compete with the reference antibody for binding to human CLDN6 and thereby reduce the amount of human CLDN6 bound to the reference antibody as determined by an *in vitro* competitive binding assay. In various aspects, the *in vitro* competitive binding assay is a FACS-based assay in which the fluorescence of a fluorophore-conjugated secondary antibody which binds to the Fc of the reference antibody is measured in the absence or presence of a particular amount of the antigen-binding protein of the present disclosure. Such a FACS-based assay is described herein in the EXAMPLES. In various aspects, the FACS-based assay is carried out with the reference antibody, fluorphore-conjugated secondary antibody and cells which express CLDN6. In various aspects, the cells are genetically-engineered to overexpress CLDN6. In some aspects, the cells are HEK293T cells transduced with a viral vector to express CLDN6. In alternative aspects, the cells endogenously express CLDN6. Before the FACS-based assay is carried out, in some aspects, the cells which endogenously express CLDN6 are pre-determined as low CLDN6-expressing cells or high CLDN6-expressing cells. In some aspects, the cells are cancer or tumor cells. In various aspects, the cells are cells from a cell line, e.g., an ovarian cell line, endometrial cell line, bladder cell line, lung cell line, gastrointestinal (GI) cell line, liver cell line, lung cell line, and the like. In various aspects, the cells which endogenously express CLDN6 as selected from the group consisting of OVCA429 ovarian cells, ARK2 endometrial cells, OAW28 ovarian cells, UMUC-4 bladder cells, PEO14 ovarian cells, OV177 ovarian cells, H1693 lung cells, MKN7 upper GI cells, OV-90 ovarian cells, HUH-7 liver cells, JHOS-4 ovarian cells, H1435 lung cells, and NUGC3 upper Gl cells. In various instances, the antigen binding proteins of the present disclosure bind to CLDN6 endogenously expressed by one or more of ARK2 cells, OVCA429 cells, LS513 cells, or MCF7 cells with high affinity. In various aspects, the antigen binding proteins exhibit an IC₅₀ of less than about 3000 nM as determined in a FACS-based competitive binding inhibition assay using one or more of ARK2 cells, OVCA429 cells, LS513 cells, or MCF7 cells. In various aspects, the antigen binding proteins exhibit an IC₅₀ of less than about 2500 nM, less than about 2000 nM, less than about 1750 nM, less than about 1500 nM, less than about 1250 nM, less than about 1000 nM, less than about 750 nM, or less than about 500 nM, as determined in a FACS-based competitive binding inhibition assay using one or more of ARK2 cells, OVCA429 cells, LS513 cells, or MCF7 cells. In various aspects, the antigen binding proteins exhibit an IC₅₀ of less than about 400 nM, less than about 300 nM, less than about 200 nM, less than about 100 nM, less than about 75 nM, less than about 50 nM, less than about 25 nM, or less than about 10 nM, as determined in a FACS-based competitive binding inhibition assay using one or more of ARK2 cells, OVCA429 cells, LS513 cells, or MCF7 cells.

Other binding assays, e.g., competitive binding assays or competition assays, which test the ability of an antibody to compete with a second antibody for binding to an antigen, or to an epitope thereof, are known in the art. See, e.g., Trikha et al., Int J Cancer 110: 326-335

(2004); Tam et al., Circulation 98(11): 1085-1091 (1998). U.S. Patent Application Publication No. US20140178905, Chand et al., Biologicals 46: 168-171 (2017); Liu et al., Anal Biochem 525: 89-91 (2017); and Goolia et al., J Vet Diagn Invest 29(2): 250-253 (2017). Also, other methods of comparing two antibodies are known in the art, and include, for example, surface plasmon resonance (SPR). SPR can be used to determine the binding constants of the antibody and second antibody and the two binding constants can be compared.

### Methods of Antibody Production and Related Methods

Suitable methods of making antigen-binding proteins (e.g., antibodies, antigen-binding antibody fragments, and antibody protein products) are known in the art. For instance, standard hybridoma methods for producing antibodies are described in, e.g., Harlow and Lane (eds.), Antibodies: A Laboratory Manual, CSH Press (1988), and CA. Janeway et al. (eds.), Immunobiology, 5th Ed., Garland Publishing, New York, NY (2001)). An various method of preparing CLDN6 monoclonal antibodies or the present disclosure is provided herein in EXAMPLES.

Depending on the host species, various adjuvants can be used to increase the immunological response leading to greater antibody production by the host. Such adjuvants include but are not limited to Freund's, mineral gels such as aluminum hydroxide, and surface active substances such as lysolecithin, pluronic polyols, polyanions, peptides, oil emulsions, keyhole limpet hemocyanin, and dinitrophenol. BCG (bacilli Calmette-Guerin) and Corynebacterium parvum are potentially useful human adjuvants.

Other methods of antibody production are summarized in Table 1.

**TABLE 1**

| Technique | Various references |
|---|---|
| EBV-hybridoma methods and Bacteriophage vector expression systems | Haskard and Archer, J. Immunol. Methods, 74(2), 361-67 (1984), Roder et al., Methods Enzymol., 121, 140-67 (1986), and Huse et al., Science, 246, 1275-81 (1989)). |
| methods of producing antibodies in non-human animals | U.S. Patents 5,545,806, 5,569,825, and 5,714,352, and U.S. Patent Application Publication No. 2002/0197266 |
| inducing in vivo production in the lymphocyte population or by screening recombinant immunoglobulin libraries or panels of highly specific binding reagents | Orlandi et al (Proc Natl Acad Sci 86: 3833-3837; 1989), and Winter G and Milstein C (Nature 349: 293-299, 1991). |
| methods of producing recombinant proteins | Protein production and purification" Nat Methods 5(2): 135-146 (2008). |
| Phage display | Janeway et al., supra, Huse et al., supra, and U.S. Patent 6,265,150). Related methods also are described in U.S. Patent No. 5,403,484; U.S. Patent No. 5,571,698; U.S. Patent No. 5,837,500; U.S. Patent No. 5,702,892. The techniques described in U.S. Patent No. 5,780,279; U.S. Patent No. 5,821,047; U.S. Patent No. 5,824,520; U.S. Patent No. 5,855,885; U.S. Patent No. 5,858,657; U.S. Patent No. 5,871,907; U.S. Patent No. 5,969,108; U.S. Patent No. 6,057,098; and U.S. Patent No. 6,225,447 |
| Antibodies can be produced by transgenic mice | U.S. Patent Nos. 5,545,806 and 5,569,825, and Janeway et al., supra. |

Methods of testing antibodies for the ability to bind to the epitope of CLDN6 regardless of how the antibodies are produced are known in the art and include any antibody-antigen binding assay, such as, for example, radioimmunoassay (RIA), ELISA, Western blot, immunoprecipitation, SPR, and competitive inhibition assays (see, e.g., Janeway et al., infra, and U.S. Patent Application Publication No. 2002/0197266, and the above section relating to competition assays).

### Sequences/Structure

Provided herein are antigen-binding proteins comprising (a) a heavy chain (HC) complementarity-determining region (CDR) 1 amino acid sequence set forth in Table A or a sequence selected from the group consisting of: SEQ ID NOs: 11, 17, 23, 29, 35, 41, 47, 53, 59, 65, 71, 77, 83, 89, 95, 101, 107, 113, 119, 125, and 131, or a variant sequence thereof which differs by only one or two amino acids or which has at least or about 70% (e.g., at least about 80%, at least about 85%, at least about 90%, at least about 95%) sequence identity; (b) an HC CDR2 amino acid sequence set forth in Table A or a sequence selected from the group consisting of: SEQ ID NOs: 12, 18, 24, 30, 36, 42, 48, 54, 60, 66, 72, 78, 84, 90, 86, 102, 108, 114, 120, 126, and 132, or a variant sequence thereof which differs by only one or two amino acids or which has at least or about 70% (e.g., at least about 80%, at least about 85%, at least about 90%, at least about 95%) sequence identity; (c) an HC CDR3 amino acid sequence set forth in Table A or a sequence selected from the group consisting of: SEQ ID NOs: 13, 19, 25, 31, 37, 43, 49, 55, 61, 67, 73, 79, 85, 91, 97, 103, 109, 115, 121, 127, and 133, or a variant sequence thereof which differs by only one or two amino acids or which has at least or about 70% (e.g., at least about 80%, at least about 85%, at least about 90%, at least about 95%) sequence identity; (d) a light chain (LC) CDR1 amino acid sequence set forth in Table A or a sequence selected from the group consisting of: SEQ ID NOs: 8, 14, 20, 32, 38, 44, 50, 56, 62, 68, 74, 80, 86, 92, 98, 104, 110, 116, 122, and 128, or a variant sequence thereof which differs by only one or two amino acids or which has at least or about 70% (e.g., at least about 80%, at least about 85%, at least about 90%, at least about 95%) sequence identity; (e) an LC CDR2 amino acid sequence set forth in Table A or a sequence selected from the group consisting of: SEQ ID NOs: 9, 15, 21, 27, 33, 39, 45, 51, 57, 63, 69, 75, 81, 87, 93, 99, 105, 111, 117, 123, and 129, or a variant sequence thereof which differs by only one or two amino acids or which has at least or about 70% (e.g., at least about 80%, at least about 85%, at least about 90%, at least about 95%) sequence identity; (f) an LC CDR3 amino acid sequence set forth in Table A or a sequence selected from the group consisting of: SEQ ID NOs: 10, 16, 22, 28, 34, 40, 46, 52, 58, 64, 70, 76, 82, 88, 94, 100, 106, 112, 118, 124, and 130, or a variant sequence thereof which differs by only one or two amino acids or which has at least or about 70% (e.g., at least about 80%, at least about 85%, at least about 90%, at least about 95%) sequence identity; or (g) a combination of any two or more of (a)-(f).

**TABLE A**

| | LC CDR1 | LC CDR2 | LC CDR3 | HC CDR1 | HC CDR2 | HC CDR3 |
|---|---|---|---|---|---|---|
| AB1 | 8 | 9 | 10 | 11 | 12 | 13 |
| AB2 | 14 | 15 | 16 | 17 | 18 | 19 |
| AB3 | 20 | 21 | 22 | 23 | 24 | 25 |
| AB4 | 26 | 27 | 28 | 29 | 30 | 31 |
| AB5 | 32 | 33 | 34 | 35 | 36 | 37 |
| AB6 | 38 | 39 | 40 | 41 | 42 | 43 |
| AB7 | 44 | 45 | 46 | 47 | 48 | 49 |
| AB8 | 50 | 51 | 52 | 53 | 54 | 55 |
| AB9 | 56 | 57 | 58 | 59 | 60 | 61 |
| AB10 | 62 | 63 | 64 | 65 | 66 | 67 |
| AB11 | 68 | 69 | 70 | 71 | 72 | 73 |
| AB12 | 74 | 75 | 76 | 77 | 78 | 79 |
| AB13 | 80 | 81 | 82 | 83 | 84 | 85 |
| AB14 | 86 | 87 | 88 | 89 | 90 | 91 |
| AB15 | 92 | 93 | 94 | 95 | 96 | 97 |
| AB16 | 98 | 99 | 100 | 101 | 102 | 103 |
| AB17 | 104 | 105 | 106 | 107 | 108 | 109 |
| AB18 | 110 | 111 | 112 | 113 | 114 | 115 |
| AB19 | 116 | 117 | 118 | 119 | 120 | 121 |
| AB20 | 122 | 123 | 124 | 125 | 126 | 127 |
| AB21 | 128 | 129 | 130 | 131 | 132 | 133 |

In various aspects, the antigen-binding protein comprises a LC CDR1 amino acid sequence, a LC CDR2 amino acid sequence, and a LC CDR3 amino acid sequence set forth in Table A and at least 1 or 2 of the HC CDR amino acid sequences set forth in Table A. In various aspects, the antigen-binding protein comprises a HC CDR1 amino acid sequence, a HC CDR2 amino acid sequence, and a HC CDR3 amino acid sequence set forth in Table A and at least 1 or 2 of the LC CDR amino acid sequences set forth in Table A.

In various embodiments, the antigen-binding protein comprises at least 3, 4, or 5 of the amino acid sequences designated by the SEQ ID NOs: in a single row of Table A. In various embodiments, the antigen-binding protein comprises each of the LC CDR amino acid sequences designated by the SEQ ID NOs: of a single row of Table A and at least 1 or 2 of the HC CDR amino acid sequences designated by the SEQ ID NOs: in of a single row of Table A. In various embodiments, the antigen-binding protein comprises each of the HC CDR amino acid sequences designated by the SEQ ID NOs: of a single row of Table A and at least 1 or 2 of the LC CDR amino acid sequences designated by the SEQ ID NOs: of a single row of Table A. In various embodiments, the antigen-binding protein comprises all 6 of the CDR amino acid sequences designated by the SEQ ID NOs: of a single row of Table A. In various embodiments, the antigen-binding protein comprises six CDR amino acid sequences selected from the group consisting of: (a) SEQ ID NOs: 74-79; (b) SEQ ID NOs: 50-55; (c) SEQ ID NOs: 122-127; (d) SEQ ID NOs: 26-31; (e) SEQ ID NOs: 128-133; (f) SEQ ID NOs: 38-43; (g) SEQ ID NOs: 62-67; (h) SEQ ID NOs: 80-85; (i) SEQ ID NOs: 44-49; (j) SEQ ID NOs: 86-91; (k) SEQ ID NOs: 104-109; (l) SEQ ID NOs: 56-61; (m) SEQ ID NOs: 32-37; (n) SEQ ID NOs: 110-115; (o) SEQ ID NOs: 98-103; (p) SEQ ID NOs: 92-97; (q) SEQ ID NOs: 116-121; (r) SEQ ID NOs: 8-13; (s) SEQ ID NOs: 68-73; (t) SEQ ID NOs: 14-19; and (u) SEQ ID NOs: 20-25.

In various instances, the amino acid sequences of Table A are separated by at least one or more (e.g., at least 2, 3, 4, 5, 6, 7, 8, 9, 10, or more) intervening amino acid(s). In various instances, there are about 10 to about 20 amino acids between the sequences of the LC CDR1 and the LC CDR2 and about 25 to about 40 amino acids between the sequences of the LC CDR2 and the LC CDR3. In various instances, there are about 14 to about 16 amino acids between the sequences of the LC CDR1 and the LC CDR2 and about 30 to about 35 amino acids between the sequences of LC CDR2 and the LC CDR3. In various instances, there are about 10 to about 20 amino acids between the sequences of the HC CDR1 and HC CDR2 and about 25 to about 40 amino acids between the sequences of the HC CDR2 and the HC CDR3. In various instances, there are about 14 to about 16 amino acids between the sequences of the HC CDR1 and HC CDR2 and about 30 to about 35 amino acids between the sequences of the HC CDR2 and HC CDR3.

In various embodiments, the antigen-binding protein comprises (a) a heavy chain variable region amino acid sequence set forth in in Table B or a sequence selected from the group consisting of: SEQ ID NOs: 135, 137, 139, 141, 143, 145, 147, 149, 151, 153, 155, 157, 159, 161, 163, 165, 167, 169, 171, 173, and 175, or a variant sequence thereof which differs by only one or two amino acids or which has at least or about 70% (e.g., at least about 80%, at least about 85%, at least about 90%, at least about 95%) sequence identity; or (b) a light chain variable region amino acid sequence set forth in Table B or a sequence selected from the group consisting of: SEQ ID NOs: 134, 136, 138, 140, 142, 144, 146, 148, 150, 152, 154, 156, 158, 160, 162, 164, 166, 168, 170, 172, 174, and 176, or a variant sequence thereof which differs by only one or two amino acids or which has at least or about 70% (e.g., at least about 80%, at least about 85%, at least about 90%, at least about 95%) sequence identity; or (c) both (a) and (b).

**TABLE B**

| | Light Chain Variable Region | Heavy Chain Variable Region |
|---|---|---|
| AB1 | 134 | 135 |
| AB2 | 136 | 137 |
| AB3 | 138 | 139 |
| AB4 | 140 | 141 |
| AB5 | 142 | 143 |
| AB6 | 144 | 145 |
| AB7 | 146 | 147 |
| AB8 | 148 | 149 |
| AB9 | 150 | 151 |
| AB10 | 152 | 153 |
| AB11 | 154 | 155 |
| AB12 | 156 | 157 |
| AB13 | 158 | 159 |
| AB14 | 160 | 161 |
| AB15 | 162 | 163 |
| AB16 | 164 | 165 |
| AB17 | 166 | 167 |
| AB18 | 168 | 169 |
| AB19 | 170 | 171 |
| AB20 | 172 | 173 |
| AB21 | 174 | 175 |

In various embodiments, the antigen-binding protein comprises a pair of amino acid sequences selected from the group consisting of: (a) SEQ ID NOs: 156 and 157; (b) SEQ ID NOs: 148 and 149; (c) SEQ ID NOs: 172 and 173; (d) SEQ ID NOs: 140 and 141; (e) SEQ ID NOs: 174 and 175; (f) SEQ ID NOs: 144 and 145; (g) SEQ ID NOs: 152 and 153; (h) SEQ ID NOs: 158 and 159; (i) SEQ ID NOs: 146 and 147; (j) SEQ ID NOs: 160 and 161; (k) SEQ ID NOs: 166 and 167; (l) SEQ ID NOs: 150 and 151; (m) SEQ ID NOs: 142 and 143; (n) SEQ ID NOs: 168 and 169; (o) SEQ ID NOs: 164 and 165; (p) SEQ ID NOs: 162 and 163; (q) SEQ ID NOs: 170 and 171; (r) SEQ ID NOs: 134 and 135; (s) SEQ ID NOs: 154 and 155; (t) SEQ ID NOs: 136 and 137; and (u) SEQ ID NOs: 138 and 139.

In various aspects, the antigen-binding protein does not comprise a pair of amino acid sequences encoded by the sequences of SEQ ID NOs: 179 and 180. In various aspects, the antigen-binding protein does not comprise a pair of amino acid sequences of SEQ ID NOs: 181 and 182. In various aspects, the antigen-binding protein does not comprise a pair of amino acid sequences encoded by the sequences of SEQ ID NOs: 183 and 184. In various aspects, the antigen-binding protein does not comprise a pair of amino acid sequences of SEQ ID NOs: 185 and 186.

In various aspects, the antigen-binding protein comprises an amino acid sequence which is similar to an above-referenced amino acid sequence, yet the antigen-binding protein substantially retains its biological function, e.g., its ability to bind to human CLDN6, reduce tumor growth, treat cancer.

In various aspects, the antigen-binding protein comprises an amino acid sequence which differs by only 1, 2, 3, 4, 5, 6, or more amino acids, relative to the above-referenced amino acid sequence(s). In various aspects, the antigen-binding protein comprises a variant sequence of the referenced sequence, which variant sequence differs by only one or two amino acids, relative to the referenced sequence. In various aspects, the antigen-binding protein comprising one or more amino acid substitutions that occur outside of the CDRs, e.g, the one or more amino acid substitutions occur within the framework region(s) of the heavy or light chain. In various aspects, the antigen-binding protein comprising one or more amino acid substitutions yet the antigen-binding protein retains the amino acid sequences of the six CDRs. In various aspects, the antigen-binding protein comprises an amino acid sequence having only 1, 2, 3, 4, 5, 6, or more conservative amino acid substitutions, relative to the above-referenced amino acid sequence(s). As used herein, the term "conservative amino acid substitution" refers to the substitution of one amino acid with another amino acid having similar properties, e.g., size, charge, hydrophobicity, hydrophilicity, and/or aromaticity, and includes exchanges within one of the following five groups:
I. Small aliphatic, nonpolar or slightly polar residues:
   Ala, Ser, Thr, Pro, Gly;
II. Polar, negatively charged residues and their amides and esters:
   Asp, Asn, Glu, Gln, cysteic acid and homocysteic acid;
III. Polar, positively charged residues:
   His, Arg, Lys; Ornithine (Orn)
IV. Large, aliphatic, nonpolar residues:
   Met, Leu, Ile, Val, Cys, Norleucine (Nle), homocysteine
V. Large, aromatic residues:
   Phe, Tyr, Trp, acetyl phenylalanine

In various aspects, the conservative amino acid substitution is an exchange within one of the following groups of amino acids:
I. aliphatic amino acids: Gly, Ala, Val, Leu, Ile
II. non-aromatic amino acids comprising a side chain hydroxyl: Sere Thr
III. amino acids comprising a sulfur side chain: Cys, Met
IV: amino acids comprising a side chain aromatic ring: Phe, Tyr, Trp
V: acidic amino acid: Glu; Asp
VI: basic amino acid: Arg; Lys
VII: amino acid comprising a side chain amide: Gin, Asn
VIII: amino acid comprising a side chain imidazole: His, alpha-dimethyl imidiazole acetic acid (DMIA)
IX: imino acid: Pro, 4-hydroxy-Pro, 4-amino-Pro

In various aspects, the antigen-binding protein comprises an amino acid sequence which has greater than or about 30%, greater than or about 50%, or greater than or about 70% sequence identity to the above-referenced amino acid sequence. In various aspects, the antigen-binding protein comprises an amino acid sequence which has at least 30%, at least 40%, at least 50%, at least 60%, at least 70%, at least 80%, at least 85%, at least 90% or has greater than 90% sequence identity to the above-referenced amino acid sequence. In various aspects, the antigen-binding protein comprises an amino acid sequence that has at least 70%, at least 80%, at least 85%, at least 90% or has greater than 90% sequence identity along the full-length of the above-referenced amino acid sequence. In various aspects, the antigen-binding protein comprises an amino acid sequence having at least 95%, 96%, 97%, 98% or 99% sequence identity along the full-length of the above-referenced amino acid sequence.

In various aspects, the antigen-binding protein comprises a variant sequence of the referenced sequence, which variant sequence has at least or about 70% sequence identity, relative to the above-referenced sequence. In various aspects, the antigen-binding protein comprises a variant sequence of the referenced sequence, which variant sequence has at least or about 80% sequence identity, relative to the above-referenced sequence. In various aspects, the antigen-binding protein comprises a variant sequence of the referenced sequence, which variant sequence has at least or about 90% sequence identity, relative to the above-referenced sequence. In various aspects, the antigen-binding protein comprises a variant sequence of the referenced sequence, which variant sequence has at least or about 95% sequence identity, relative to the above-referenced sequence.

In various embodiments, the antigen-binding protein comprises one, two, three, four, or five sequences of the SEQ ID NOs. in a single row of Table A and at least one variant sequence having at least or about 70% (e.g., at least about 80%, at least about 85%, at least about 90%, at least about 95%) sequence identity to any of SEQ ID NOs: 8-133. In various embodiments, the antigen-binding protein comprises one, two, three, four, or five sequences of a set of sequences selected from: (a) SEQ ID NOs: 74-79; (b) SEQ ID NOs: 50-55; (c) SEQ ID NOs: 122-127; (d) SEQ ID NOs: 26-31; (e) SEQ ID NOs: 128-133; (f) SEQ ID NOs: 38-43; (g) SEQ ID NOs: 62-67; (h) SEQ ID NOs: 80-85; (i) SEQ ID NOs: 44-49; (j) SEQ ID NOs: 86-91; (k) SEQ ID NOs: 104-109; (l) SEQ ID NOs: 56-61; (m) SEQ ID NOs: 32-37; (n) SEQ ID NOs: 110-115; (o) SEQ ID NOs: 98-103; (p) SEQ ID NOs: 92-97; (q) SEQ ID NOs: 116-121; (r) SEQ ID NOs: 8-13; (s) SEQ ID NOs: 68-73; (t) SEQ ID NOs: 14-19; and (u) SEQ ID NOs: 20-25, wherein the antigen-binding protein further comprises at least one variant sequence having at least or about 70% (e.g., at least about 80%, at least about 85%, at least about 90%, at least about 95%) sequence identity to at least one of the sequences of the set. For instance, in various aspects, the antigen-binding protein comprises four sequences of SEQ ID NOs: 74-79, namely, SEQ ID NOs: 74-77, wherein the antigen-binding protein comprises two variant sequences: one variant sequence having at least or about 70% (e.g., at least about 80%, at least about 85%, at least about 90%, at least about 95%) sequence identity to SEQ ID NO: 78 and another variant sequence having at least or about 70% (e.g., at least about 80%, at least about 85%, at least about 90%, at least about 95%) sequence identity to SEQ ID NO: 79.

In various embodiments, the antigen-binding protein comprises a pair of variant sequences having at least or about 70% (e.g., at least about 80%, at least about 85%, at least about 90%, at least about 95%) sequence identity to any of SEQ ID NOs: 134-175. In various instances, the antigen binding protein comprises a pair of variant sequences which have at least or about 70% (e.g., at least about 80%, at least about 85%, at least about 90%, at least about 95%) sequence identity to (a) SEQ ID NOs: 156 and 157; (b) SEQ ID NOs: 148 and 149; (c) SEQ ID NOs: 172 and 173; (d) SEQ ID NOs: 140 and 141; (e) SEQ ID NOs: 174 and 175; (f) SEQ ID NOs: 144 and 145; (g) SEQ ID NOs: 152 and 153; (h) SEQ ID NOs: 158 and 159; (i) SEQ ID NOs: 146 and 147; (j) SEQ ID NOs: 160 and 161; (k) SEQ ID NOs: 166 and 167; (l) SEQ ID NOs: 150 and 151; (m) SEQ ID NOs: 142 and 143; (n) SEQ ID NOs: 168 and 169; (o) SEQ ID NOs: 164 and 165; (p) SEQ ID NOs: 162 and 163; (q) SEQ ID NOs: 170 and 171; (r) SEQ ID NOs: 134 and 135; (s) SEQ ID NOs: 154 and 155; (t) SEQ ID NOs: 136 and 137; and (u) SEQ ID NOs: 138 and 139. In various embodiments, the antigen-binding protein comprises a pair of sequences: one sequence of Table B and another sequence which is a variant sequence having at least or about 70% (e.g., at least about 80%, at least about 85%, at least about 90%, at least about 95%) sequence identity to any of SEQ ID NOs: 134-175. In various embodiments, the antigen-binding protein comprises a pair of sequences: one sequence selected from (a) SEQ ID NOs: 156 and 157; (b) SEQ ID NOs: 148 and 149; (c) SEQ ID NOs: 172 and 173; (d) SEQ ID NOs: 140 and 141; (e) SEQ ID NOs: 174 and 175; (f) SEQ ID NOs: 144 and 145; (g) SEQ ID NOs: 152 and 153; (h) SEQ ID NOs: 158 and 159; (i) SEQ ID NOs: 146 and 147; (j) SEQ ID NOs: 160 and 161; (k) SEQ ID NOs: 166 and 167; (l) SEQ ID NOs: 150 and 151; (m) SEQ ID NOs: 142 and 143; (n) SEQ ID NOs: 168 and 169; (o) SEQ ID NOs: 164 and 165; (p) SEQ ID NOs: 162 and 163; (q) SEQ ID NOs: 170 and 171; (r) SEQ ID NOs: 134 and 135; (s) SEQ ID NOs: 154 and 155; (t) SEQ ID NOs: 136 and 137; and (u) SEQ ID NOs: 138 and 139, and another sequence which is a variant sequence having at least or about 70% (e.g., at least about 80%, at least about 85%, at least about 90%, at least about 95%) sequence identity to a sequence of (a) - (u). For instance, in various aspects, the antigen-binding protein comprises a sequences of SEQ ID NO: 134 and the antigen-binding protein further comprises a variant sequence having at least or about 70% (e.g., at least about 80%, at least about 85%, at least about 90%, at least about 95%) sequence identity to SEQ ID NO 135.

In various instances, the antigen-binding protein comprises an amino acid sequence of an above-referenced amino acid sequence with one or more amino acid substitutions to reduce or eliminate reactive amino acids to decrease or prevent unwanted side chain reactions. For instance, the antigen-binding protein comprises an amino acid sequence of an above-referenced amino acid sequence with one or more (i) Trp residues substituted with His, Tyr, or Phe; (ii) Asn residues substituted with Gln, Ser, Ala, or Asp; (iii) Asp residues occurring immediately before a Pro residue substituted with Ala, Ser, or Glu, (iv) Asn residues substituted with Gin, Ser, or Ala; and/or (v) Cys residues substituted with Tyr, Ser, or Ala. In various aspects, the antigen-binding protein comprises an amino acid sequence of an above-referenced amino acid sequence with an amino acid substitution predicted to have greater binding affinity, greater stability, or other positive attribute, based on SHM events or based on statistical analyses of a multitude of other similar antibody sequences. In some aspects, the antigen-binding protein comprises (a) an HC CDR1 amino acid sequence set forth in Table A1 or a sequence selected from the group consisting of: SEQ ID NOs: 452, 455, 461, 465, 71, and 472, or a variant sequence thereof which differs by only one or two amino acids or which has at least or about 70(e.g., at least about 80%, at least about 85%, at least about 90%, at least about 95%) sequence identity; (b) an HC CDR2 amino acid sequence set forth in Table A1 or a sequence selected from the group consisting of: SEQ ID NOs: 475, 456, 462, 466, 468, and 473; or a variant sequence thereof which differs by only one or two amino acids or which has at least or about 70% (e.g., at least about 80%, at least about 85%, at least about 90%, at least about 95%) sequence identity; (c) an HC CDR3 amino acid sequence set forth in Table A1 or a sequence selected from the group consisting of: SEQ ID NOs: 453, 457, 463, 467, 469, and 474; or a variant sequence thereof which differs by only one or two amino acids or which has at least or about 70% (e.g., at least about 80%, at least about 85%, at least about 90%, at least about 95%) sequence identity; (d) a LC CDR1 amino acid sequence set forth in Table A1 or a sequence selected from the group consisting of: SEQ ID NOs: 449, 476, 458, 464, 68, and 470; or a variant sequence thereof which differs by only one or two amino acids or which has at least or about 70% (e.g., at least about 80%, at least about 85%, at least about 90%, at least about 95%) sequence identity; (e) an LC CDR2 amino acid sequence set forth in Table A1 or a sequence selected from the group consisting of: SEQ ID NOs: 450, 477, 459, 57, 69, and 471; or a variant sequence thereof which differs by only one or two amino acids or which has at least or about 70% (e.g., at least about 80%, at least about 85%, at least about 90%, at least about 95%) sequence identity; (f) an LC CDR3 amino acid sequence set forth in Table A1 or a sequence selected from the group consisting of: SEQ ID NOs: 451, 454, 460, 58, 70, and 112, or a variant sequence thereof which differs by only one or two amino acids or which has at least or about 70%(e.g., at least about 80%, at least about 85%, at least about 90%, at least about 95%) sequence identity or (g) a combination of any two or more of (a)-(f).

**TABLE A1**

| | **LC CDR1** | **LC CDR2** | **LC CDR3** | **HC CDR1** | **HC CDR2** | **HC CDR3** |
|---|---|---|---|---|---|---|
| **AB1*** | 449 | 450 | 451 | 452 | 475 | 453 |
| **AB3*** | 476 | 477 | 454 | 455 | 456 | 457 |
| **AB4*** | 458 | 459 | 460 | 461 | 462 | 463 |
| **AB9*** | 464 | 57 | 58 | 465 | 466 | 467 |
| **AB11*** | 68 | 69 | 70 | 71 | 468 | 469 |
| **AB18*** | 470 | 471 | 112 | 472 | 473 | 474 |

In some aspects, the HC CDR1 comprises Gly immediately N-terminal of SEQ ID NO: 452 and, optionally, in some aspects, the HC CDR1 comprises MX immediately C-terminal of SEQ 452, wherein X is H, N, or S. In various aspects, the HC CDR3 comprises Ala immediately N-terminal of SEQ ID NO: 453. In various aspects, the LC CDR1 further comprises TAS immediately N-terminal of SEQ ID NO: 449, and, optionally, XH immediately C-terminal of SEQ ID NO: 449, wherein X is H, S, Y, or Q. In some aspects, as described below, the first amino acid of SEQ ID NO: 449 is S or Q. In some aspects, as described below, the first amino acid of SEQ ID NO: 451 is S or Q.

In various aspects, the HC CDR1 comprises Gly immediately N-terminal of SEQ ID NO: 455, and optionally, in various aspects, the HC CDR1 comprises MX immediately C-terminal of SEQ ID NO: 455, wherein X is N, S, or H. In some aspects, HC CDR2 comprises Gln immediately N-terminal of SEQ ID NO: SEQ ID NO: 456, and optionally H immediately C-terminal of SEQ ID NO: 456. In various aspects, the LC CDR1 comprises RIS immediately N-terminal of SEQ ID NO: 476, and optionally, comprises LA immediately C-terminal of SEQ ID NO: 476. In various aspects, the LC CDR2 comprises XLVE immediately C-terminal of SEQ ID NO: 477, wherein X is I or S.

In various aspects, the HC CDR1 comprises MH immediately C-terminal of SEQ ID NO: 461. In various aspects, the HC CDR2 comprises Tyr immediately N-terminal of SEQ ID NO: 462, and optionally, TH immediately C-terminal of SEQ ID NO: 462. In exemplary aspects, the HC CDR3 does not include the first two amino acids of SEQ ID NO: 463. In various aspects, the LC CDR1 comprises RSS immediately N-terminal of SEQ ID NO: 458, and optionally, LN immediately C-terminal of SEQ ID NO: 458. In various aspects, the LC CDR2 comprises XRFS immediately C-terminal of SEQ ID NO: 459, wherein X is Q, S, A, or D.

In various aspects, the HC CDR1 comprises MH immediately C-terminal of SEQ ID NO: 465. In various aspects, the HC CDR2 comprises YI immediately N-terminal of SEQ ID NO: 466, and optionally, Xaa immediately C-terminal of SEQ ID NO: 466, wherein Xaa is N, S, Q, or A. In various aspects, the LC CDR1 comprises LAS immediately N-terminal of SEQ ID NO: 464, and optionally, LA immediately C-terminal of SEQ ID NO: 464. In various aspects, the LC CDR2 comprises SLAD immediately C-terminal of SEQ ID NO: 57.

In various aspects, the HC CDR1 comprises MH immediately C-terminal of SEQ ID NO: 71. In various aspects, the HC CDR2 comprises Tyr immediately N-terminal of SEQ ID NO: 468 and optionally IY immediately C-terminal of SEQ ID NO: 468. In various aspects, the LC CDR1 comprises RAS immediately N-terminal of SEQ ID NO: 68, and optionally SYIH immediately C-terminal to SEQ 68. In various aspects, the LC CDR2 comprises XLES immediately C-terminal to SEQ ID NO: 69, wherein X is N, Q, S, A, or D.

In various aspects, the LC CDR1 comprises KSS immediately N-terminal of SEQ ID NO: 470, and optionally YLA immediately C-terminal to SEQ 470. In various aspects, the LC CDR2 comprises TRES immediately C-terminal of SEQ I DNO: 471. In various aspects, the HC CDR1 comprises MN immediately C-terminal of SEQ ID NO: 472. In various aspects, the HC CDR2 comprises Xaa immediately N-terminal of SEQ 473, wherein Xaa is N, Q, S, or A, and optionally, Thr immediately C-terminal of SEQ 473.

In various aspects, the antigen-binding protein comprises a LC CDR1 amino acid sequence, a LC CDR2 amino acid sequence, and a LC CDR3 amino acid sequence set forth in Table A1 and at least 1 or 2 of the HC CDR amino acid sequences set forth in Table A1. In various aspects, the antigen-binding protein comprises a HC CDR1 amino acid sequence, a HC CDR2 amino acid sequence, and a HC CDR3 amino acid sequence set forth in Table A1 and at least 1 or 2 of the LC CDR amino acid sequences set forth in Table A1.

In various embodiments, the antigen-binding protein comprises at least 3, 4, or 5 of the amino acid sequences designated by the SEQ ID NOs: in a single row of Table A1. In various embodiments, the antigen-binding protein comprises each of the LC CDR amino acid sequences designated by the SEQ ID NOs: of a single row of Table A1 and at least 1 or 2 of the HC CDR amino acid sequences designated by the SEQ ID NOs: in of a single row of Table A1. In various embodiments, the antigen-binding protein comprises each of the HC CDR amino acid sequences designated by the SEQ ID NOs: of a single row of Table A1 and at least 1 or 2 of the LC CDR amino acid sequences designated by the SEQ ID NOs: of a single row of Table A1. In various embodiments, the antigen-binding protein comprises all 6 of the CDR amino acid sequences designated by the SEQ ID NOs: of a single row of Table A1. In various embodiments, the antigen-binding protein comprises six CDR amino acid sequences selected from the group consisting of: (a) SEQ ID NOs: 449-453 and 475; (b) SEQ ID NOs: 476-477, 454-457; (c) SEQ ID NOs: 458-463; (d) SEQ ID NOs: 57, 58, 464-467; (e) SEQ ID NOs: 68-71 and 468-469; and (f) SEQ ID NOs: 112, and 470-474.

In various instances, the amino acid sequences of Table A1 are separated by at least one or more (e.g., at least 2, 3, 4, 5, 6, 7, 8, 9, 10, or more) intervening amino acid(s). In various instances, there are about 10 to about 20 amino acids between the sequences of the LC CDR1 and the LC CDR2 and about 25 to about 40 amino acids between the sequences of the LC CDR2 and the LC CDR3. In various instances, there are about 14 to about 16 amino acids between the sequences of the LC CDR1 and the LC CDR2 and about 30 to about 35 amino acids between the sequences of LC CDR2 and the LC CDR3. In various instances, there are about 10 to about 20 amino acids between the sequences of the HC CDR1 and HC CDR2 and about 25 to about 40 amino acids between the sequences of the HC CDR2 and the HC CDR3. In various instances, there are about 14 to about 16 amino acids between the sequences of the HC CDR1 and HC CDR2 and about 30 to about 35 amino acids between the sequences of the HC CDR2 and HC CDR3.

In various embodiments, the antigen-binding protein comprises (a) a heavy chain variable region amino acid sequence set forth in in Table B1 or a sequence selected from the group consisting of: SEQ ID NO: 478, 480, 482, 484, 486, and 488, or a variant sequence thereof which differs by only one or two amino acids or which has at least or about 70%(e.g., at least about 80%, at least about 85%, at least about 90%, at least about 95%) sequence identity; or (b) a light chain variable region amino acid sequence set forth in Table B1 or a sequence selected from the group consisting of: SEQ ID NO: 479, 481, 483, 485, 487, and 489, or a variant sequence thereof which differs by only one or two amino acids or which has at least or about 70% (e.g., at least about 80%, at least about 85%, at least about 90%, at least about 95%) sequence identity; or (c) both (a) and (b).

**TABLE B1**

| | **HC variable** | **LC variable** |
|---|---|---|
| **AB1*** | 478 | 479 |
| **AB3*** | 480 | 481 |
| **AB4*** | 482 | 483 |
| **AB9*** | 488 | 489 |
| **AB11*** | 486 | 487 |
| **AB18*** | 484 | 485 |

In various embodiments, the antigen-binding protein comprises a pair of amino acid sequences selected from the group consisting of: (a) SEQ ID NO: 478 and 479; (b) SEQ ID NO: 480 and 481; (c) SEQ ID NO: 482 and 483; (d) SEQ ID NO: 484 and 485; (e) SEQ ID NO: 486 and 487; and (f) SEQ ID NO: 488 and 489. In various aspects, the antigen-binding protein comprises a variant sequence of a sequence having a SEQ ID NO: listed in Table B1 which differs by only one or two amino acids or which has at least or about 70% (e.g., at least about 80%, at least about 85%, at least about 90%, at least about 95%) sequence identity, wherein the different amino acid(s) occur(s) at the positions described below in "Humanized Antibodies".

### Humanized Antibodies

In various aspects, the antigen-binding protein is a humanized version of an antigen binding protein described in Table A, Table A1, Table B, or Table B1.

### Humanized AB1

In various aspects, the antigen-binding protein is a humanized version of AB1 as set forth in Table B or B1 with one or more (e.g., 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, or 35) amino acid substitutions in the heavy chain variable region at one or more of the following positions: 5, 8, 11, 12, 13, 20, 31, 33, 35, 38, 40, 48, 50, 55, 57, 59, 61, 65, 66, 67, 68, 70, 72, 74, 76, 79, 80, 82, 87, 90, 91, 98, 101, and 116. In various instances, the antigen-binding protein comprises an amino acid sequence of SEQ ID NO: 428. In various aspects, the antigen-binding protein is a humanized version of AB1 as set forth in Table B or B1 with one or more amino acid substitutions in the heavy chain variable region at one or more (e.g., 1, 2, 3, 4, 5, 6, 7, 8, 9, 10, 11, or 12) of the following positions: 20, 31, 35, 48, 50, 59, 67, 70, 74, 79, 98, 101. In various instances, the antigen-binding protein comprises an amino acid sequence of SEQ ID NO: 429. In various aspects, the amino acids at the above-recited positions are selected from the amino acids according to the table below:

| *Position* | *Amino acids* | *Position* | *Amino acids* | *Position* | *Amino acids* |
|---|---|---|---|---|---|
| 5 | Q, V | 8 | A, G | 11 | L, V |
| 12 | A, K | 13 | R, K | 20 | M, V |
| 31 | S, T, V, D | 33 | Y, T | 35 | H, N, S |
| 38 | K, R | 40 | R, A | 48 | I, M |
| 50 | F, V, T, Y, I | 55 | G, S | 57 | S, Y |
| 59 | D, E, N, S | 61 | N, A | 65 | K, Q |
| 66 | D, G | 67 | R, Q, N, K | 68 | T, V |
| 70 | L, M | 72 | R, A | 74 | K, T |
| | | 79 | V, D, S, A | 82 | Q, E |
| 87 | T, R | 91 | S, T | 98 | N, Q, H, D, R |
| 101 | Y | 76 | ST | 116 | A, S |

In various aspects, the antigen-binding protein is a humanized version of AB1 as set forth in Table B or B1 with one or more amino acid substitutions in the light chain variable region at one or more e.g., 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, or 41) of the following positions: 1, 3, 4, 9, 10, 11, 15, 17, 21, 24, 27, 29, 32, 34, 35, 43, 44, 48, 51, 52, 53, 54, 55, 56, 61, 67, 71, 72, 73, 79, 80, 81, 84, 90, 92, 93, 94, 95, 96, 101, 107. In various instances, the antigen-binding protein comprises an amino acid sequence of SEQ ID NO: 430. In various aspects, the antigen-binding protein is a humanized version of AB1 as set forth in Table B or B1 with one or more amino acid substitutions in the light chain variable region at one or more (e.g., 1, 2, 3, 4, 5, 6, 7, 8, 9, 10, 11, 12, or 13) of the following positions: 4, 21, 32, 34, 48, 51, 53, 61, 67, 79, 84, 91, and 93. In various instances, the antigen-binding protein comprises an amino acid sequence of SEQ ID NO: 431. In various aspects, the amino acids at the above-recited positions are selected from the amino acids according to the table below:

| *Position* | *Amino acids* | *Position* | *Amino acids* | *Position* | *Amino acids* |
|---|---|---|---|---|---|
| 1 | Q, D | 3 | V, Q | 4 | L, M |
| 9 | A, S | 10 | I, S | 11 | M, L |
| 15 | L, V | 17 | E, D | 21 | M, I |
| 24 | T, R | 27 | S, Q | 32 | T, V, F, D, S |
| 34 | F, L | 35 | H, S, Y, Q, N | 43 | S, K |
| 44 | S, A | 48 | W, L | 51 | S, T, Q, A |
| 52 | T, A | 53 | S, T, D, Q | 54 | N, S |
| | | 56 | A, Q | 61 | R, Q, S, D, |
| 67 | A, S, T, G | 71 | S, D | 72 | Y, F |
| 73 | S, T | 79 | M, L | 80 | E, Q |
| 81 | A, P | 84 | A, F | 90 | H, Q |
| 91 | Q, H, S | 93 | H, Q, S, Y | 94 | R, S |
| 97 | L, P | 101 | A, Q | 107 | L, I |
| 29 | V, I | 92 | Y, S | 95 | S, T |

### Humanized AB3

In various aspects, the antigen-binding protein is a humanized version of AB3 as set forth in Table B or B1 with one or more amino acid substitutions in the heavy chain variable region at one or more (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, or 33) of the following positions: 3, 5, 18, 19, 23, 31, 33, 35, 40, 42, 49, 50, 52, 53, 54, 55, 56, 57, 58, 59, 61, 64, 76, 79, 80, 81, 87, 94, 95, 99, 106, 112, 114. In various instances, the antigen-binding protein comprises an amino acid sequence of SEQ ID NO: 432. In various aspects, the antigen-binding protein is a humanized version of AB3 as set forth in Table B or B1 with one or more amino acid substitutions in the heavy chain variable region at one or more (e.g., 1, 2, 3, 4, 5, 6, or 7) of the following positions: 31, 35, 50, 55, 79, 99, 106. In various instances, the antigen-binding protein comprises an amino acid sequence of SEQ ID NO: 433. In various aspects, the amino acids at the above-recited positions are selected from the amino acids according to the table below:

| *Position* | *Amino acids* | *Position* | *Amino acids* | *Position* | *Amino acids* |
|---|---|---|---|---|---|
| 3 | K, Q | 5 | E, L | 18 | M, L |
| 19 | K, R | 23 | V, A | 31 | N, S, R |
| 33 | W, A | 35 | N, S, H | 40 | S, A |
| 42 | E, G | 49 | A, S | 50 | Q, S, N, H, A |
| | | 52 | R, S | 53 | L, G |
| 54 | K, S | 55 | S, N, T, A, G | 56 | D, G |
| | | | | 59 | A, S |
| 61 | H, Y | 64 | E, D | 76 | D, N |
| 79 | R, N, Q, D, S | 80 | S, T | 81 | V, L |
| 87 | N, S | 94 | G, A | 95 | T, V, I |
| 99 | N, D, T, K, A | 106 | C, Y, A, S, T | 112 | T, L |
| 114 | I, T | | | | |

In various aspects, the antigen-binding protein is a humanized version of AB3 as set forth in Table B or B1 with one or more amino acid substitutions in the light chain variable region at one or more (e.g., 1, 2, 3, 4, 5, 6, 7, 8, 9, 10, 11, 12, 13, 14, 15, 16, 17, 18, 19, 20, 21, 22, 23, 24, 25, 26, 27, 28, or 29) of the following positions: 9, 17, 18, 25, 27, 28, 30, 34, 40, 43, 45, 48, 50, 52, 53, 55, 56, 70, 72, 74, 76, 84, 85, 90, 91, 93, 94, 97, and 100. In various instances, the antigen-binding protein comprises an amino acid sequence of SEQ ID NO: 434. In various aspects, the antigen-binding protein is a humanized version of AB3 as set forth in Table B or B1 with one or more amino acid substitutions in the light chain variable region at one or more (e.g., 1, 2, 3, 4, 5, 6, 7, 8, or 9) of the following positions: 25, 34, 48, 53, 55, 84, 85, 90, and 93. In various instances, the antigen-binding protein comprises an amino acid sequence of SEQ ID NO: 435. In various aspects, the amino acids at the above-recited positions are selected from the amino acids according to the table below:

| *Position* | *Amino acids* | *Position* | *Amino acids* | *Position* | *Amino acids* |
|---|---|---|---|---|---|
| 9 | A, S | 17 | E, D | 18 | T, R |
| 25 | I, V, L, T, A | 34 | A, S, N | 40 | Q, P |
| 43 | S, A | 45 | Q, K | 48 | V, I |
| 53 | I, V, L, T, S | 55 | V, T, L, A, Q | 70 | Q, D |
| 72 | S, T | 74 | K, T | 76 | N, S |
| 84 | G, A | 85 | N, Q, S, T | 90 | H, Q, S, T |
| 93 | T, S, N, G | 100 | G, Q | 27 | E, Q |
| 28 | N, S | 30 | Y, S | 50 | N, A |
| 52 | K, S | 56 | E, S | 91 | H, S |
| 94 | V, T | 97 | T, P | | |

### Humanized AB4

In various aspects, the antigen-binding protein is a humanized version of AB4 as set forth in Table B or B1 with one or more amino acid substitutions in the heavy chain variable region at one or more (e.g., 1, 2, 3, 4, 5, 6, 7, 8, 9, 10, 11, 12, 13, 14, 15, 16, 17, 18, 20, 21, 22, 23, 24, 25, 26, 27, 28, 29, 30, 31, 32, 33, 34, 35, or 36 of the following positions: 5, 11, 12, 13, 20, 29, 31, 33, 37, 38, 40, 45, 48, 50, 55, 56, 57, 59, 61, 62, 65, 66, 67, 68, 70, 72, 74, 76, 79, 82, 84, 87, 91, 97, 101, 117. In various instances, the antigen-binding protein comprises an amino acid sequence of SEQ ID NO: 436. In various aspects, the antigen-binding protein is a humanized version of AB4 as set forth in Table B or B1 with one or more amino acid substitutions in the heavy chain variable region at one or more (e.g., 1, 2, 3, 4, 5, 6, 7, 8, 9, 10, 11, 12, 13, 14, 15, 16, 17, 18, or 19) of the following positions: 20, 29, 31, 37, 45, 48, 56, 59, 61, 62, 65, 66, 68, 70, 74, 79, 84, 97, and 101. In various instances, the antigen-binding protein comprises an amino acid sequence of SEQ ID NO: 437. In various aspects, the amino acids at the above-recited positions are selected from the amino acids according to the table below:

| *Position* | *Amino acids* | *Position* | *Amino acids* | *Position* | *Amino acids* |
|---|---|---|---|---|---|
| 5 | Q, V | 11 | L, V | 12 | A, K |
| 13 | R, K | 20 | M, V | 33 | T, Y |
| 37 | I, V, F, Y | 38 | K, R | 40 | R, A |
| 45 | Q, L, V, T, N | 48 | I, M | 50 | Y, I |
| 55 | S, G | 56 | T, G, S, V, D | 57 | Y, S |
| 59 | H, K, S, Q, N | 61 | I, A, N, F, Y, V | 62 | K, Q |
| 65 | K, Q | 66 | D, G | 67 | K, R |
| 68 | A, V | 70 | L, M | 72 | A, R |
| 74 | T, K | 76 | S, T | 79 | A, V |
| 82 | Q, E | 84 | R, S, Q, D | 87 | T, R |
| 91 | S, T | 97 | S, A, T, V | 101 | L, V, F |
| 117 | A, S | 29 | F, Y, S, T | 31 | S, T, Y, D |

In various aspects, the antigen-binding protein is a humanized version of AB4 as set forth in Table B or B1 with one or more amino acid substitutions in the light chain variable region at one or more (e.g., 1, 2, 3, 4, 5, 6, 7, 8, 9, 10, 11, 12, 13, 14, 15, 16, 17, 18, 19, 20, 21, 22, 23, or 24) of the following positions: 7, 14, 17, 18, 31, 33, 39, 41, 42, 44, 50, 51, 55, 57, 60, 81, 88, 92, 94, 95, 96, 99, 100, 105. In various instances, the antigen-binding protein comprises an amino acid sequence of SEQ ID NO: 438. In various aspects, the antigen-binding protein is a humanized version of AB4 as set forth in Table B or B1 with one or more amino acid substitutions in the light chain variable region at one or more (e.g., 1, 2, 3, 4, 5, 6, or 7) of the following positions: 33, 39, 55, 57, 81, 95, and 96. In various instances, the antigen-binding protein comprises an amino acid sequence of SEQ ID NO: 439. In various aspects, the amino acids at the above-recited positions are selected from the amino acids according to the table below:

| *Position* | *Amino acids* | *Position* | *Amino acids* | *Position* | *Amino acids* |
|---|---|---|---|---|---|
| 7 | T, S | 14 | S, T | 17 | D, Q |
| 18 | Q, P | 31 | Y, H | 33 | D, N, E, Q |
| 39 | H, N, Q, D | 41 | F, Y | 42 | L, Q |
| 44 | K, R | 50 | K, R | 51 | R, L |
| 55 | K, R, Q | 57 | S, T, V | 60 | D, F |
| 81 | R, S, N, D | 88 | L, V | 92 | F, Y |
| 94 | M, S | 95 | Q, H, T | 96 | S, T, G, D |
| 99 | W, V | 105 | G, Q | | |

### Humanized AB18

In various aspects, the antigen-binding protein is a humanized version of AB18 as set forth in Table B or B1 with one or more amino acid substitutions in the heavy chain variable region at one or more (e.g., 1, 2, 3, 4, 5, 6, 7, 8, 9, 10, 11, 12, 13, 14, 15, 16, 17, 18, 19, 20, 21, 22, 23, 24, or 25) of the following positions: 5, 9, 11, 12, 20, 38, 40, 41, 43, 44, 48, 61, 65, 67, 68, 70, 72, 74, 76, 79, 82, 84, 87, 91, and 116, optionally, one or more (e.g., 1, 2, 3, 4, or 5) of the following positions: 20, 48, 68, 70, 79. In various instances, the antigen-binding protein comprises an amino acid sequence of SEQ ID NO: 440 or 441. In various aspects, the amino acids at the above-recited positions are selected from the amino acids according to the table below:

| *Position* | *Amino acids* | *Position* | *Amino acids* | *Position* | *Amino acids* |
|---|---|---|---|---|---|
| 5 | K, V | 9 | P, A | 11 | L, V |
| 12 | E, K | 20 | I, V | 38 | K, R |
| 40 | S, A | 41 | N, P | 43 | K, Q |
| 44 | S, G | 48 | I, V, M | 61 | N, A |
| 65 | T, Q | 67 | K, R | 68 | A, V |
| 70 | L, M | 72 | V, R | 74 | K, T |
| 76 | S, T | 79 | A, V | 82 | Q, E |
| 84 | K, S | 87 | T, R | 91 | S, T |
| 116 | S, L | | | | |

In various aspects, the antigen-binding protein is a humanized version of AB18 as set forth in Table B or B1 with one or more amino acid substitutions in the light chain variable region at one or more (e.g., 1, 2, 3, 4, 5, 6, 7, 8, 9, 10, 11, 12, 13, 14, 15, or 16) of the following positions: 1, 3, 9, 15, 18, 19, 21, 22, 49, 51, 69, 93, 84, 78, 105, and 111, optionally, one or more (e.g., 1, 2, or 3) of the following positions: 19, 21, or 84. In various instances, the antigen-binding protein comprises an amino acid sequence of SEQ ID NO: 442 or 443. In various aspects, the amino acids at the above-recited positions are selected from the amino acids according to the table below:

| *Position* | *Amino acids* | *Position* | *Amino acids* | *Position* | *Amino acids* |
|---|---|---|---|---|---|
| 1 | N, D | 3 | M, V | 9 | S, D |
| 15 | A, L | 18 | K, R | 19 | V, A |
| 21 | M, I | 22 | S, N | 49 | S, P |
| 51 | R, K | 69 | T, S | 83 | N, S |
| 84 | V, L | 89 | L, V | 105 | A, Q |
| 111 | L, I | | | | |

### Humanized AB9

In various aspects, the antigen-binding protein is a humanized version of AB9 as set forth in Table B or B1 with one or more amino acid substitutions in the heavy chain variable region at one or more (e.g., 1, 2, 3, 4, 5, 6, 7, 8, 9, 10, 11, 12, 13, 14, 15, 16, 17, 18, 19, 20, 21, 22, 23, 24, 25, 26, 27, 28, or 29) of the following positions: 1, 5, 9, 11, 12, 20, 38, 40, 41, 43, 44, 48, 61, 63, 65, 67, 69, 70, 72, 73, 74, 76, 79, 84, 87, 91, 93, 112, and 113. In various instances, the antigen-binding protein comprises an amino acid sequence of SEQ ID NO: 444. In various aspects, the amino acids at the above-recited positions are selected from the amino acids according to the table below:

| *Position* | *Amino acids* | *Position* | *Amino acids* | *Position* | *Amino acids* |
|---|---|---|---|---|---|
| 1 | E, Q | 5 | Q, V | 9 | P, A |
| 11 | L, V | 12 | V, K | 20 | M, V |
| 38 | K R, | 40 | S, A | 41 | H, P |
| 43 | K Q | 44 | S, G | 48 | I, M |
| 61 | N, A | 63 | N, K | 65 | K, Q |
| 67 | K, R | 69 | A, V | 70 | L, M |
| 72 | V, R | 73 | N, D | 74 | K, T |
| 76 | S, T | 79 | A, V | 84 | R, S |
| 87 | T, R | 91 | S, T | 93 | A, V |
| 112 | T, L | 113 | L, V | | |

In various aspects, the antigen-binding protein is a humanized version of AB9 as set forth in Table B or B1 with one or more amino acid substitutions in the light chain variable region at one or more (e.g., 1, 2, 3, 4, 5, 6, 7, 8, 9, 10, 11, 12, 13, 14, or 15) of the following positions: 9, 11, 15, 17, 18, 43, 45, 70, 72, 73, 74, 80, 84, 85, and 100. In various instances, the antigen-binding protein comprises an amino acid sequence of SEQ ID NO: 445. In various aspects, the amino acids at the above-recited positions are selected from the amino acids according to the table below:

| *Position* | *Amino acids* | *Position* | *Amino acids* | *Position* | *Amino acids* |
|---|---|---|---|---|---|
| 9 | A, S | 11 | Q, L | 15 | L, V |
| 17 | E, D | 18 | S, R | 43 | S, A |
| 45 | Q, K | 70 | R, D | 72 | S, T |
| 73 | F, L | 74 | K, R | 80 | A, P |
| 84 | V, A | 85 | S, T | 100 | G, Q |

### Humanized AB11

In various aspects, the antigen-binding protein is a humanized version of AB11 as set forth in Table B or B1 with one or more amino acid substitutions in the heavy chain variable region at one or more (e.g., 1, 2, 3, 4, 5, 6, 7, 8, 9, 10, 11, 12, 13, 14, or 15) of the following positions: 1, 15, 18, 19, 42, 49, 63, 75, 76, 78, 80, 84, 88, and 93. In various instances, the antigen-binding protein comprises an amino acid sequence of SEQ ID NO: 446. In various aspects, the amino acids at the above-recited positions are selected from the amino acids according to the table below:

| *Position* | *Amino acids* | *Position* | *Amino acids* | *Position* | *Amino acids* |
|---|---|---|---|---|---|
| 1 | D, E | 15 | R, G | 18 | R, L |
| 19 | K, R | 42 | E, G | 49 | A, S |
| 63 | T, S | 75 | P, A | 76 | T, K |
| 78 | T, S | 80 | F, Y | 84 | T, N |
| 88 | S, A | 93 | M, V | | |

In various aspects, the antigen-binding protein is a humanized version of AB11 as set forth in Table B or B1 with one or more amino acid substitutions in the light chain variable region at one or more (e.g., 1, 2, 3, 4, 5, 6, 7, 8, 9, 10, 11, 12, 13, 14, or 15) of the following positions: 4, 9, 17, 22, 64, 78, 80, 81, 82, 83, 84, 87, 89, 104, and 110, optionally, one or more of the following positions: 4, 82, 110. In various instances, the antigen-binding protein comprises an amino acid sequence of SEQ ID NO: 447 or 448. In various aspects, the amino acids at the above-recited positions are selected from the amino acids according to the table below:

| *Position* | *Amino acids* | *Position* | *Amino acids* | *Position* | *Amino acids* |
|---|---|---|---|---|---|
| 4 | L, M | 9 | A, D | 17 | Q, E |
| 22 | S, N | 64 | A, D | 78 | N, T |
| 80 | H, S | 81 | P, S | 82 | V, L |
| 83 | E, Q | 84 | E, A | 87 | A, V |
| 89 | T, V | 104 | A, Q | 110 | L, I |

In various embodiments, the antigen-binding protein comprises (a) a heavy chain variable region amino acid sequence set forth in in Table C or a sequence selected from the group consisting of: 376-379, 384-387, 391-396, 403-408, 412, 413, 416-419, and 422-427 or a variant sequence thereof which differs by only one or two amino acids or which has at least or about 70%, or about 80%, or about 85%, or about 90%, or about 95% sequence identity; or (b) a light chain variable region amino acid sequence set forth in Table C or a sequence selected from the group consisting of: 380-383, 388-390, 397-402, 409-411, 414, 415, 420, and 421 or a variant sequence thereof which differs by only one or two amino acids or which has at least or about 70%, or about 80%, or about 85%, or about 90%, or about 95% sequence identity; or (c) both (a) and (b).

**TABLE C**

| | Humanized Light Chain Variable Region | Humanized Heavy Chain Variable Region |
|---|---|---|
| AB1 | 380, 381, 382,383 | 376, 377, 378, 379 |
| AB3 | 388, 389, 390 | 384, 385, 386, 387, 422 |
| AB4 | 397, 398, 399, 400, 401, 402 | 391, 392, 392, 394, 395, 396, 423, 424, 425, 426, 427 |
| AB9 | 409, 410, 411, | 403, 404, 405, 406, 407, 408 |
| AB11 | 414, 415 | 412, 413 |
| AB18 | 420, 421 | 416, 417, 418, 419 |

In various embodiments, the humanized antigen-binding protein comprises a pair of amino acid sequences as shown in Table D.

**TABLE D**

| **Humanized AB** | **HC** | **LC** |
|---|---|---|
| 1-1 | 376 | 380 |
| 1-2 | 377 | 380 |
| 1-3 | 377 | 381 |
| 1-4 | 377 | 382 |
| 1-5 | 377 | 383 |
| 1-6 | 378 | 381 |
| 1-7 | 378 | 382 |
| 1-8 | 378 | 383 |
| 1-9 | 379 | 381 |
| 1-10 | 379 | 382 |
| 1-11 | 379 | 383 |
| 3-1 | 384 | 388 |
| 3-2 | 385 | 388 |
| 3-3 | 385 | 389 |
| 3-4 | 386 | 388 |
| 3-5 | 386 | 389 |
| 3-6 | 387 | 388 |
| 3-7 | 387 | 389 |
| 3-9 | 422 | 389 |
| 4-1 | 391 | 397 |
| 4-2 | 392 | 397 |
| 4-3 | 392 | 398 |
| 4-4 | 393 | 398 |
| 4-5 | 394 | 398 |
| 4-6 | 395 | 398 |
| 4-7 | 396 | 398 |
| 4-8 | 423 | 398 |
| 4-9 | 424 | 398 |
| 4-10 | 425 | 398 |
| 4-11 | 426 | 398 |
| 4-12 | 427 | 398 |
| 9-1 | 403 | 409 |
| 9-2 | 404 | 409 |
| 9-3 | 405 | 410 |
| 9-4 | 405 | 411 |
| 9-5 | 406 | 410 |
| 9-6 | 406 | 411 |
| 9-7 | 407 | 410 |
| 9-8 | 407 | 411 |
| 9-9 | 408 | 410 |
| 9-10 | 408 | 411 |
| 11-1 | 412 | 414 |
| 11-2 | 413 | 414 |
| 11-3 | 413 | 415 |
| 18-1 | 416 | 420 |
| 18-2 | 417 | 420 |
| 18-3 | 417 | 420 |
| 18-4 | 417 | 421 |
| 18-5 | 418 | 420 |
| 18-6 | 418 | 421 |
| 18-7 | 419 | 420 |
| 18-8 | 419 | 421 |

In various embodiments, the antigen-binding protein comprises a pair of variant sequences, each having at least or about 70% (e.g., at least about 80%, at least about 85%, at least about 90%, at least about 95%) sequence identity to a SEQ ID NO listed in Table C. In various embodiments, the antigen-binding protein comprises a pair of sequences: one sequence selected from a SEQ ID NO: listed in Table C and another sequence which is a variant sequence having at least or about 70% (e.g., at least about 80%, at least about 85%, at least about 90%, at least about 95%) sequence identity to a sequence having a SEQ ID NO: listed in Table D a sequence having a SEQ ID NO: listed in Table C.

In various embodiments, the antigen-binding protein comprises a pair of sequences: one sequence selected from a SEQ ID NO: listed in Table D, and another sequence which is a variant sequence having at least or about 70% (e.g., at least about 80%, at least about 85%, at least about 90%, at least about 95%) sequence identity to a sequence having a SEQ ID NO: listed in Table D. For instance, in various aspects, the antigen-binding protein comprises a sequences of SEQ ID NO: 419 and the antigen-binding protein further comprises a variant sequence having at least or about 70% (e.g., at least about 80%, at least about 85%, at least about 90%, at least about 95%) sequence identity to SEQ ID NO 421.

### Nucleic acids

The present disclosure further provides nucleic acids comprising a nucleotide sequence encoding an antigen-binding protein of the present disclosure. By "nucleic acid" as used herein includes "polynucleotide," "oligonucleotide," and "nucleic acid molecule," and generally means a polymer of DNA or RNA, or modified forms thereof, which can be single-stranded or double- stranded, synthesized or obtained (e.g., isolated and/or purified) from natural sources, which can contain natural, non-natural or altered nucleotides, and which can contain a natural, non-natural or altered inter-nucleotide linkage, such as a phosphoroamidate linkage or a phosphorothioate linkage, instead of the phosphodiester found between the nucleotides of an unmodified oligonucleotide. The nucleic acid can comprise any nucleotide sequence which encodes any of the antigen-binding proteins of the present disclosure. In various aspects, the nucleic acid comprises a nucleotide sequence which encodes an antigen-binding protein comprising (a) a heavy chain (HC) complementarity-determining region (CDR) 1 amino acid sequence set forth in Table A or A1 or a sequence selected from the group consisting of: SEQ ID NOs: 11, 17, 23, 29, 35, 41, 47, 53, 59, 65, 71, 77, 83, 89, 95, 101, 107, 113, 119, 125, 131, 452, 455, 461, 465, and 472, or a variant sequence thereof which differs by only one or two amino acids or which has at least or about 70% (e.g., at least or about 80%, at least or about 85%, at least or about 90%, at least or about 95%) sequence identity; (b) an HC CDR2 amino acid sequence set forth in Table A or A1 or a sequence selected from the group consisting of: SEQ ID NOs: 12, 18, 24, 30, 36, 42, 48, 54, 60, 66, 72, 78, 84, 90, 86, 102, 108, 114, 120, 126, 132, 475, 456, 462, 466, 468, and 473;, or a variant sequence thereof which differs by only one or two amino acids or which has at least or about 70% (e.g., at least or about 80%, at least or about 85%, at least or about 90%, at least or about 95%) sequence identity; (c) an HC CDR3 amino acid sequence set forth in Table A or A1 or a sequence selected from the group consisting of: SEQ ID NOs: 13, 19, 25, 31, 37, 43, 49, 55, 61, 67, 73, 79, 85, 91, 97, 103, 109, 115, 121, 127, 133, 453, 457, 463, 467, 469, and 474;, or a variant sequence thereof which differs by only one or two amino acids or which has at least or about 70% (e.g., at least or about 80%, at least or about 85%, at least or about 90%, at least or about 95%) sequence identity; (d) a light chain (LC) CDR1 amino acid sequence set forth in Table A or A1 or a sequence selected from the group consisting of: SEQ ID NOs: 8, 14, 20, 32, 38, 44, 50, 56, 62, 68, 74, 80, 86, 92, 98, 104, 110, 116, 122, 128, 449, 476, 458, 464, and 470; or a variant sequence thereof which differs by only one or two amino acids or which has at least or about 70% (e.g., at least or about 80%, at least or about 85%, at least or about 90%, at least or about 95%) sequence identity; (e) an LC CDR2 amino acid sequence set forth in Table A or A1 or a sequence selected from the group consisting of: SEQ ID NOs: 9, 15, 21, 27, 33, 39, 45, 51, 57, 63, 69, 75, 81, 87, 93, 99, 105, 111, 117, 123, 129, 450, 477, 459, and 471; or a variant sequence thereof which differs by only one or two amino acids or which has at least or about 70% (e.g., at least or about 80%, at least or about 85%, at least or about 90%, at least or about 95%) sequence identity; (f) an LC CDR3 amino acid sequence set forth in Table A or a sequence selected from the group consisting of: SEQ ID NOs: 10, 16, 22, 28, 34, 40, 46, 52, 58, 64, 70, 76, 82, 88, 94, 100, 106, 112, 118, 124, 130, 451, 454, and 460, or a variant sequence thereof which differs by only one or two amino acids or which has at least or about 70% (e.g., at least or about 80%, at least or about 85%, at least or about 90%, at least or about 95%) sequence identity; or (g) a combination of any two or more of (a)-(f). In various aspects, the nucleic acid comprises a nucleotide sequence encoding an antigen-binding protein comprising a LC CDR1 amino acid sequence, a LC CDR2 amino acid sequence, and a LC CDR3 amino acid sequence set forth in Table A or A1 and at least 1 or 2 of the HC CDR amino acid sequences set forth in Table A or A1. In various aspects, the nucleic acid comprises a nucleotide sequence encoding an antigen-binding protein comprising a HC CDR1 amino acid sequence, a HC CDR2 amino acid sequence, and a HC CDR3 amino acid sequence set forth in Table A or A1 and at least 1 or 2 of the LC CDR amino acid sequences set forth in Table A or A1. In various embodiments, the nucleic acid comprises a nucleotide sequence encoding an antigen-binding protein comprising (a) at least 3, 4, or 5 of the amino acid sequences designated by the SEQ ID NOs: in a single row of Table A or A1, (b) each of the LC CDR amino acid sequences designated by the SEQ ID NOs: of a single row of Table A or A1 and at least 1 or 2 of the HC CDR amino acid sequences designated by the SEQ ID NOs: in of a single row of Table A or A1, (c) each of the HC CDR amino acid sequences designated by the SEQ ID NOs: of a single row of Table A or A1 and at least 1 or 2 of the LC CDR amino acid sequences designated by the SEQ ID NOs: of a single row of Table A or A1, (d) all 6 of the CDR amino acid sequences designated by the SEQ ID NOs: of a single row of Table A, and/or (e) six CDR amino acid sequences selected from the group consisting of: (a) SEQ ID NOs: 74-79; (b) SEQ ID NOs: 50-55; (c) SEQ ID NOs: 122-127; (d) SEQ ID NOs: 26-31; (e) SEQ ID NOs: 128-133; (f) SEQ ID NOs: 38-43; (g) SEQ ID NOs: 62-67; (h) SEQ ID NOs: 80-85; (i) SEQ ID NOs: 44-49; (j) SEQ ID NOs: 86-91; (k) SEQ ID NOs: 104-109; (l) SEQ ID NOs: 56-61; (m) SEQ ID NOs: 32-37; (n) SEQ ID NOs: 110-115; (o) SEQ ID NOs: 98-103; (p) SEQ ID NOs: 92-97; (q) SEQ ID NOs: 116-121; (r) SEQ ID NOs: 8-13; (s) SEQ ID NOs: 68-73; (t) SEQ ID NOs: 14-19; (u) SEQ ID NOs: 20-25, (v) SEQ ID NOs: 449-453 and 475; (w) SEQ ID NOs: 476-477, 454-457; (x) SEQ ID NOs: 458-463; (y) SEQ ID NOs: 57, 58, 464-467; (z) SEQ ID NOs: 68-71 and 468-469; and (aa) SEQ ID NOs: 112, and 470-474. In various embodiments, the nucleic acid comprises a nucleotide sequence encoding an antigen-binding protein comprising (a) a heavy chain variable region amino acid sequence set forth in in Table B or B1 or a sequence selected from the group consisting of: 135, 137, 139, 141, 143, 145, 147, 149, 151, 153, 155, 157, 159, 161, 163, 165, 167, 169, 171, 173, 175, 478, 480, 482, 484, 486 and 488, or a variant sequence thereof which differs by only one or two amino acids or which has at least or about 70% (e.g., at least or about 80%, at least or about 85%, at least or about 90%, at least or about 95%) sequence identity; or (b) a light chain variable region amino acid sequence set forth in Table B or B1 or a sequence selected from the group consisting of: 134, 136, 138, 140, 142, 144, 146, 148, 150, 152, 154, 156, 158, 160, 162, 164, 166, 168, 170, 172, 174, 176, 479, 481, 483, 485, 487, and 489 or a variant sequence thereof which differs by only one or two amino acids or which has at least or about 70% (e.g., at least or about 80%, at least or about 85%, at least or about 90%, at least or about 95%) sequence identity; or (c) both (a) and (b). In various embodiments, the nucleic acid comprises a nucleotide sequence encoding an antigen-binding protein comprising a pair of amino acid sequences selected from the group consisting of: (a) SEQ ID NOs: 156 and 157; (b) SEQ ID NOs: 148 and 149; (c) SEQ ID NOs: 172 and 173; (d) SEQ ID NOs: 140 and 141; (e) SEQ ID NOs: 174 and 175; (f) SEQ ID NOs: 144 and 145; (g) SEQ ID NOs: 152 and 153; (h) SEQ ID NOs: 158 and 159; (i) SEQ ID NOs: 146 and 147; (j) SEQ ID NOs: 160 and 161; (k) SEQ ID NOs: 166 and 167; (l) SEQ ID NOs: 150 and 151; (m) SEQ ID NOs: 142 and 143; (n) SEQ ID NOs: 168 and 169; (o) SEQ ID NOs: 164 and 165; (p) SEQ ID NOs: 162 and 163; (q) SEQ ID NOs: 170 and 171; (r) SEQ ID NOs: 134 and 135; (s) SEQ ID NOs: 154 and 155; (t) SEQ ID NOs: 136 and 137; and (u) SEQ ID NOs: 138 and 139. In various embodiments, the nucleic acid comprises a nucleotide sequence encoding an antigen-binding protein comprising a pair of amino acid sequences selected from the group consisting of the pairs listed in Table D. In various aspects, the nucleic acid comprises a nucleotide sequence comprising a sequence of any one or more of SEQ ID NOs: 208-375. In some embodiments, the nucleic acid does not comprise any insertions, deletions, inversions, and/or substitutions. In other embodiments, the nucleic acid comprises one or more insertions, deletions, inversions, and/or substitutions.

In some aspects, the nucleic acids of the present disclosure are recombinant. As used herein, the term "recombinant" refers to (i) molecules that are constructed outside living cells by joining natural or synthetic nucleic acid segments to nucleic acid molecules that can replicate in a living cell, or (ii) molecules that result from the replication of those described in (i) above. For purposes herein, the replication can be in vitro replication or in vivo replication.

The nucleic acids in some aspects are constructed based on chemical synthesis and/or enzymatic ligation reactions using procedures known in the art. See, for example, Sambrook et al., *supra;* and Ausubel et al., *supra.* For example, a nucleic acid can be chemically synthesized using naturally occurring nucleotides or variously modified nucleotides designed to increase the biological stability of the molecules or to increase the physical stability of the duplex formed upon hybridization (e.g., phosphorothioate derivatives and acridine substituted nucleotides). Examples of modified nucleotides that can be used to generate the nucleic acids include, but are not limited to, 5-fluorouracil, 5-bromouracil, 5-chlorouracil, 5-iodouracil, hypoxanthine, xanthine, 4-acetylcytosine, 5-(carboxyhydroxymethyl) uracil, 5-carboxymethylaminomethyl-2-thiouridme, 5-carboxymethylaminomethyluracil, dihydrouracil, beta-D-galactosylqueosine, inosine, N⁶-isopentenyladenine, 1-methylguanine, 1-methylinosine, 2,2-dimethylguanine, 2-methyladenine, 2-methylguanine, 3-methylcytosine, 5-methylcytosine, N -substituted adenine, 7-methylguanine, 5-methylammomethyluracil, 5- methoxyaminomethyl-2-thiouracil, beta-D-mannosylqueosine, 5'- methoxycarboxymethyluracil, 5-methoxyuracil, 2-methylthio-N⁶-isopentenyladenine, uracil- 5-oxyacetic acid (v), wybutoxosine, pseudouratil, queosine, 2-thiocytosine, 5-methyl-2- thiouracil, 2-thiouracil, 4-thiouracil, 5-methyluracil, uracil-5-oxyacetic acid methylester, 3- (3-amino-3-N-2-carboxypropyl) uracil, and 2,6-diaminopurine. Alternatively, one or more of the nucleic acids of the present disclosure can be purchased from companies, such as Macromolecular Resources (Fort Collins, CO) and Synthegen (Houston, TX).

### Vector

The nucleic acids of the present disclosure in some aspects are incorporated into a vector. In this regard, the present disclosure provides vectors comprising any of the presently disclosed nucleic acids. In various aspects, the vector is a recombinant expression vector. For purposes herein, the term "recombinant expression vector" means a genetically-modified oligonucleotide or polynucleotide construct that permits the expression of an mRNA, protein, polypeptide, or peptide by a host cell, when the construct comprises a nucleotide sequence encoding the mRNA, protein, polypeptide, or peptide, and the vector is contacted with the cell under conditions sufficient to have the mRNA, protein, polypeptide, or peptide expressed within the cell. The vectors of the present disclosure are not naturally-occurring as a whole. However, parts of the vectors can be naturally-occurring. The presently disclosed vectors can comprise any type of nucleotides, including, but not limited to DNA and RNA, which can be single-stranded or double-stranded, synthesized or obtained in part from natural sources, and which can contain natural, non-natural or altered nucleotides. The vectors can comprise naturally-occurring or non-naturally-occurring internucleotide linkages, or both types of linkages. In some aspects, the altered nucleotides or non-naturally occurring internucleotide linkages do not hinder the transcription or replication of the vector.

The vector of the present disclosure can be any suitable vector, and can be used to transduce, transform or transfect any suitable host. Suitable vectors include those designed for propagation and expansion or for expression or both, such as plasmids and viruses. The vector can be a plasmid based expression vector. In various aspects, the vector is selected from the group consisting of the pUC series (Fermentas Life Sciences), the pBluescript series (Stratagene, LaJolla, CA), the pET series (Novagen, Madison, Wl), the pGEX series (Pharmacia Biotech, Uppsala, Sweden), and the pEX series (Clontech, Palo Alto, CA). Bacteriophage vectors, such as λGTIO, λGTI 1, λZapII (Stratagene), λEMBL4, and λNMI 149, also can be used. Examples of plant expression vectors include pBIOI, pBI101.2, pBI101.3, pBI121 and pBIN19 (Clontech). Examples of animal expression vectors include pEUK-CI, pMAM and pMAMneo (Clontech). In some aspects, the vector is a viral vector, e.g., a retroviral vector. In various aspects, the vector is an adenovirus vector, an adeno-associated virus (AAV) vector, a Herpes Simplex Virus (HSV) vector, a Vesicular stomatitis virus (VSV) vector, vaccinia virus vector, or lentivirus vector. See, e.g., Howarth et al., Cell Biol. Toxicol. 26(1): 1-20 (2010). In various aspects, the vector is a baculovirus vector which infects arthropods, e.g., insects. In various aspects, the baculovirus vector is an Autographacalifornica multiple nuclear virus (AcMNPV) or a Bombyxmorinuclear polyhedrosis (BmNPV). See, e.g., Khan, Adv Pharm Bull 3(2): 257-263 (2013); Miller, Bioessays 11(4): 91-96 (1989); Atkinson et al., Pestic Sci 28: 215-224 (1990).

The vectors of the present disclosure can be prepared using standard recombinant DNA techniques described in, for example, Sambrook et al., supra, and Ausubel et al., supra. Constructs of expression vectors, which are circular or linear, can be prepared to contain a replication system functional in a prokaryotic or eukaryotic host cell. Replication systems can be derived, e.g., from ColEl, 2 µ plasmid, λ, SV40, bovine papilloma virus, and the like.

In some aspects, the vector comprises regulatory sequences, such as transcription and translation initiation and termination codons, which are specific to the type of host (e.g., bacterium, fungus, plant, or animal) into which the vector is to be introduced, as appropriate and taking into consideration whether the vector is DNA- or RNA- based.

The vector can include one or more marker genes, which allow for selection of transformed or transfected hosts. Marker genes include biocide resistance, e.g., resistance to antibiotics, heavy metals, etc., complementation in an auxotrophic host to provide prototrophy, and the like. Suitable marker genes for the presently disclosed expression vectors include, for instance, neomycin/G418 resistance genes, hygromycin resistance genes, histidinol resistance genes, tetracycline resistance genes, and ampicillin resistance genes.

The vector can comprise a native or normative promoter operably linked to the nucleotide sequence encoding the polypeptide (including functional portions and functional variants thereof), or to the nucleotide sequence which is complementary to or which hybridizes to the nucleotide sequence encoding the polypeptide. The selection of promoters, e.g., strong, weak, inducible, tissue-specific and developmental- specific, is within the ordinary skill of the artisan. Similarly, the combining of a nucleotide sequence with a promoter is also within the skill of the artisan. The promoter can be a non-viral promoter or a viral promoter, e.g., a cytomegalovirus (CMV) promoter, an SV40 promoter, an RSV promoter, and a promoter found in the long-terminal repeat of the murine stem cell virus.

### Host cells

Provided herein are host cells comprising a nucleic acid or vector of the present disclosure. As used herein, the term "host cell" refers to any type of cell that can contain the presently disclosed vector and is capable of producing an expression product encoded by the nucleic acid (e.g., mRNA, protein). The host cell in some aspects is an adherent cell or a suspended cell, i.e., a cell that grows in suspension. The host cell in various aspects is a cultured cell or a primary cell, i.e., isolated directly from an organism, e.g., a human. The host cell can be of any cell type, can originate from any type of tissue, and can be of any developmental stage.

In various aspects, the antigen-binding protein is a glycosylated protein and the host cell is a glycosylation-competent cell. In various aspects, the glycosylation-competent cell is an eukaryotic cell, including, but not limited to, a yeast cell, filamentous fungi cell, protozoa cell, algae cell, insect cell, or mammalian cell. Such host cells are described in the art. See, e.g., Frenzel, et al., Front Immunol 4: 217 (2013). In various aspects, the eukaryotic cells are mammalian cells. In various aspects, the mammalian cells are non-human mammalian cells. In some aspects, the cells are Chinese Hamster Ovary (CHO) cells and derivatives thereof (e.g., CHO-K1, CHO pro-3), mouse myeloma cells (e.g., NS0, GS-NS0, Sp2/0), cells engineered to be deficient in dihydrofolatereductase (DHFR) activity (e.g., DUKX-X11, DG44), human embryonic kidney 293 (HEK293) cells or derivatives thereof (e.g., HEK293T, HEK293-EBNA), green African monkey kidney cells (e.g., COS cells, VERO cells), human cervical cancer cells (e.g., HeLa), human bone osteosarcoma epithelial cells U2-OS, adenocarcinomic human alveolar basal epithelial cells A549, human fibrosarcoma cells HT1080, mouse brain tumor cells CAD, embryonic carcinoma cells P19, mouse embryo fibroblast cells NIH 3T3, mouse fibroblast cells L929, mouse neuroblastoma cells N2a, human breast cancer cells MCF-7, retinoblastoma cells Y79, human retinoblastoma cells SO-Rb50, human liver cancer cells Hep G2, mouse B myeloma cells J558L, or baby hamster kidney (BHK) cells (Gaillet et al. 2007; Khan, Adv Pharm Bull 3(2): 257-263 (2013)).

For purposes of amplifying or replicating the vector, the host cell is in some aspects is a prokaryotic cell, e.g., a bacterial cell.

Also provided by the present disclosure is a population of cells comprising at least one host cell described herein. The population of cells in some aspects is a heterogeneous population comprising the host cell comprising vectors described, in addition to at least one other cell, which does not comprise any of the vectors. Alternatively, in some aspects, the population of cells is a substantially homogeneous population, in which the population comprises mainly host cells (e.g., consisting essentially of) comprising the vector. The population in some aspects is a clonal population of cells, in which all cells of the population are clones of a single host cell comprising a vector, such that all cells of the population comprise the vector. In various embodiments of the present disclosure, the population of cells is a clonal population comprising host cells comprising a vector as described herein.

### Manufacture methods

Also provided herein are methods of producing an antigen-binding protein which binds to CLDN6. In various embodiments, the method comprises culturing a host cell comprising a nucleic acid comprising a nucleotide sequence encoding the antigen-binding protein as described herein in a cell culture medium and harvesting the antigen-binding protein from the cell culture medium. The host cell can be any of the host cells described herein. In various aspects, the host cell is selected from the group consisting of: CHO cells, NS0 cells, COS cells, VERO cells, and BHK cells. In various aspects, the step of culturing a host cell comprises culturing the host cell in a growth medium to support the growth and expansion of the host cell. In various aspects, the growth medium increases cell density, culture viability and productivity in a timely manner. In various aspects, the growth medium comprises amino acids, vitamins, inorganic salts, glucose, and serum as a source of growth factors, hormones, and attachment factors. In various aspects, the growth medium is a fully chemically defined media consisting of amino acids, vitamins, trace elements, inorganic salts, lipids and insulin or insulin-like growth factors. In addition to nutrients, the growth medium also helps maintain pH and osmolality. Several growth media are commercially available and are described in the art. See, e.g., Arora, "Cell Culture Media: A Review" MATER METHODS 3:175 (2013).

In various aspects, the method comprises culturing the host cell in a feed medium. In various aspects, the method comprises culturing in a feed medium in a fed-batch mode. Methods of recombinant protein production are known in the art. See, e.g., Li et al., "Cell culture processes for monoclonal antibody production" MAbs 2(5): 466-477 (2010).

The method making an antigen-binding protein can comprise one or more steps for purifying the protein from a cell culture or the supernatant thereof and preferably recovering the purified protein. In various aspects, the method comprises one or more chromatography steps, e.g., affinity chromatography (e.g., protein A affinity chromatography), ion exchange chromatography, hydrophobic interaction chromatography. In various aspects, the method comprises purifying the protein using a Protein A affinity chromatography resin.

In various embodiments, the method further comprises steps for formulating the purified protein, etc., thereby obtaining a formulation comprising the purified protein. Such steps are described in Formulation and Process Development Strategies for Manufacturing, eds. Jameel and Hershenson, John Wiley & Sons, Inc. (Hoboken, NJ), 2010.

In various aspects, the antigen-binding protein linked to a polypeptide and the antigen-binding protein is part of a fusion protein. Thus, the present disclosure further provides methods of producing a fusion protein comprising an antigen-binding protein which binds to CLDN6. In various embodiments, the method comprises culturing a host cell comprising a nucleic acid comprising a nucleotide sequence encoding the fusion protein as described herein in a cell culture medium and harvesting the fusion protein from the cell culture medium.

### Conjugates

The present disclosure also provides antigen-binding proteins attached, linked or conjugated to a second moiety (e.g., a heterologous moiety, a conjugate moiety). Accordingly, the present disclosure provides a conjugate comprising an antigen-binding protein and a heterologous moiety. As used herein, the term "heterologous moiety" is synonymous with "conjugate moiety" and refers to any molecule (chemical or biochemical, naturally-occurring or non-coded) which is different from the antigen-binding proteins of the present disclosure. Various heterologous moieties include, but are not limited to, a polymer, a carbohydrate, a lipid, a nucleic acid, an oligonucleotide, a DNA or RNA, an amino acid, peptide, polypeptide, protein, therapeutic agent, (e.g., a cytotoxic agent, cytokine), or a diagnostic agent.

In some embodiments, the heterologous moiety is a polymer. The polymer can be branched or unbranched. The polymer can be of any molecular weight. The polymer in some embodiments has an average molecular weight of between about 2 kDa to about 100 kDa (the term "about" indicating that in preparations of a water soluble polymer, some molecules will weigh more, some less, than the stated molecular weight). The average molecular weight of the polymer is in some aspect between about 5 kDa and about 50 kDa, between about 12 kDa to about 40 kDa or between about 20 kDa to about 35 kDa.

In some embodiments, the polymer is modified to have a single reactive group, such as an active ester for acylation or an aldehyde for alkylation, so that the degree of polymerization can be controlled. The polymer in some embodiments is water soluble so that the protein to which it is attached does not precipitate in an aqueous environment, such as a physiological environment. In some embodiments, when, for example, the composition is used for therapeutic use, the polymer is pharmaceutically acceptable. Additionally, in some aspects, the polymer is a mixture of polymers, e.g., a co-polymer, a block co-polymer.

In some embodiments, the polymer is selected from the group consisting of: polyamides, polycarbonates, polyalkylenes and derivatives thereof including, polyalkylene glycols, polyalkylene oxides, polyalkylene terepthalates, polymers of acrylic and methacrylic esters, including poly(methyl methacrylate), poly(ethyl methacrylate), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), and poly(octadecyl acrylate), polyvinyl polymers including polyvinyl alcohols, polyvinyl ethers, polyvinyl esters, polyvinyl halides, poly(vinyl acetate), and polyvinylpyrrolidone, polyglycolides, polysiloxanes, polyurethanes and co-polymers thereof, celluloses including alkyl cellulose, hydroxyalkyl celluloses, cellulose ethers, cellulose esters, nitro celluloses, methyl cellulose, ethyl cellulose, hydroxypropyl cellulose, hydroxy-propyl methyl cellulose, hydroxybutyl methyl cellulose, cellulose acetate, cellulose propionate, cellulose acetate butyrate, cellulose acetate phthalate, carboxylethyl cellulose, cellulose triacetate, and cellulose sulphate sodium salt, polypropylene, polyethylenes including polyethylene glycol), polyethylene oxide), and polyethylene terephthalate), and polystyrene.

A particularly preferred water-soluble polymer for use herein is polyethylene glycol (PEG). As used herein, polyethylene glycol is meant to encompass any of the forms of PEG that can be used to derivatize other proteins, such as mono-(C1-C10) alkoxy- or aryloxy-polyethylene glycol. PEG is a linear or branched neutral polyether, available in a broad range of molecular weights, and is soluble in water and most organic solvents.

In some embodiments, the heterologous moiety is a carbohydrate. In some embodiments, the carbohydrate is a monosaccharide (e.g., glucose, galactose, fructose), a disaccharide (e.g., sucrose, lactose, maltose), an oligosaccharide (e.g., raffinose, stachyose), a polysaccharide (a starch, amylase, amylopectin, cellulose, chitin, callose, laminarin, xylan, mannan, fucoidan, galactomannan.

In some embodiments, the heterologous moiety is a lipid. The lipid, in some embodiments, is a fatty acid, eicosanoid, prostaglandin, leukotriene, thromboxane, N-acyl ethanolamine), glycerolipid (e.g., mono-, di-, tri-substituted glycerols), glycerophospholipid (e.g., phosphatidylcholine, phosphatidylinositol, phosphatidylethanolamine, phosphatidylserine), sphingolipid (e.g., sphingosine, ceramide), sterol lipid (e.g., steroid, cholesterol), prenol lipid, saccharolipid, or a polyketide, oil, wax, cholesterol, sterol, fat-soluble vitamin, monoglyceride, diglyceride, triglyceride, a phospholipid.

In some embodiments, the heterologous moiety is a therapeutic agent. The therapeutic agent can be any of those known in the art. Examples of therapeutic agents that are contemplated herein include, but are not limited to, natural enzymes, proteins derived from natural sources, recombinant proteins, natural peptides, synthetic peptides, cyclic peptides, antibodies, receptor agonists, cytotoxic agents, immunoglobins, beta-adrenergic blocking agents, calcium channel blockers, coronary vasodilators, cardiac glycosides, antiarrhythmics, cardiac sympathomemetics, angiotensin converting enzyme (ACE) inhibitors, diuretics, inotropes, cholesterol and triglyceride reducers, bile acid sequestrants, fibrates, 3-hydroxy-3-methylgluteryl (HMG)-CoA reductase inhibitors, niacin derivatives, antiadrenergic agents, alpha-adrenergic blocking agents, centrally acting antiadrenergic agents, vasodilators, potassium-sparing agents, thiazides and related agents, angiotensin II receptor antagonists, peripheral vasodilators, antiandrogens, estrogens, antibiotics, retinoids, insulins and analogs, alpha-glucosidase inhibitors, biguanides, meglitinides, sulfonylureas, thizaolidinediones, androgens, progestogens, bone metabolism regulators, anterior pituitary hormones, hypothalamic hormones, posterior pituitary hormones, gonadotropins, gonadotropin-releasing hormone antagonists, ovulation stimulants, selective estrogen receptor modulators, antithyroid agents, thyroid hormones, bulk forming agents, laxatives, antiperistaltics, flora modifiers, intestinal adsorbents, intestinal anti-infectives, antianorexic, anticachexic, antibulimics, appetite suppressants, antiobesity agents, antacids, upper gastrointestinal tract agents, anticholinergic agents, aminosalicylic acid derivatives, biological response modifiers, corticosteroids, antispasmodics, 5-HT₄ partial agonists, antihistamines, cannabinoids, dopamine antagonists, serotonin antagonists, cytoprotectives, histamine H2-receptor antagonists, mucosal protective agent, proton pump inhibitors, H. pylori eradication therapy, erythropoieses stimulants, hematopoietic agents, anemia agents, heparins, antifibrinolytics, hemostatics, blood coagulation factors, adenosine diphosphate inhibitors, glycoprotein receptor inhibitors, fibrinogen-platelet binding inhibitors, thromboxane-A₂ inhibitors, plasminogen activators, antithrombotic agents, glucocorticoids, mineralcorticoids, corticosteroids, selective immunosuppressive agents, antifungals, drugs involved in prophylactic therapy, AIDS-associated infections, cytomegalovirus, non-nucleoside reverse transcriptase inhibitors, nucleoside analog reverse transcriptse inhibitors, protease inhibitors, anemia, Kaposi's sarcoma, aminoglycosides, carbapenems, cephalosporins, glycopoptides, lincosamides, macrolies, oxazolidinones, penicillins, streptogramins, sulfonamides, trimethoprim and derivatives, tetracyclines, anthelmintics, amebicies, biguanides, cinchona alkaloids, folic acid antagonists, quinoline derivatives, Pneumocystis carinii therapy, hydrazides, imidazoles, triazoles, nitroimidzaoles, cyclic amines, neuraminidase inhibitors, nucleosides, phosphate binders, cholinesterase inhibitors, adjunctive therapy, barbiturates and derivatives, benzodiazepines, gamma aminobutyric acid derivatives, hydantoin derivatives, iminostilbene derivatives, succinimide derivatives, anticonvulsants, ergot alkaloids, antimigrane preparations, biological response modifiers, carbamic acid eaters, tricyclic derivatives, depolarizing agents, nondepolarizing agents, neuromuscular paralytic agents, CNS stimulants, dopaminergic reagents, monoamine oxidase inhibitors, COMT inhibitors, alkyl sulphonates, ethylenimines, imidazotetrazines, nitrogen mustard analogs, nitrosoureas, platinum-containing compounds, antimetabolites, purine analogs, pyrimidine analogs, urea derivatives, antracyclines, actinomycinds, camptothecin derivatives, epipodophyllotoxins, taxanes, vinca alkaloids and analogs, antiandrogens, antiestrogens, nonsteroidal aromatase inhibitors, protein kinase inhibitor antineoplastics, azaspirodecanedione derivatives, anxiolytics, stimulants, monoamind reuptake inhibitors, selective serotonin reuptake inhibitors, antidepressants, benzisooxazole derivatives, butyrophenone derivatives, dibenzodiazepine derivatives, dibenzothiazepine derivatives, diphenylbutylpiperidine derivatives, phenothiazines, thienobenzodiazepine derivatives, thioxanthene derivatives, allergenic extracts, nonsteroidal agents, leukotriene receptor antagonists, xanthines, endothelin receptor antagonist, prostaglandins, lung surfactants, mucolytics, antimitotics, uricosurics, xanthine oxidase inhibitors, phosphodiesterase inhibitors, metheamine salts, nitrofuran derivatives, quinolones, smooth muscle relaxants, parasympathomimetic agents, halogenated hydrocarbons, esters of amino benzoic acid, amides (e.g. lidocaine, articaine hydrochloride, bupivacaine hydrochloride), antipyretics, hynotics and sedatives, cyclopyrrolones, pyrazolopyrimidines, nonsteroidal anti-inflammatory drugs, opioids, para-aminophenol derivatives, alcohol dehydrogenase inhibitor, heparin antagonists, adsorbents, emetics, opoid antagonists, cholinesterase reactivators, nicotine replacement therapy, vitamin A analogs and antagonists, vitamin B analogs and antagonists, vitamin C analogs and antagonists, vitamin D analogs and antagonists, vitamin E analogs and antagonists, vitamin K analogs and antagonists.

The antigen-binding proteins of the present disclosure can be conjugated to one or more cytokines and growth factors that are effective in inhibiting tumor metastasis, and wherein the cytokine or growth factor has been shown to have an antiproliferative effect on at least one cell population. Such cytokines, lymphokines, growth factors, or other hematopoietic factors include, but are not limited to: M-CSF, GM-CSF, TNF, IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IFN, TNFα, TNF1, TNF2, G-CSF, Meg-CSF, GM-CSF, thrombopoietin, stem cell factor, and erythropoietin. Additional growth factors for use herein include angiogenin, bone morphogenic protein-1, bone morphogenic protein-2, bone morphogenic protein-3, bone morphogenic protein-4, bone morphogenic protein-5, bone morphogenic protein-6, bone morphogenic protein-7, bone morphogenic protein-8, bone morphogenic protein-9, bone morphogenic protein-10, bone morphogenic protein-11, bone morphogenic protein-12, bone morphogenic protein-13, bone morphogenic protein-14, bone morphogenic protein-15, bone morphogenic protein receptor IA, bone morphogenic protein receptor IB, brain derived neurotrophic factor, ciliary neutrophic factor, ciliary neutrophic factor receptor α, cytokine-induced neutrophil chemotactic factor 1, cytokine-induced neutrophil, chemotactic factor 2 α, cytokine-induced neutrophil chemotactic factor 2 β, β endothelial cell growth factor, endothelin 1, epithelial-derived neutrophil attractant, glial cell line-derived neutrophic factor receptor α 1, glial cell line-derived neutrophic factor receptor α 2, growth related protein, growth related protein α, growth related protein β, growth related protein γ, heparin binding epidermal growth factor, hepatocyte growth factor, hepatocyte growth factor receptor, insulin-like growth factor I, insulin-like growth factor receptor, insulin-like growth factor II, insulin-like growth factor binding protein, keratinocyte growth factor, leukemia inhibitory factor, leukemia inhibitory factor receptor α, nerve growth factor nerve growth factor receptor, neurotrophin-3, neurotrophin-4, pre-B cell growth stimulating factor, stem cell factor, stem cell factor receptor, transforming growth factor α, transforming growth factor β, transforming growth factor β1, transforming growth factor β1.2, transforming growth factor β2, transforming growth factor β3, transforming growth factor β5, latent transforming growth factor β1, transforming growth factor β binding protein I, transforming growth factor β binding protein II, transforming growth factor β binding protein III, tumor necrosis factor receptor type I, tumor necrosis factor receptor type II, urokinase-type plasminogen activator receptor, and chimeric proteins and biologically or immunologically active fragments thereof.

In some embodiments, the conjugate comprises an antigen-binding protein as described herein and a cytotoxic agent. The cytotoxic agent is any molecule (chemical or biochemical) which is toxic to a cell. In some aspects, when a cytotoxic agent is conjugated to an antigen-binding protein of the present disclosure, the results obtained are synergistic. That is to say, the effectiveness of the combination therapy of an antigen-binding protein and the cytotoxic agent is synergistic, i.e., the effectiveness is greater than the effectiveness expected from the additive individual effects of each. Therefore, the dosage of the cytotoxic agent can be reduced and thus, the risk of the toxicity problems and other side effects is concomitantly reduced. In some embodiments, the cytotoxic agent is a chemotherapeutic agent. Chemotherapeutic agents are known in the art and include, but not limited to, platinum coordination compounds, topoisomerase inhibitors, antibiotics, antimitotic alkaloids and difluoronucleosides, as described in U.S. Pat. No. 6,630,124.

In some embodiments, the chemotherapeutic agent is a platinum coordination compound. The term "platinum coordination compound" refers to any tumor cell growth inhibiting platinum coordination compound that provides the platinum in the form of an ion. In some embodiments, cisplatin is the platinum coordination compound employed in the compositions and methods of the present disclosure. In some embodiments, the chemotherapeutic agent is a topoisomerase inhibitor. In some aspects, the topoisomerase inhibitor is camptothecin or a camptothecin analog. In still yet other embodiments of the present disclosure, the chemotherapeutic agent is an antibiotic compound. Suitable antibiotic include, but are not limited to, doxorubicin, mitomycin, bleomycin, daunorubicin and streptozocin. In some embodiments, the chemotherapeutic agent is an antimitotic alkaloid. In general, antimitotic alkaloids can be extracted from Cantharanthus roseus, and have been shown to be efficacious as anticancer chemotherapy agents. In other embodiments of the present disclosure, the chemotherapeutic agent is a difluoronucleoside. 2'-deoxy-2',2'-difluoronucleosides are known in the art as having antiviral activity. Such compounds are disclosed and taught in U.S. Pat. Nos. 4,526,988 and 4,808614. European Patent Application Publication 184,365 discloses that these same difluoronucleosides have oncolytic activity.

The present disclosure also provides conjugates comprising an antigen-binding protein of the present disclosure linked to a polypeptide, such that the conjugate is a fusion protein. Therefore, the present disclosure provides fusion proteins comprising an antigen-binding protein of the present disclosure linked to a polypeptide. In various embodiments, the polypeptide is a diagnostic label, e.g., a fluorescent protein, such as green fluorescent protein, or other tag, e.g., Myc tag. In various aspects, the polypeptide is one of the cytokines, lymphokines, growth factors, or other hematopoietic factors listed above.

### Linkers

In some embodiments, the conjugate is directly linked to the heterologous moiety. In alternative embodiments, the conjugate comprises a linker that joins the compound of the present disclosure to the heterologous moiety. In some aspects, the linker comprises a chain of atoms from 1 to about 60, or 1 to 30 atoms or longer, 2 to 5 atoms, 2 to 10 atoms, 5 to 10 atoms, or 10 to 20 atoms long. In some embodiments, the chain atoms are all carbon atoms. In some embodiments, the chain atoms in the backbone of the linker are selected from the group consisting of C, O, N, and S. Chain atoms and linkers can be selected according to their expected solubility (hydrophilicity) so as to provide a more soluble conjugate. In some embodiments, the linker provides a functional group that is subject to cleavage by an enzyme or other catalyst or hydrolytic conditions found in the target tissue or organ or cell. In some embodiments, the length of the linker is long enough to reduce the potential for steric hindrance. In some embodiments, the linker is an amino acid or a peptidyl linker. Such peptidyl linkers can be any length. Various linkers are from about 1 to 50 amino acids in length, 5 to 50, 3 to 5, 5 to 10, 5 to 15, or 10 to 30 amino acids in length.

### Compositions, Pharmaceutical Compositions and Formulations

Compositions comprising an antigen-binding protein, a nucleic acid, a vector, a host cell, or a conjugate as presently disclosed are provided herein. The compositions in some aspects comprise the antigen-binding proteins in isolated and/or purified form. In some aspects, the composition comprises a single type (e.g., structure) of an antigen-binding protein of the present disclosure or comprises a combination of two or more antigen-binding proteins of the present disclosure, wherein the combination comprises two or more antigen-binding proteins of different types (e.g., structures).

In some aspects, the composition comprises agents which enhance the chemico-physico features of the antigen-binding protein, e.g., via stabilizing the antigen-binding protein at certain temperatures, e.g., room temperature, increasing shelf life, reducing degradation, e.g., oxidation protease mediated degradation, increasing half-life of the antigen-binding protein, etc. In some aspects, the composition comprises any of the agents disclosed herein as a heterologous moiety or conjugate moiety, optionally in admixture with the antigen-binding proteins of the present disclosure or conjugated to the antigen-binding proteins.

In various aspects of the present disclosure, the composition additionally comprises a pharmaceutically acceptable carrier, diluents, or excipient. In some embodiments, the antigen-binding protein, a nucleic acid, a vector, a host cell, or a conjugate as presently disclosed (hereinafter referred to as "active agents") is formulated into a pharmaceutical composition comprising the active agent, along with a pharmaceutically acceptable carrier, diluent, or excipient. In this regard, the present disclosure further provides pharmaceutical compositions comprising an active agent which is intended for administration to a subject, e.g., a mammal.

In some embodiments, the active agent is present in the pharmaceutical composition at a purity level suitable for administration to a patient. In some embodiments, the active agent has a purity level of at least about 90%, about 91%, about 92%, about 93%, about 94%, about 95%, about 96%, about 97%, about 98% or about 99%, and a pharmaceutically acceptable diluent, carrier or excipient. In some embodiments, the compositions contain an active agent at a concentration of about 0.001 to about 30.0 mg/ml.

In various aspects, the pharmaceutical compositions comprise a pharmaceutically acceptable carrier. As used herein, the term "pharmaceutically acceptable carrier" includes any of the standard pharmaceutical carriers, such as a phosphate buffered saline solution, water, emulsions such as an oil/water or water/oil emulsion, and various types of wetting agents. The term also encompasses any of the agents approved by a regulatory agency of the US Federal government or listed in the US Pharmacopeia for use in animals, including humans.

The pharmaceutical composition can comprise any pharmaceutically acceptable ingredients, including, for example, acidifying agents, additives, adsorbents, aerosol propellants, air displacement agents, alkalizing agents, anticaking agents, anticoagulants, antimicrobial preservatives, antioxidants, antiseptics, bases, binders, buffering agents, chelating agents, coating agents, coloring agents, desiccants, detergents, diluents, disinfectants, disintegrants, dispersing agents, dissolution enhancing agents, dyes, emollients, emulsifying agents, emulsion stabilizers, fillers, film forming agents, flavor enhancers, flavoring agents, flow enhancers, gelling agents, granulating agents, humectants, lubricants, mucoadhesives, ointment bases, ointments, oleaginous vehicles, organic bases, pastille bases, pigments, plasticizers, polishing agents, preservatives, sequestering agents, skin penetrants, solubilizing agents, solvents, stabilizing agents, suppository bases, surface active agents, surfactants, suspending agents, sweetening agents, therapeutic agents, thickening agents, tonicity agents, toxicity agents, viscosity-increasing agents, water-absorbing agents, water-miscible cosolvents, water softeners, or wetting agents. *See, e.g.,* the Handbook of Pharmaceutical Excipients, Third Edition, A. H. Kibbe (Pharmaceutical Press, London, UK, 2000), which is incorporated by reference in its entirety. Remington's Pharmaceutical Sciences, Sixteenth Edition, E. W. Martin (Mack Publishing Co., Easton, Pa., 1980), which is incorporated by reference in its entirety.

In various aspects, the pharmaceutical composition comprises formulation materials that are nontoxic to recipients at the dosages and concentrations employed. In specific embodiments, pharmaceutical compositions comprising an active agent and one or more pharmaceutically acceptable salts; polyols; surfactants; osmotic balancing agents; tonicity agents; anti-oxidants; antibiotics; antimycotics; bulking agents; lyoprotectants; anti-foaming agents; chelating agents; preservatives; colorants; analgesics; or additional pharmaceutical agents. In various aspects, the pharmaceutical composition comprises one or more polyols and/or one or more surfactants, optionally, in addition to one or more excipients, including but not limited to, pharmaceutically acceptable salts; osmotic balancing agents (tonicity agents); anti-oxidants; antibiotics; antimycotics; bulking agents; lyoprotectants; anti-foaming agents; chelating agents; preservatives; colorants; and analgesics.

In certain embodiments, the pharmaceutical composition can contain formulation materials for modifying, maintaining or preserving, for example, the pH, osmolarity, viscosity, clarity, color, isotonicity, odor, sterility, stability, rate of dissolution or release, adsorption or penetration of the composition. In such embodiments, suitable formulation materials include, but are not limited to, amino acids (such as glycine, glutamine, asparagine, arginine or lysine); antimicrobials; antioxidants (such as ascorbic acid, sodium sulfite or sodium hydrogen-sulfite); buffers (such as borate, bicarbonate, Tris-HCI, citrates, phosphates or other organic acids); bulking agents (such as mannitol or glycine); chelating agents (such as ethylenediamine tetraacetic acid (EDTA)); complexing agents (such as caffeine, polyvinylpyrrolidone, beta-cyclodextrin or hydroxypropyl-beta-cyclodextrin); fillers; monosaccharides; disaccharides; and other carbohydrates (such as glucose, mannose or dextrins); proteins (such as serum albumin, gelatin or immunoglobulins); coloring, flavoring and diluting agents; emulsifying agents; hydrophilic polymers (such as polyvinylpyrrolidone); low molecular weight polypeptides; salt-forming counterions (such as sodium); preservatives (such as bcnzalkonium chloride, benzoic acid, salicylic acid, thimerosal, phenethyl alcohol, methylparaben, propylparaben, chlorhexidine, sorbic acid or hydrogen peroxide); solvents (such as glycerin, propylene glycol or polyethylene glycol); sugar alcohols (such as mannitol or sorbitol); suspending agents; surfactants or wetting agents (such as pluronics, PEG, sorbitan esters, polysorbates such as polysorbate 20, polysorbatc, triton, tromethamine, lecithin, cholesterol, tyloxapal); stability enhancing agents (such as sucrose or sorbitol); tonicity enhancing agents (such as alkali metal halides, preferably sodium or potassium chloride, mannitol sorbitol); delivery vehicles; diluents; excipients and/or pharmaceutical adjuvants. See, REMINGTON'S PHARMACEUTICAL SCIENCES, 18" Edition, (A. R. Genrmo, ed.), 1990, Mack Publishing Company.

The pharmaceutical compositions can be formulated to achieve a physiologically compatible pH. In some embodiments, the pH of the pharmaceutical composition can be for example between about 4 or about 5 and about 8.0 or about 4.5 and about 7.5 or about 5.0 to about 7.5. In various embodiments, the pH of the pharmaceutical composition is between 5.5 and 7.5.

The present disclosure provides methods of producing a pharmaceutical composition. In various aspects, the method comprises combining the antigen-binding protein, conjugate, fusion protein, nucleic acid, vector, host cell, or a combination thereof, with a pharmaceutically acceptable carrier, diluent, or excipient.

### Routes of Administration

With regard to the present disclosure, the active agent, or pharmaceutical composition comprising the same, can be administered to the subject via any suitable route of administration. For example, the active agent can be administered to a subject via parenteral, nasal, oral, pulmonary, topical, vaginal, or rectal administration. The following discussion on routes of administration is merely provided to illustrate various embodiments and should not be construed as limiting the scope in any way.

Formulations suitable for parenteral administration include aqueous and non-aqueous, isotonic sterile injection solutions, which can contain anti-oxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The term, "parenteral" means not through the alimentary canal but by some other route such as subcutaneous, intramuscular, intraspinal, or intravenous. The active agent of the present disclosure can be administered with a physiologically acceptable diluent in a pharmaceutical carrier, such as a sterile liquid or mixture of liquids, including water, saline, aqueous dextrose and related sugar solutions, an alcohol, such as ethanol or hexadecyl alcohol, a glycol, such as propylene glycol or polyethylene glycol, dimethylsulfoxide, glycerol, ketals such as 2,2- dimethyl-153-dioxolane-4-methanol, ethers, poly(ethyleneglycol) 400, oils, fatty acids, fatty acid esters or glycerides, or acetylated fatty acid glycerides with or without the addition of a pharmaceutically acceptable surfactant, such as a soap or a detergent, suspending agent, such as pectin, carbomers, methylcellulose, hydroxypropylmethylcellulose, or carboxymethylcellulose, or emulsifying agents and other pharmaceutical adjuvants.

Oils, which can be used in parenteral formulations include petroleum, animal, vegetable, or synthetic oils. Specific examples of oils include peanut, soybean, sesame, cottonseed, corn, olive, petrolatum, and mineral. Suitable fatty acids for use in parenteral formulations include oleic acid, stearic acid, and isostearic acid. Ethyl oleate and isopropyl myristate are examples of suitable fatty acid esters.

Suitable soaps for use in parenteral formulations include fatty alkali metal, ammonium, and triethanolamine salts, and suitable detergents include (a) cationic detergents such as, for example, dimethyl dialkyl ammonium halides, and alkyl pyridinium halides, (b) anionic detergents such as, for example, alkyl, aryl, and olefin sulfonates, alkyl, olefin, ether, and monoglyceride sulfates, and sulfosuccinates, (c) nonionic detergents such as, for example, fatty amine oxides, fatty acid alkanolamides, and polyoxyethylenepolypropylene copolymers, (d) amphoteric detergents such as, for example, alkyl-β-aminopropionates, and 2-alkyl -imidazoline quaternary ammonium salts, and (e) mixtures thereof.

The parenteral formulations in some embodiments contain from about 0.5% to about 25% by weight of the active agent of the present disclosure in solution. Preservatives and buffers can be used. In order to minimize or eliminate irritation at the site of injection, such compositions can contain one or more nonionic surfactants having a hydrophile-lipophile balance (HLB) of from about 12 to about 17. The quantity of surfactant in such formulations will typically range from about 5% to about 15% by weight. Suitable surfactants include polyethylene glycol sorbitan fatty acid esters, such as sorbitan monooleate and the high molecular weight adducts of ethylene oxide with a hydrophobic base, formed by the condensation of propylene oxide with propylene glycol. The parenteral formulations in some aspects are presented in unit-dose or multi-dose sealed containers, such as ampoules and vials, and can be stored in a freeze-dried (lyophilized) condition requiring only the addition of the sterile liquid excipient, for example, water, for injections, immediately prior to use. Extemporaneous injection solutions and suspensions in some aspects are prepared from sterile powders, granules, and tablets of the kind previously described.

Injectable formulations are in accordance with the present disclosure. The requirements for effective pharmaceutical carriers for injectable compositions are well-known to those of ordinary skill in the art (see, e.g., Pharmaceutics and Pharmacy Practice, J. B. Lippincott Company, Philadelphia, PA, Banker and Chalmers, eds., pages 238-250 (1982), and ASHP Handbook on Injectable Drugs, Toissel, 4th ed., pages 622-630 (1986)).

### Dosages

The active agents of the disclosure are believed to be useful in methods of inhibiting tumor growth, as well as other methods, as further described herein, including methods of treating or preventing cancer. For purposes of the disclosure, the amount or dose of the active agent administered should be sufficient to effect, e.g., a therapeutic or prophylactic response, in the subject or animal over a reasonable time frame. For example, the dose of the active agent of the present disclosure should be sufficient to treat cancer as described herein in a period of from about 1 to 4 minutes, 1 to 4 hours or 1 to 4 weeks or longer, e.g., 5 to 20 or more weeks, from the time of administration. In certain embodiments, the time period could be even longer. The dose will be determined by the efficacy of the particular active agent and the condition of the animal (e.g., human), as well as the body weight of the animal (e.g., human) to be treated.

Many assays for determining an administered dose are known in the art. For purposes herein, an assay, which comprises comparing the extent to which cancer is treated upon administration of a given dose of the active agent of the present disclosure to a mammal among a set of mammals, each set of which is given a different dose of the active agent, could be used to determine a starting dose to be administered to a mammal. The extent to which cancer is treated upon administration of a certain dose can be represented by, for example, the extent of tumor regression achieved with the active agent in a mouse xenograft model. Methods of assaying tumor regression are known in the art and described herein in EXAMPLES.

The dose of the active agent of the present disclosure also will be determined by the existence, nature and extent of any adverse side effects that might accompany the administration of a particular active agent of the present disclosure. Typically, the attending physician will decide the dosage of the active agent of the present disclosure with which to treat each individual patient, taking into consideration a variety of factors, such as age, body weight, general health, diet, sex, active agent of the present disclosure to be administered, route of administration, and the severity of the condition being treated. By way of example and not intending to limit the present disclosure, the dose of the active agent of the present disclosure can be about 0.0001 to about 1 g/kg body weight of the subject being treated/day, from about 0.0001 to about 0.001 g/kg body weight/day, or about 0.01 mg to about 1 g/kg body weight/day.

### Controlled Release Formulations

In some embodiments, the active agents described herein can be modified into a depot form, such that the manner in which the active agent of the present disclosure is released into the body to which it is administered is controlled with respect to time and location within the body (see, for example, U.S. Patent No. 4,450,150). Depot forms of active agents of the present disclosure can be, for example, an implantable composition comprising the active agents and a porous or non-porous material, such as a polymer, wherein the active agent is encapsulated by or diffused throughout the material and/or degradation of the non-porous material. The depot is then implanted into the desired location within the body of the subject and the active agent is released from the implant at a predetermined rate.

The pharmaceutical composition comprising the active agent in certain aspects is modified to have any type of *in vivo* release profile. In some aspects, the pharmaceutical composition is an immediate release, controlled release, sustained release, extended release, delayed release, or bi-phasic release formulation. Methods of formulating peptides for controlled release are known in the art. See, for example, Qian et al., J Pharm 374: 46-52 (2009) and International Patent Application Publication Nos. WO 2008/130158, WO2004/033036; WO2000/032218; and WO 1999/040942.

The instant compositions can further comprise, for example, micelles or liposomes, or some other encapsulated form, or can be administered in an extended release form to provide a prolonged storage and/or delivery effect.

### Use

The antigen-binding proteins of the present disclosure are useful for inhibiting tumor growth. Without being bound to a particular theory, the inhibiting action of the antigen-binding proteins provided herein allow such entities to be useful in methods of treating cancer.

Accordingly, provided herein are methods of inhibiting tumor growth in a subject and methods of reducing tumor size in a subject. In various embodiments, the methods comprise administering to the subject the pharmaceutical composition of the present disclosure in an amount effective for inhibiting tumor growth or reducing tumor size in the subject. In various aspects, the growth of an ovarian tumor, melanoma tumor, bladder tumor, or endometrial tumor is inhibited. In various aspects, the size of an ovarian tumor, melanoma tumor, bladder tumor, or endometrial tumor is reduced.

As used herein, the term "inhibit" or "reduce" and words stemming therefrom may not be a 100% or complete inhibition or reduction. Rather, there are varying degrees of inhibition or reduction of which one of ordinary skill in the art recognizes as having a potential benefit or therapeutic effect. In this respect, the antigen-binding proteins of the present disclosure may inhibit tumor growth or reduce tumor size to any amount or level. In various embodiments, the inhibition provided by the methods of the present disclosure is at least or about a 10% inhibition (e.g., at least or about a 20% inhibition, at least or about a 30% inhibition, at least or about a 40% inhibition, at least or about a 50% inhibition, at least or about a 60% inhibition, at least or about a 70% inhibition, at least or about a 80% inhibition, at least or about a 90% inhibition, at least or about a 95% inhibition, at least or about a 98% inhibition). In various embodiments, the reduction provided by the methods of the present disclosure is at least or about a 10% reduction (e.g., at least or about a 20% reduction, at least or about a 30% reduction, at least or about a 40% reduction, at least or about a 50% reduction, at least or about a 60% reduction, at least or about a 70% reduction, at least or about a 80% reduction, at least or about a 90% reduction, at least or about a 95% reduction, at least or about a 98% reduction).

Additionally provided herein are methods of treating a subject with cancer, e.g., CLDN6-expressing cancer. In various embodiments, the method comprises administering to the subject the pharmaceutical composition of the present disclosure in an amount effective for treating the cancer in the subject.

For purposes herein, the cancer of the methods disclosed herein can be any cancer, e.g., any malignant growth or tumor caused by abnormal and uncontrolled cell division that may spread to other parts of the body through the lymphatic system or the blood stream. The cancer in some aspects is one selected from the group consisting of acute lymphocytic cancer, acute myeloid leukemia, alveolar rhabdomyosarcoma, bone cancer, brain cancer, breast cancer, cancer of the anus, anal canal, or anorectum, cancer of the eye, cancer of the intrahepatic bile duct, cancer of the joints, cancer of the neck, gallbladder, or pleura, cancer of the nose, nasal cavity, or middle ear, cancer of the oral cavity, cancer of the vulva, chronic lymphocytic leukemia, chronic myeloid cancer, colon cancer, esophageal cancer, cervical cancer, gastrointestinal carcinoid tumor, Hodgkin lymphoma, hypopharynx cancer, kidney cancer, larynx cancer, liver cancer, lung cancer, malignant mesothelioma, melanoma, multiple myeloma, nasopharynx cancer, non-Hodgkin lymphoma, ovarian cancer, pancreatic cancer, peritoneum, omentum, and mesentery cancer, pharynx cancer, prostate cancer, rectal cancer, renal cancer (e.g., renal cell carcinoma (RCC)), small intestine cancer, soft tissue cancer, stomach cancer, testicular cancer, thyroid cancer, ureter cancer, and urinary bladder cancer. In particular aspects, the cancer is selected from the group consisting of: head and neck, ovarian, cervical, bladder and oesophageal cancers, pancreatic, gastrointestinal cancer, gastric, breast, endometrial and colorectal cancers, hepatocellular carcinoma, glioblastoma, bladder, lung cancer, e.g., non-small cell lung cancer (NSCLC), bronchioloalveolar carcinoma. In various aspects, the cancer is ovarian cancer, melanoma, bladder cancer, lung cancer, liver cancer, endometrial cancer. In various aspects, the cancer is any cancer characterized by moderate to high expression of CLDN6. See, e.g., Figures 1-3. In various aspects, the cancer is acute myeloid leukemia, large B-cell lymphoma, stomach cancer, prostate cancer, melanoma, colon cancer, rectal cancer, bladder cancer, cervical cancer, liver cancer, breast cancer, kidney clear cell carcinoma, head and neck cancer, sarcoma, kidney chromophobe cancer, lower grade glioma, adrenocortical cancer, glioblastoma, kidney papillary cell carcinoma, lung squamous cell carcinoma, thyroid cancer, lung adenocarcinoma, pancreatic cancer, endometroid cancer, uterine carcinsarcoma, or ovarian cancer. In various aspects, the cancer is selected from ovarian cancer, endometrioid cancer, uterine cancer, lung cancer, gastric cancer, breast cancer Head and Neck Squamous Cell Carcinoma (HNSCC) cancer, cervical cancer, and bladder.

As used herein, the term "treat," as well as words related thereto, do not necessarily imply 100% or complete treatment. Rather, there are varying degrees of treatment of which one of ordinary skill in the art recognizes as having a potential benefit or therapeutic effect. In this respect, the methods of treating cancer of the present disclosure can provide any amount or any level of treatment. Furthermore, the treatment provided by the method of the present disclosure can include treatment of one or more conditions or symptoms or signs of the cancer being treated. Also, the treatment provided by the methods of the present disclosure can encompass slowing the progression of the cancer. For example, the methods can treat cancer by virtue of enhancing the T cell activity or an immune response against the cancer, reducing tumor or cancer growth, reducing metastasis of tumor cells, increasing cell death of tumor or cancer cells, and the like. In various aspects, the methods treat by way of delaying the onset or recurrence of the cancer by at least 1 day, 2 days, 4 days, 6 days, 8 days, 10 days, 15 days, 30 days, two months, 3 months, 4 months, 6 months, 1 year, 2 years, 3 years, 4 years, or more. In various aspects, the methods treat by way increasing the survival of the subject.

The antigen binding proteins of the present disclosure also may be used to detect CLDN6 in a sample or diagnose a CLDN6-positive cancer. Therefore, the present disclosure provides methods of detecting Claudin6 (CLDN6) in a sample. In various embodiments, the method comprises contacting the sample with an antigen-binding protein, a conjugate, or a fusion protein, as described herein, and assaying for an immunocomplex comprising the antigen-binding protein, conjugate or fusion protein bound to CLDN6. The present disclosure also provides methods of diagnosing a Claudin6 (CLDN6)-positive cancer in a subject. In various embodiments, the method comprises contacting a biological sample comprising cells or tissue obtained from the subject with an antigen-binding protein, a conjugate, or a fusion protein, as described herein, and assaying for an immunocomplex comprising the antigen-binding protein, conjugate or fusion protein bound to CLDN6.

### Subjects

In some embodiments of the present disclosure, the subject is a mammal, including, but not limited to, mammals of the order Rodentia, such as mice and hamsters, and mammals of the order Logomorpha, such as rabbits, mammals from the order Carnivora, including Felines (cats) and Canines (dogs), mammals from the order Artiodactyla, including Bovines (cows) and Swines (pigs) or of the order Perssodactyla, including Equines (horses). In some aspects, the mammals are of the order Primates, Ceboids, or Simoids (monkeys) or of the order Anthropoids (humans and apes). In some aspects, the mammal is a human.

### Kits

In some embodiments, the antigen-binding proteins of the present disclosure are provided in a kit. In various aspects, the kit comprises the antigen-binding protein(s) as a unit dose. For purposes herein "unit dose" refers to a discrete amount dispersed in a suitable carrier. In various aspects, the unit dose is the amount sufficient to provide a subject with a desired effect, e.g., inhibition of tumor growth, reduction of tumor size, treatment of cancer. Accordingly, provided herein are kits comprising an antigen-binding protein of the present disclosure optionally provided in unit doses. In various aspects, the kit comprises several unit doses, e.g., a week or month supply of unit doses, optionally, each of which is individually packaged or otherwise separated from other unit doses. In some embodiments, the components of the kit/unit dose are packaged with instructions for administration to a patient. In some embodiments, the kit comprises one or more devices for administration to a patient, e.g., a needle and syringe, and the like. In some aspects, the antigen-binding protein of the present disclosure, a pharmaceutically acceptable salt thereof, a conjugate comprising the antigen-binding protein, or a multimer or dimer comprising the antigen-binding protein, is pre-packaged in a ready to use form, e.g., a syringe, an intravenous bag, etc. In some aspects, the kit further comprises other therapeutic or diagnostic agents or pharmaceutically acceptable carriers (e.g., solvents, buffers, diluents, etc.), including any of those described herein. In particular aspects, the kit comprises an antigen-binding protein of the present disclosure, along with an agent, e.g., a therapeutic agent, used in chemotherapy or radiation therapy.

### Various embodiments

In various embodiments of the present disclosure, the antigen-binding protein binds to a human Claudin6 (CLDN6) protein (SEQ ID NO: 200), wherein (a) the antigen-binding protein binds *to* Extracellular Loop 2 (EL2) of an extracellular domain (ECD) of CLDN6 and does not bind to Extracellular Loop 1 (EL1) of the ECD of CLDN6; or (b) does not bind to any of Claudin3 (CLDN3), Claudin4 (CLDN4), and Claudin9 (CLDN9) and inhibits binding of a reference antibody to CLDN6 endogenously expressed by OVCA429 cells with less than about 1200 nM; or (c) a combination thereof. In various instances, the antigen- binding protein binds to an epitope within the amino acid sequence of WTAHAIIRDFYNPLVAEAQKREL (SEQ ID NO: 2), or binds to the amino acid sequence of TAHAIIRDFYNPL (SEQ ID NO: 3) or LVAEAQKREL (SEQ ID NO: 4) of CLDN 6. In various aspects, the antigen-binding protein does not bind to any one or more of Claudin3 (CLDN3), Claudin4 (CLDN4), and Claudin9 (CLDN9). In various instances, the antigen-binding protein does not bind to CLDN3. In various instances, the antigen-binding protein binds to CLDN6, CLDN4, and CLDN9 but does not bind to CLDN3. In various instances, the antigen-binding protein binds to CLDN6 and CLDN4 but does not bind to CLDN3 or CLDN9. In various aspects, the antigen-binding protein binds to CLDN6 and CLDN9 but does not bind to CLDN3 or CLDN4.

In various instances, the antigen-binding protein of the present disclosure inhibits binding of a reference antibody to CLDN6 endogenously expressed by OVCA429 cells with less than about 1200 nM and the reference antibody comprises a light chain variable sequence of SEQ ID NO: 181 and a heavy chain variable sequence of SEQ ID NO: 182 or a light chain variable sequence of SEQ ID NO: 185 and a heavy chain variable sequence of SEQ ID NO: 186. In various aspects, the antigen-binding protein of the present disclosure inhibits binding of a reference antibody to CLDN6 endogenously expressed by OVCA429 cells with less than about 1000 nM or less than 750 nM (e.g., less than about 500 nM, less than about 250 nM, less than aobut 100 nM) and the reference antibody comprises a light chain variable sequence of SEQ ID NO: 181 and a heavy chain variable sequence of SEQ ID NO: 182 or a light chain variable sequence of SEQ ID NO: 185 and a heavy chain variable sequence of SEQ ID NO: 186.

In various embodiments, the antigen-binding protein comprises (a) a heavy chain CDR1 amino acid sequence set forth in Table A or A1 or a sequence selected from the group consisting of: SEQ ID NOs: 11, 17, 23, 29, 35, 41, 47, 53, 59, 65, 71, 77, 83, 89, 95, 101, 107, 113, 119, 125, 131, 452, 455, 461, 465, and 472, or a variant sequence thereof which differs by only one or two amino acids or which has at least or about 70% (e.g., at least or about 85%, at least or about 90%) sequence identity; (b) a heavy chain CDR2 amino acid sequence set forth in Table A or A1 or a sequence selected from the group consisting of: SEQ ID NOs: 12, 18, 24, 30, 36, 42, 48, 54, 60, 66, 72, 78, 84, 90, 86, 102, 108, 114, 120, 126, 132, 475, 456, 462, 466, 468, and 473, or a variant sequence thereof which differs by only one or two amino acids or which has at least or about 70% (e.g., at least or about 85%, at least or about 90%) sequence identity; (c) a heavy chain CDR3 amino acid sequence set forth in Table A or A1 or a sequence selected from the group consisting of: SEQ ID NOs: 13, 19, 25, 31, 37, 43, 49, 55, 61, 67, 73, 79, 85, 91, 97, 103, 109, 115, 121, 127, 133, 453, 457, 463, 467, 469, and 474, or a variant sequence thereof which differs by only one or two amino acids or which has at least or about 70% (e.g., at least or about 85%, at least or about 90%) sequence identity; (d) a light chain CDR1 amino acid sequence set forth in Table A or A1 or a sequence selected from the group consisting of: SEQ ID NOs: 8, 14, 20, 32, 38, 44, 50, 56, 62, 68, 74, 80, 86, 92, 98, 104, 110, 116, 122, 128, 449, 476, 458, 464, and 470, or a variant sequence thereof which differs by only one or two amino acids or which has at least or about 70% (e.g., at least or about 85%, at least or about 90%) sequence identity; (e) a light chain CDR2 amino acid sequence set forth in Table A or A1 or a sequence selected from the group consisting of: SEQ ID NOs: 9, 15, 21, 27, 33, 39, 45, 51, 57, 63, 69, 75, 81, 87, 93, 99, 105, 111, 117, 123, 129, 450, 477, 459, and 471, or a variant sequence thereof which differs by only one or two amino acids or which has at least or about 70% (e.g., at least or about 85%, at least or about 90%) sequence identity; (f) a light chain CDR3 amino acid sequence set forth in Table A or A1 or a sequence selected from the group consisting of: SEQ ID NOs: 10, 16, 22, 28, 34, 40, 46, 52, 58, 64, 70, 76, 82, 88, 94, 100, 106, 112, 118, 124, 130, 451, 454, and 460, or a variant sequence thereof which differs by only one or two amino acids or which has at least or about 70% (e.g., at least or about 85%, at least or about 90%) sequence identity; (g) a combination of any two or more of (a)-(f).

In various aspects, the antigen-binding protein comprises a light chain CDR1 amino acid sequence, a light chain CDR2 amino acid sequence, and a light chain CDR3 amino acid sequence set forth in Table A or A1 and 1 or 2 of the heavy chain CDR amino acid sequences set forth in Table A or A1. In some instances, the antigen-binding protein comprises a heavy chain CDR1 amino acid sequence, a heavy chain CDR2 amino acid sequence, and a heavy chain CDR3 amino acid sequence set forth in Table A or A1 and 1 or 2 of the light chain CDR amino acid sequences set forth in Table A or A1. In various aspects, the antigen-binding protein comprises six CDR amino acid sequences selected from the group consisting of: (a) SEQ ID NOs: 74-79; (b) SEQ ID NOs: 50-55; (c) SEQ ID NOs: 122-127; (d) SEQ ID NOs: 26-31; (e) SEQ ID NOs: 128-133; (f) SEQ ID NOs: 38-43; (g) SEQ ID NOs: 62-67; (h) SEQ ID NOs: 80-85; (i) SEQ ID NOs: 44-49; (j) SEQ ID NOs: 86-91; (k) SEQ ID NOs: 104-109; (l) SEQ ID NOs: 56-61; (m) SEQ ID NOs: 32-37; (n) SEQ ID NOs: 110-115; (o) SEQ ID NOs: 98-103; (p) SEQ ID NOs: 92-97; (q) SEQ ID NOs: 116-121; (r) SEQ ID NOs: 8-13; (t) SEQ ID NOs: 68-73; (u) SEQ ID NOs: 14-19; (v) SEQ ID NOs: 20-25, (v) SEQ I D NOs: 449-453 and 475; (w) SEQ ID NOs: 476-477, 454-457, (x) SEQ ID NOs: 458-463; (y) SEQ ID NOs: 57, 58, 464-467; (z) SEQ ID NOs: 68-71 and 468-469; and (aa) SEQ ID NOs: 112, and 470-474. In various aspects, the antigen-binding protein of comprises (a) a heavy chain variable region amino acid sequence set forth in in Table B or a sequence selected from the group consisting of: SEQ ID NOs: 135, 137, 139, 141, 143, 145, 147, 149, 151, 153, 155, 157, 159, 161, 163, 165, 167, 169, 171, 173, and 175, or a variant sequence thereof which differs by only one or two amino acids or which has at least or about 70% (e.g., at least or about 85%, at least or about 90%) sequence identity; or (b) a light chain variable region amino acid sequence set forth in in Table B or a sequence selected from the group consisting of: SEQ ID NOs: 134, 136, 138, 140, 142, 144, 146, 148, 150, 152, 154, 156, 158, 160, 162, 164, 166, 168, 170, 172, 174, and 176, or a variant sequence thereof which differs by only one or two amino acids or which has at least or about 70% (e.g., at least or about 85%, at least or about 90%) sequence identity; or both (a) and (b). In various aspects, the antigen-binding protein comprises a pair of amino acid sequences selected from the group consisting of: (a) SEQ ID NOs: 156 and 157; (b) SEQ ID NOs: 148 and 149; (c) SEQ ID NOs: 172 and 173; (d) SEQ ID NOs: 140 and 141; (e) SEQ ID NOs: 174 and 175; (f) SEQ ID NOs: 144 and 145; (g) SEQ ID NOs: 152 and 153; (h) SEQ ID NOs: 158 and 159; (i) SEQ ID NOs: 146 and 147; (j) SEQ ID NOs: 160 and 161; (k) SEQ ID NOs: 166 and 167; (l) SEQ ID NOs: 150 and 151; (m) SEQ ID NOs: 142 and 143; (n) SEQ ID NOs: 168 and 169; (o) SEQ ID NOs: 164 and 165; (p) SEQ ID NOs: 162 and 163; (q) SEQ ID NOs: 170 and 171; (r) SEQ ID NOs: 134 and 135; (s) SEQ ID NOs: 154 and 155; (t) SEQ ID NOs: 136 and 137; and (u) SEQ ID NOs: 138 and 139.

In various embodiments, the antigen-binding protein comprises (a) a heavy chain variable region amino acid sequence set forth in in Table B1 or C or a sequence selected from the group consisting of: SEQ ID NOs: 376-379, 384-387, 391-396, 403-408, 412, 413, 416-419, 422-427, 478, 480, 482, 484, 486, and 488 or a variant sequence thereof which differs by only one or two amino acids or which has at least or about 70%, or about 80%, or about 90%, or about 95% sequence identity; or (b) a light chain variable region amino acid sequence set forth in Table B1 or C or a sequence selected from the group consisting of: SEQ ID NOs: 380-383, 388-390, 397-402, 409-411, 414, 415, 420, 421, and 479, 481, 483, 485, 487, and 489 or a variant sequence thereof which differs by only one or two amino acids or which has at least or about 70%, or about 80%, or about 90%, or about 95% sequence identity; or (c) both (a) and (b). In various aspects, the antigen-binding protein comprises a pair of amino acid sequences as listed in Table D.

The present disclosure provides an antigen-binding protein comprising: (A) an HC CDR1 comprising the amino acid sequence of YTFTXYT, wherein X is T, V, D, or S (SEQ ID NO: 452), optionally, comprising the amino acid sequence of YTFTTYT (SEQ ID NO: 11); (B) an HC CDR2 comprising the amino acid sequence of IXPSSGYT, wherein X is Q, S, A, or N (SEQ ID NO: 475), optionally, comprising the amino acid sequence of INPSSGYT (SEQ ID NO: 12); (C) an HC CDR3 comprising the amino acid sequence of AXGDYYVAY, wherein X is N, Q, H, or D (SEQ ID NO: 453), optionally, comprising the amino acid sequence of ANGDYYVAY (SEQ ID NO:13); (D) an LC CDR1 comprising the amino acid sequence of SSVSSXY, wherein X is T, V, F, or D (SEQ ID NO: 449), optionally, comprising the amino acid sequence of SSVSSTY (SEQ ID NO: 8); (E) an LC CDR2 comprising the amino acid sequence of XTX, wherein X at position 1 is S, T, Q, or A and X at position 3 is S, T, D, or Q (SEQ ID NO: 450), optionally, comprising the amino acid sequence of STS (SEQ ID NO: 9); and (F) an LC CDR3 comprising the amino acid sequence of HXYXRSPLT, wherein X at position 2 is Q, H, or S and X at position 4 is H, Y, Q, or S (SEQ ID NO: 451), optionally, comprising the amino acid sequence of HQYHRSPLT (SEQ ID NO: 10).

An antigen-binding protein comprising: (A) an HC CDR1 comprising the amino acid sequence of FTFSXYX, wherein X at position 5 is N, S, R, Q, or A and X at position 7 is W, H, Y, F (SEQ ID NO: 455), optionally, comprising the amino acid sequence of FTFSNYW (SEQ ID NO: 23); (B) an HC CDR2 comprising the amino acid sequence of IRLKXDXYAT, wherein X at position 5 is S, N, A, or T and X at position 7 is Q, S, A, N (SEQ ID NO: 456), optionally, comprising the amino acid sequence of IRLKSDNYAT (SEQ ID NO: 24); (C) an HC CDR3 comprising the amino acid sequence of XDGPPSGX, wherein X at position 1 is N, D, or T and X at position 8 is S, T, A, C, or Y (SEQ ID NO: 457), optionally, comprising the amino acid sequence of NDGPPSGC (SEQ ID NO: 25); (D) an LC CDR1 comprising the amino acid sequence of EXIYSY, wherein X is Q, S, A, D, or N (SEQ ID NO: 476), optionally, comprising the amino acid sequence of ENIYSY (SEQ ID NO: 20); (E) an LC CDR2 comprising the amino acid sequence of XAK, wherein X at position 1 is Q, S, A, D, or N (SEQ ID NO: 477), optionally, comprising the amino acid sequence of NAK (SEQ ID NO: 21); and (F) an LC CDR3 comprising the amino acid sequence of QXHYXVPWT, wherein X at position 2 is H, Q, S, or T and X at position 5 is T, S, N, or G (SEQ ID NO: 454), optionally, comprising the amino acid sequence of QHHYTVPWT (SEQ ID NO: 22).

An antigen-binding protein comprising: (A) an HC CDR1 comprising the amino acid sequence of YTXTXYT, wherein X at position 3 is F, Y, S, or T and X at position 5 is S, T, Y, or D (SEQ ID NO: 461), optionally, comprising the amino acid sequence of YTFTSYT (SEQ ID NO: 29); (B) an HC CDR2 comprising the amino acid sequence of IXPSSXYT, wherein X at position 2 is Q, S, A, or N and X at position 6 is T, S, V, D, or G (SEQ ID NO: 462), optionally, comprising the amino acid sequence of INPSSTYT (SEQ ID NO: 30); (C) an HC CDR3 comprising the amino acid sequence of XRGEXGGFAY, wherein X at position 1 is S, A, T, or V and X at position 5 is L, V, or F (SEQ ID NO: 463), optionally, comprising the amino acid sequence of SRGELGGFAY (SEQ ID NO: 31); (D) an LC CDR1 comprising the amino acid sequence of QSLVHSXGXTY, wherein X at position 7 is D, N, E, Q, S, or A and X at position 9 is Q, S, A, D, or N (SEQ ID NO: 458), optionally, comprising the amino acid sequence of QSLVHSDGNTY (SEQ ID NO: 26); (E) an LC CDR2 comprising the amino acid sequence of XVX, wherein X at position 1 is K, Q, or R and X at position 3 is S, T, or V (SEQ ID NO: 459), optionally, comprising the amino acid sequence of KVS (SEQ ID NO: 27); and (F) an LC CDR3 comprising the amino acid sequence of SXXTHVPYT, wherein X at position 2 is Q, H, or T and X at position 3 is S, G, T, or D (SEQ ID NO: 460), optionally, comprising the amino acid sequence of SQSTHVPYT (SEQ ID NO: 28).

In various aspects, the antigen-binding protein of the present disclosure is an antibody, e.g., a monoclonal antibody. In various instances, the antigen-binding protein is an IgG. In various aspects, the antigen-binding protein inhibits at least about 50% colony growth in a soft agar 3D proliferation assays or inhibits tumor growth in xenograft mice injected with human cancer cells. In various aspects, the antigen-binding protein inhibits tumor growth of in xenograft mice injected with ovarian cancer cells, melanoma cancer cells, bladder cancer cells, or endometrial cancer cells. In various instances, the antigen-binding protein inhibits at least 50% tumor growth in xenograft mice injected with ovarian cancer cells, bladder cancer cells, or endometrial cancer cells.

The present disclosure provides a conjugate comprising an antigen-binding protein described herein and a heterologous moiety. The present disclosure also provides a fusion protein comprising an antigen-binding protein described herein. The present disclosure further provides a nucleic acid comprising a nucleotide sequence encoding an antigen binding protein, a conjugate, or a fusion protein, of the present disclosure. The present disclosure provides a vector comprising the nucleic acid comprising a nucleotide sequence encoding an antigen binding protein, a conjugate, or a fusion protein, of the present disclosure. The present disclosure additionally provides a host cell comprising the nucleic acid or the vector of the present disclosure.

The present disclosure provides a method of producing an antigen-binding protein that binds to a Claudin6 (CLDN6) protein, comprising (i) culturing the host cell of the present disclosure in a cell culture medium, wherein the host cell comprises a nucleic acid comprising a nucleotide sequence encoding an antigen binding protein of any one of the previous claims, and (ii) harvesting the antigen-binding protein from the cell culture medium. Also, provided is a method of producing a fusion protein comprising an antigen-binding protein that binds to a Claudin6 (CLDN6) protein, comprising (i) culturing the host cell of the present disclosure in a cell culture medium, wherein the host cell comprises a nucleic acid comprising a nucleotide sequence encoding a fusion protein of the present disclosure, and (ii) harvesting the fusion protein from the cell culture medium.

The present disclosure furthermore provides a method of producing a pharmaceutical composition comprising combining an antigen-binding protein, a conjugate, a fusion protein, a nucleic acid, a vector, a host cell, of the present disclosure, or a combination thereof, and a pharmaceutically acceptable carrier, diluent or excipient. Also provided are pharmaceutical compositions comprising antigen-binding protein, a conjugate, a fusion protein, a nucleic acid, a vector, a host cell, of the present disclosure, or a combination thereof, and a pharmaceutically acceptable carrier, diluent or excipient.

Provided herein is a method of treating a subject with a CLDN6-expressing cancer comprising administering to the subject a pharmaceutical composition described herein in an amount effective to treat the cancer. Also provided is a method of inhibiting tumor growth in a subject, comprising administering to the subject a pharmaceutical composition described herein in an amount effective to inhibit tumor growth. The present disclosure provides a method of reducing tumor size in a subject, comprising administering to the subject a pharmaceutical composition described herein in an amount effective to reduce tumor size. Further provided is a method of preventing the recurrence of cancer in a subject, comprising administering to the subject a pharmaceutical composition described herein in an amount effective to prevent the recurrence of cancer.

The present disclosure provides a method of detecting Claudin6 (CLDN6) in a sample, comprising contacting the sample with an antigen-binding protein, a conjugate, or a fusion protein, of the present disclosure, and assaying for an immunocomplex comprising the antigen-binding protein, conjugate or fusion protein bound to CLDN6. Also provided herein is a method of diagnosing a Claudin6 (CLDN6)-positive cancer in a subject, comprising contacting a biological sample comprising cells or tissue obtained from the subject with an antigen-binding protein, a conjugate, or a fusion protein, of the present disclosure, and assaying for an immunocomplex comprising the antigen-binding protein, conjugate or fusion protein bound to CLDN6.

The present disclosure also provides a method of treating cancer in a subject diagnosed to be a low over-expresser of CLDN6. In various embodiments, the method comprises administering to the subject a presently disclosed pharmaceutical composition in an amount effective to prevent the recurrence of cancer. In some aspects, the administering induces apoptosis in tumor cells, optionally, the administering induces apoptosis in cells expressing CLDN6. In various aspects, the subject has a tumor and the tumor is semi-quantitatively categorized into one of four groups: high expressers, moderate expressers, low expressers, and non-expressers. In various instances, high expressers are defined as CLDN6 RNA greater than 12 log Fragments Per Kilobase Million (FPKM), wherein the CLDN6 RNA is measured by RNASeq, or CLDN6 protein levels are greater than 3+ as measured by immunohistochemistry (IHC). In various instances, moderate expressers are defined as CLDN6 RNA greater than 10 log FPKM, wherein the CLDN6 RNA is measured by RNASeq, or CLDN6 protein levels are greater than 2+ as measured by IHC. In various instances, low expressers are defined as CLDN6 RNA greater than 6 log FPKM, wherein the CLDN6 RNA is measured by RNASeq, or CLDN6 protein levels are greater than 1+ as measured by IHC. In various instances, non-expressers are defined as CLDN6 RNA less than than 6 log FPKM, wherein the CLDN6 RNA is measured by RNASeq, or CLDN6 protein levels are below IHC detection limits. In various aspects, the subject having said tumor is likewise described as a high expresser, moderate expresser, low expresser, or non-expresser of CLDN6.

The following examples are given merely to illustrate the present present disclosure and not in any way to limit its scope.

### EXAMPLES

### EXAMPLE 1

This example demonstrates an analysis of CLDN6 RNA levels in different cell and tissue sources.

In order to establish a baseline for expression of CLDN6 in different source materials, expression levels of CLDN6 expression in patient samples, normal tissue and cell lines created by the Translational Oncology Research laboratory (TORL) were assayed.

Levels of CLDN6 RNA in patient samples were measured using information contained in The Cancer Genome Atlas (TCGA) database managed by the National Cancer Institute (NCI). CLDN6 levels in normal tissue were measured using information in the Genotype-Tissue Expression (GTEX) database maintained by the Common Fund. The analysis of tissues from the GTEX database showed that CLDN6 is detectable in various sites, including the brain, pituitary, pancreas, kidney, lung, thyroid, and cervix, among other tissues (Figure 1).

CLDN6 expression levels were measured in TORL cancer cell lines using Agilent 44K microarrays (4x44K array chip, Agilent Technologies, Santa Clara, CA) and RNA sequencing (RNA-Seq) assays. RNASeq was performed by BGI Americas (Cambridge, MA) using their "RNASeq for quantification" service. As shown in Figures 2 and 3, ovarian, head and neck, lung, and bladder cancer cells expressed the highest levels of CLDN6, though CLDN6 expression levels were detectable in breast, kidney, colon, sarcoma and liver cancer cells.

### EXAMPLE 2

This example demonstrates the production of cells engineered to overexpress CLDN6.

Models engineered to overexpress CLDN6 were generated. These models were used to determine the efficacy of CLDN6 antibodies described in Example 5. Briefly, a nucleotide sequence encoding CLDN6 was engineered into a bicistronic vector having a CMV promoter and an attenuated Internal ribosome entry site (IRES) of encephalomyocarditis virus (EMCV). The IRES was located between the Gene of Interest (GOI) cDNA (CLDN6) and puromycin cDNA. A woodchuck posttranscriptional regulatory element (WPRE) was located downstream of the puromycin cDNA. The vector also expressed either a GFP marker sequence or a MycDDK tag. The sequence of the expression vector containing GFP is provided herein as SEQ ID NO: 189.

The expression vector was virally transduced into HEK293T cells (for screening purposes) and NIH3T3 cells (for immunizations). Positively transduced cells were selected based on survival in medium containing puromycin (1 µg/ml). The positive cells were subcloned to obtain a stable, uniform, clonal population of CLDN6 overexpressing cells.

Subclone CLDN6 expression was confirmed by flow cytometry using a reference CLDN6 monoclonal antibody (mAb) on a BD Biosciences Accuri^{™} flow cytometer (San Jose, CA). Secondary antibody and conjugate: Alexa Fluor@ 647 Goat anti-mouse IgG (minimal x-reactivity) antibody (Biolegend, San Diego, CA; Cat#405322) was used to detect binding activity between the reference CLDN6 mAb and the CLDN6 expressed by subclone.

Cellular localization of CLDN6 was determined by fluorescence microscopy using the Cellavista^{®} imaging system (Synentec (Mountain View, CA) with cells expressing a CLDN6-green fluorescence protein (GFP) fusion protein. As shown in Figure 4, fluorescence of the GFP was detected in the cell membrane, evidencing that CLDN6 localizes to the cell membrane.

### EXAMPLE 3

This example demonstrates the production of reference and control antibodies.

Benchmark (reference) CLDN6-specific antibodies and control antibodies were made by cloning the antibody heavy and light chain variable regions into the ExpiCHO^{™} expression system (ThermoFisher Scientific, Waltham, MA) to produce recombinant mouse IgG2A chimeric antibodies. These antibodies were tested alongside newly generated CLDN6 specific antibodies described in Example 5.

Briefly, plasmids containing the control and benchmark antibody sequences were transfected using the ExpiCHO^{™} Expression System (Catalog Number: A29133, ThermoFisher Scientific, USA) according to the manufacturer's protocol. The cells were cultured at 37º C and 8% CO₂ at day 1 and then at 32° C and 5% CO₂ post-transfection in media provided in the kit. Antibodies were purified by clarifying the ExpiCHO^{™} culture medium by centrifugation at 1,000g for 10 min followed by 5,000g for 30 min. The supernatant was then filtered using a 0.45µm filter followed by a 0.22µm filter. Subsequently, the supernatant was subjected to affinity purification using protein A/G resins (Life Technologies, Carlsbad, CA; Catalog# 20424) according to the manufacturer's protocol. Prior to ELISA purification, antibody titer in the culture medium was roughly determined to ensure the amount of medium loaded occupied less than 80% of the resin binding capacity. After incubation, the resins were washed with PBS and eluted with Elution Buffer (Life Technologies, Catalog# 21004). The elution fractions were immediately adjusted to physiologic pH by adding Tris Buffer, pH8.0. The purified antibodies were subsequently subjected to buffer exchange and protein concentration using Amicon Ultra-15 Centrifugal Filter Unit (Life Technologies, Catalog# UFC900324) in PBS buffer. Antibody concentration was determined by BCA Protein Assay. SDS-PAGE and Coomassie-staining were carried out to test the antibody purity. The purified protein was aliquoted and stored at - 80°C for long time storage or kept at 4°C for immediate use.

The integrity of the antibody was validated by SDS-PAGE followed by Coomassie staining under non-reducing vs reducing conditions; under non-reducing condition, one dominating band around 150 kDa, whereas under reducing conditions, two bands were observed, 50 kDa and 25 kDa.

Antibodies specific for other CLDN family members having sequence similarity (Figure 5), namely, CLDN3, CLDN4, and CLDN9 were produced in essentially the same manner, except that the antibody sequences contained in the plasmids were antibody sequences specific to CLDN3, CLDN4, or CLDN9.

### EXAMPLE 4

This example demonstrates the characterization of cell lines with high endogenous CLDN6 expression.

A panel of cancer cell lines was analyzed for their endogenous expression of CLDN6 by FACS and Western blot. Briefly, the binding of antibodies to targets were validated by FACS using cells overexpressing CLDN6 (e.g., HEK293T cells overexpressing CLDN6, described in Example 2), and cell lines that endogenously express CLDN6 at high or low levels, as determined in Example 1. The CLDN6-expressing cells were incubated with reference or control antibodies (described in Example 3) for 30 min on ice, and, after washing, incubated with Alexa Fluor@ 647 conjugated Goat anti-mouselgG (minimal x-reactivity) antibody, Biolegend cat#405322 for 30 min on ice. Fluorescence was read by a BD Biosciences Accuri^{™} flow cytometer (San Jose, CA).

Western blots were carried out on nitrocellulose with reference and control antibodies. Briefly, samples from cell lysates were boiled to denature protein content. SDS-PAGE (SDS-polyacrylamide gel electrophoresis) was used to separate the denatured proteins by the length of the polypeptide. Separated proteins were then transferred from the acrylamide gel to a nitrocellulose membrane. A 2% Bovine Serum Albumin (BSA) solution was used to block the membrane, minimizing non-specific antibody binding. The membrane was incubated with reference or control antibodies. The membrane was stained with a horseradish peroxidase (HRP)-conjugated secondary antibody that recognizes the reference or control antibodies and detection of secondary antibody was via chemiluminescence.

The overexpressed lines were used to validate the control and reference antibodies, and, once validated, the control and reference antibodies were used to characterize the endogenous cell lines. The cells overexpressing CLDN6 were included as positive controls in these assays.

The FACS assays showed that four ovarian cancer cell lines, in addition to an endometrial cancer cell line, bladder cancer cell line, lung cancer cell line, and upper GI cancer cell line, express CLDN6 on the surface at high levels. The high levels of CLDN6 expression was also detected by Western blot. An additional two ovarian cancer cell lines, an additional liver cancer cell line, additional lung cancer cell line, and additional upper GI cancer cell line were shown to express CLDN6 to a moderate level on the surface, as detected by Western blot. Endometrial tumor cells and bladder tumor cells also expressed high levels of CLDN6 as xenografts *in vivo.* Endogenous expression levels of CLDN6 by the tested cancer cells lines are summarized in Table 2.

**TABLE 2**

| **Cell_Line_Na me** | **Primary Histolog y** | **CLDN6 RNAS eq** | **Group** | **Surface Expressi on by FACS** | **Protein Expressi on by WB (Cell Lines)** | **Protein Expressi on by WB (xenogra ft)** | **Tumorige nic Sub Q** | **Tumorige nic IP (Nude)** | **Tumorige nic IP (SCID)** |
|---|---|---|---|---|---|---|---|---|---|
| OVCA429 | Ovarian | 393.99 | Pos Con | ++++ | ++++ | | No | Slow | TBD |
| ARK2 | Endometr ial | 335.06 | Pos Con | ++++ | ++++ | +++ | Yes | | |
| OAW28 | Ovarian | 273.78 | Pos Con | ++++ | ++++ | | No | TBD | TBD |
| UMUC-4 | Bladder | 242.93 | Pos Con | ++++ | ++++ | ++ | Yes | Slow | TBD |
| PEO14 | Ovarian | 196.63 | Pos Con | ++++ | +++ | | No | TBD | TBD |
| OV177 | Ovarian | 195.53 | Pos Con | ++++ | + | | No | TBD | TBD |
| H1693 | Lung | 184.12 | Pos Con | ++++ | +++ | | TBD | | |
| MKN7 | Upper GI | 118.6 | Pos Con | +++ | ++ | | TBD | | |
| OV-90 | Ovarian | 108.23 | Pos Con | ++ | ++ | + | Yes | | |
| HUH-7 | Liver | 93.52 | Pos Con | ++ | + | | Yes | | |
| JHOS-4 | Ovarian | 69.3 | Pos Con | ++ | + | | No | TBD | TBD |
| H1435 | Lung | 42.8 | Pos Con | ++ | +/- | | TBD | | |
| NUGC 3 | Upper GI | 41.11 | Pos Con | ++ | ++ | + | Yes | | |
| RMG-1 | Ovarian | 0.63 | Neg Con | - | - | - | Yes | TBD | TBD |
| COLO704 | Ovarian | 0.44 | Neg Con | - | - | - | Yes | TBD | TBD |
| MCF-7 | Breast | 0 | Neg Con | - | - | - | Yes | | |
| LS513 | Colon | 0 | Neg Con | - | - | - | Yes | | |
| M202 | Melanom a | 0 | Neg Con | - | - | - | Yes | | |
| M275 | Melanom a | 0 | Neg Con | - | - | - | Yes | | |
| KOC-7C | Ovarian | 0 | Neg Con | - | - | - | Yes | Yes | TBD |

### EXAMPLE 5

This example demonstrates the immunization of mice for the production of CLDN6 specific antibodies.

CLDN6-specific antibodies were produced by immunizing Balb/c and CD1 mice with a mixture of three different peptide immunogens following techniques of the Fred Hutchinson Cancer Research Center. The three peptides spanned the second loop in the CLDN6 extracellular domain (i.e., EL2). The peptides include the full length of EL2, a peptide spanning the first (N-terminal) half of EL2, and a peptide spanning the second (C-terminal) half of EL2. Table 3 provides the sequences of the three peptides.

**TABLE 3**

| Peptide Immunogen | EL2 | SEQ ID NO: |
|---|---|---|
| Ac-CWTAHAIIRDFYNPLVAEAQKREL-amide | Full length EL2 | 2 |
| Ac-CTAHAIIRDFYNPL-amide | N-terminal half | 3 |
| Ac-LVAEAQKRELGC-amide | C-terminal half | 4 |

Mice also were immunized with 3T3 cells overexpressing full length CLDN6 using a plasmid comprising a human CLDN6-myc-DDK expression vector.

Splenocytes were harvested from the immunized mice and fused with myeloma lines by BTX Electrofusion (BTX, Holliston, MA) to generate hydridomas. 7680 primary hybridoma cultures were generated and cultured in 384-well plates. The ability of the antibodies to bind peptide was assessed by bead array using beads expressing the three different peptides targets. 1920 potential positive antibodies were re-arrayed into 96 well plates further screened by flow cytometry against endogenous and artificial cell line models.

Positive hybridoma supernatants were then counter-screened by flow cytometry against endogenous and artificial models of proteins that have sequence similarity to the target region (e.g., other CLDN proteins). From the secondary screen and counterscreen, ~20 CLDN6-specfic antibodies were chosen for additional study. These antibodies were subcloned and the variable heavy and light chain sequences were determined. See Table B and sequence listing.

CLDN6 antibodies were formatted as full-length IgG antibodies using ExpiCHO^{™} expression. The heavy and light chain variable regions of the antibodies were cloned into an antibody expression vector which was engineered in the lab based on a pcDNA^{™}3.4-TOPO^{®} vector (Catalog Number: A14697, ThermoFisher Scientific, USA) and transfected into CHO cells by (According to protocol provided in the kit (ExpiCHO^{™} Expression System, Catalog Number: A29133, ThermoFisher Scientific, USA)). Antibodies were purified and cell surface binding of the antibodies to CLDN6 and the antibody IC50 were determined by FACS in which CLDN6 antibodies were directly conjugated with Alexa Fluor@ 647 NHS Ester (Succinimidyl Ester), Cat# A20106 (ThermoFisher Scientific) following the manufacturer's protocol. CLDN6 antibodies were tested from 0.32nM to 1000 nM (serial dilution 1:5, 6 points) in a 50µl volume with 150,000 cells system.

CLDN6-expressing cells were used in FACS assays to determine the CLDN6 antibody's ability to bind to CLDN6 on the surface of cells and to cross-react with other CLDN family members. HEK293 T cells engineered to express human CLDN6 fused to GFP, mouse CLDN6 fused to GFP, CLDN9-GFP, CLDN4-GFP, or CLDN3-GFP, or GFP alone (without CLDN6) were used as artificial models of CLDN6 expression. ARK2, OVCA429, LS513 and MCF7 cells were used as endogenous models of CLDN6 expression, as well as models for CLDN3/4 expression.

For each type of cell tested and for each mAb, cells were detached from the surface of the culture flasks by EDTA (instead of trypsin) in order to protect the cell surface proteins. The detached cells were then incubated with Alexa Fluor^{®}-labeled CLDN6 mAbs for 30 min in the dark on ice at a pre-determined concentration. The CLDN6 mAbs were directly labeled with Alexa Fluor@ 647 NHS Ester (Succinimidyl Ester). After washing, the cells were read by a BD Accuri^{™} Flow Cytometer C6 to detect antibody-antigen protein binding in channel FL4H. Each antibody was tested at varied concentrations to establish a dose-fluorescence curve. The EC50/IC50 of the antibodies (the concentration of the antibody at which half the max value were calculated based on the values of FL4H (gated in viable singlet cells) using the Very Simple IC50 Tool kit available online which allows biological dose-response data to be plotted and fitted to curve types to give the EC50/IC50. The max value was the lowest concentration of the antibody at which fluorescence maxes out. The antibodies were also screened for their ability to cross-react with other CLDN proteins such as CLDN9, CLDN3, and CLDN4. These values were used to determine each antibody's relative affinity among the set of antibodies tested. Cross-reactivity data were obtained using a similar methodology, but with cells having a different expression profile for CLDN6, CLDN3, CLDN4 and CLDN9.

Relative affinity data and cross-reactivity data as determined in this manner are set out in Tables 4 and 5.

**TABLE 4**

| **AB#** | **Rank** | **HEK293T-CLDN6-mGFP** | **HEK293T-CLDN6-mGFP** | **HEK293T CLDN9-mGFP** | **HEK293T CLDN4-mGFP** | **HEK293T CLDN3-mGFP** | **HEK293T-mGFP** |
|---|---|---|---|---|---|---|---|
| | | **Artificial CLDN6+** | **Artificial CLDN6+ (mouse)** | **Artificial CLDN9+** | **Artificial CLDN4+** | **Artificial CLDN3+** | **Parental** |
| 1 | 1 | 109 | 72 | 2765 | 5000 | 5000 | 5000 |
| 2 | 2 | 182 | 5000 | 5000 | 5000 | 5000 | 5000 |
| 3 | 3 | 604 | 2487 | 5000 | 5000 | 5000 | 5000 |
| 4 | 4 | 17 | 5000 | 1860 | 739 | 5000 | 5000 |
| 5 | 5 | 10 | 11 | 25 | 5000 | 5000 | 5000 |
| 6 | 6 | 1427 | 2222 | 2769 | 26 | 5000 | 5000 |
| 7 | 7 | 579 | 1548 | 1938 | 2088 | 5000 | 5000 |
| 8 | 8 | 1918 | 5000 | 2769 | 5000 | 5000 | 5000 |
| 9 | 9 | 2671 | 5000 | 5000 | 5000 | 5000 | 5000 |
| 10 | 10 | 2757 | 5000 | 246 | 5000 | 5000 | 5000 |
| 11 | 11 | 1696.29 | 2375 | 5000 | 2238 | 5000 | 5000 |
| 12 | Neg | 5000 | 5000 | 2468 | 2087 | 5000 | 5000 |
| 13 | Neg | 5000 | 5000 | 5000 | 5000 | 5000 | 5000 |
| 14 | Neg | 5000 | 5000 | 5000 | 5000 | 5000 | 5000 |
| 16 | Neg | 5000 | 5000 | 5000 | 5000 | 5000 | 5000 |
| 17 | Neg | 2,645.95 | 5000 | 5000 | 5000 | 5000 | 5000 |
| 18 | Neg | 5000 | 5000 | 5000 | 5000 | 5000 | 5000 |
| 15 | Neg | 5000 | 5000 | 5000 | 5000 | 5000 | 5000 |
| 19 | Neg | 5000 | 5000 | 5000 | 5000 | 5000 | 5000 |
| Reference Ab2 | 0 | 122 | 46 | 5000 | 5000 | 5000 | 5000 |
| Reference Ab1 | 0 | 57 | 651 | 2943 | 5000 | 5000 | 5000 |

| | | | | | | | |
|---|---|---|---|---|---|---|---|
| AB# corresponds to AB# listed in Tables A and B. | | | | | | | |

**TABLE 5**

| **AB#** | **ARK2** | **OVCA429** | **LS513** | **MCF7** |
|---|---|---|---|---|
| | **Endogenous CLDN6+** | **Endogenous CLDN6+** | **Endogenous CLDN3/4+** | **Endogenous CLDN3/4+** |
| 1 | 105 | 84 | 5000 | 5000 |
| 2 | 1365 | 912 | 5000 | 5000 |
| 3 | 1653 | 1096 | 5000 | 5000 |
| 4 | 48 | 168 | 730 | 653 |
| 5 | 8 | 5 | 781 | 1485 |
| 6 | 765 | 394 | 133 | 1135 |
| 7 | 353 | 531 | 635 | 979 |
| 8 | 1136 | 1090 | 1126 | 1063 |
| 9 | 1140 | 1165 | 1040 | 5000 |
| 10 | 2143 | 2473 | 1947 | 5000 |
| 11 | 362.81 | 834.65 | 492.567 | 427.031 |
| 12 | 1297 | 2294 | 2266 | 5000 |
| 13 | 1147 | 2287 | 1594 | 5000 |
| 14 | 1304 | 1661 | 1161 | 546 |
| 16 | 5000 | 5000 | 5000 | 5000 |
| 17 | 5000 | 5000 | 5000 | 5000 |
| 18 | 1,354.80 | 2,435.18 | 5000 | 5000 |
| 15 | 5000 | 5000 | 5000 | 5000 |
| 19 | 5000 | 5000 | 5000 | 5000 |
| Reference Ab2 | 1115 | 1283 | 2222 | 5000 |
| Reference Ab1 | 162 | 62 | 5000 | 5000 |

| | | | | |
|---|---|---|---|---|
| AB# corresponds to AB# listed in Tables A and B. | | | | |

### EXAMPLE 6

This example demonstrates the characterization of chimeric mouse IgG mAbs.

Soft agar 3D proliferation assays and xenograft binding assays were carried out to further characterize mAbs described in Example 5. Briefly, a 250 µL top layer mixture containing 10,000 cells in 0.6% SeaPlaque agarose in 1x RPMI medium was plated on top of a solidified 250uL bottom layer of 0.6% SeaPlaque agarose in 1x RPMI medium for each well of a 48-well plate. A 250uL liquid feeder layer containing 1x RPMI medium was placed above the solidified top layer. All three layers of the soft agar assay were prepared with or without Trastuzumab, Cldn6 mAbs, or mouse IgG2a control starting at 150ng/mL (1uM) and ending at 1.5ng/mL, diluting 1:10. Each test condition was performed in duplicates. Cells were allowed to form colonies for 3 weeks before staining with 0.05% neutral red, and imaged on the EVOS XL inverted light microscope. Cell lines with a ≥20% decrease in colony number in treated versus control were considered sensitive.

As shown in Table 6, many of cell lines exhibited a decrease in colony number when treated with the indicated antibody.

**TABLE 6**

| | **Activity in vitro 3D** |
|---|---|
| Reference Ab1** | 70% |
| Reference Ab2** | 50% |
| AB1 | 50% |
| AB2 | 50% |
| AB3 | 50% |
| AB4 | 70% |
| AB5 | 70% |
| AB6 | 70% |
| AB7 | 50% |
| AP8 | 0% |
| AB9 | 0% |
| AB10 | 50% |
| AB11 | 50% |
| AB12 | 0% |
| AB13 | 50% |
| AB14 | 0% |
| AB19 | 50% |
| AB16 | 25% |
| AB17 | 0% |
| AB18 | 0% |
| AB19 | 0% |

*In vivo* binding studies were carried out in xenograft mice injected with human cancer cell lines. Briefly, xenograft models of human cancer cell lines were established in six-week-old CD-1 athymic nude mice (Charles River Laboratories). The following conditions were followed for subcutaneous injection of each cell line: ARK2 0.75 × 10⁷, UMUC4 1.0 × 10⁷, OV90 1.0 × 10⁷ and M202 0.5 × 10⁷ cells all with 50% matrigel (BD Biosciences). Sufficient numbers of mice were injected to achieve 8 mice per treatment arm. When tumors reached an average size of 150 to 300 mm³, mice were randomized into treatment groups. For treatment, each therapeutic antibody (AB3, AB2, Reference Ab1, Reference Ab2, Reference Ab 3 (trastuzumab) and non-targeting IgG2-control were diluted in sterile saline to a working concentration of 1mg/ml for intravenous tail vein (IV) injection. For the M202 study, trametinib (DMSO-solvate, MedChem Express) was dosed at 1.0mg/kg (10% Cremaphor, 10% PEG400) by PO on a 5 days on, 2 days off schedule for the first weekly cycle, followed by reduction to 0.5mg/kg for the remaining two weeks of dosing. Tumor xenografts were measured with calipers thrice a week, and tumor volume in mm³ was determined by multiplying height × width × length. Mice were treated for 2-7 weeks. At the end of study, animals were euthanized and tumor tissue was excised and divided to be stored as snap-frozen or formalin fixed paraffin embedded (FFPE) tissue for biomarker analysis. All animal work was carried out under a protocol approved by IACUC and the University of California at Los Angeles Animal Research Committee. Data was analyzed using StudyLog software from StudyDirector (San Francisco, CA). Results are presented as mean volumes for each group. Error bars represent the standard error (SE) of the mean.

The results of the xenograft assays are shown in Figures 6-10. As shown in Figures 6A and 6B, each of AB2 and AB3 caused a substantial mean change in tumor volume at Day 14, relative to control IgG2 antibody, in mice bearing endometrial tumors. As shown in Figures 7A and 7B, AB3 caused a substantial mean change in tumor volume at Day 35, relative to control IgG2 antibody, in mice bearing bladder tumors. Figures 8A and 8B show that each of AB2 and AB3 caused a substantial mean change in tumor volume at Day 20, relative to control IgG2 antibody, in mice bearing ovarian tumors. Figures 9A and 9B demonstrates that AB3 functions in a CLDN-specific manner, since the model used in Figures 9A and 9B did not express any of CLDN6, CLDN3, CLDN4, and CLDN9 and was, therefore, used as a negative control. The data of Figures 9A and 9B also suggests that AB3 has less off-target activity than Reference Ab1 and Reference Ab2. Figure 10A summarizes the results of Figures 6-9. As shown in Figure 10A, AB3 significantly reduced tumor growth in mice bearing endometrial, bladder, and ovarian tumors, each of which express CLDN6, but did not reduce tumor growth in mice bearing melanoma tumors, which did not express CLDN6 (Figure 10B). As shown in Figure 11, the mean chain in mouse body weight during treatment did not substantially change, suggesting the safety of the treatment.

A second set of experiments were carried out in xenograft models of a human ovarian cancer cell line, OV90. Mice were injected with one of 10 mAbs described in Example 5 or with a control antibody (mouse IgG2a antibody, reference CLDN6 ab) or with a PBS vehicle control. There were 8 mice per group and each animal was intravenously injected with 10 mg/kg antibody every 4 days. As shown in Figures 12A and 12B, several antibodies described in Example 5 reduced tumor volume in mice bearing ovarian tumors. Among the best performers were AB3, AB4, AB7, and AB10, though all antibodies tested reduced tumor volume, relative to vehicle control. As shown in Figure 13, the body weight of the animals treated with AB3, AB4, AB7, or AB10 did not significantly change, suggesting their safety.

### EXAMPLE 7

This example demonstrates the further characterization of chimeric mouse IgG mAbs.

Internalization quantification assays were carried out. Briefly, the study of CLDN6 protein internalization triggered by Reference Ab1, AB3, or AB4 binding was performed with a positive control, ubiquitously expressed Transferrin Receptor (TfR) which is a known cell surface receptor that is internalized after antibody binding.

TfR and CLDN6 antibodies were labeled with Texas Red^{™}-X, Succinimidyl Ester, mixed isomers, cat# T6134 (ThermoFisher Scientific). Cells were seeded in a µ-Slide 8 Well chamber (Cat# 80826, ibidi Cells In Focus Inc.) one day before the antibody treatment to allow cells to attach and grow. Cells were incubated with labeled antibodies for 30 min in dark on ice. Then the chamber containing CLDN6 or TfR labeled cells were read by Echo lab fluorescence microscope to collect images before internalization. The chamber was then incubated at 37°C for 40min to allow internalization process and images were collected again by the Echo lab fluorescence microscope. AB3 and AB4 caused a greater degree of CLDN6 internalization, compared to that caused by Reference Ab1 (Data not shown).

### EXAMPLE 8

This example demonstrates the further characterization of chimeric mouse IgG mAbs.

Two-dimensional (2D) proliferation assays were carried out with select antibodies described in Example 5 as follows: Cells were seeded in duplicate at 5,000 to 20,000 cells per well in a 24-well plate. On the following day, cells were treated with six 1-5 dilutions of mAb (starting at 100 nM of either Trastuzumab, Cldn6 mAbs, or mouse IgG2A control) and a fixed concentration of 1ng/µL Monomethyl auristatin E (MMAE) -conjugated anti-mouse secondary (Moradec, LLC) to generate dose response curves. Untreated wells of cells were quantified on Day 1, the day of antibody treatment, and later on Day 6 to determine the range of cell growth. Wells treated with mAbs were quantified on Day 6 and a growth in each treatment condition was determined as a normalized percent ratio against the growth of untreated cells. Quantification was performed on the Z1 Particle Counter (Beckman Coulter, Inc).

The results are shown in Figure 14. AB2, AB3, AB4, and AB5 demonstrated the greatest efficacy at inhibiting proliferation. The IC50 for each of these antibodies was between 0.1 nM and 1 nM. Each of AB7, AB10, AB11, and AB15 also demonstrated the ability to inhibit proliferation in this assay, though to a lesser extent than AB2, AB3, AB4, and AB5.

### EXAMPLE 9

This example demonstrates the humanization of antibodies of the present disclosures.

A subset of antibodies listed in Table A were selected for humanization analysis. The heavy chain variable (VH) and light chain variable (VL) sequences of AB1, AB3, AB4, AB9, AB11, and AB18 antibodies were compared to a library of known human germline sequences from human VH genes and human VLkappa genes (IMGT^{®} the international ImMunoGeneTics information system^{®} www.imgt.org; founder and director: Marie-Paule Lefranc, Montpellier, France).; the databases used were IMGT human VH genes (F+ORF, 273 germline sequences) and IMGT human VLkappa genes (F+ORF, 74 germline sequences). The acceptor human germline was chosen from those closest in sequence to the parental antibody.

Table 7 provides, for each VH and VL of each antibody, information on the human germline sequences chosen as the acceptor sequence and the human heavy chain joining region (J gene) chosen. The joining region (J gene) was chosen from human joining region sequences compiled at *IMGT^{®} the international ImMunoGeneTics information system^{®}* www.imgt.org *founder and director: Marie-Paule Lefranc, Montpellier, France).*

**TABLE 7**

| | Human HC germline | Human HC joining region (J gene) | Human LC germline | Human LC joining region (J gene) |
|---|---|---|---|---|
| AB1 | IGHV1-46(allele 1) | IGHJ4(allele 1) | IGLV1-39(allele 1) | IGKJ2(allele 1) |
| AB3 | IGHV3-23(allele 1) | IGHJ4(allele 1) | IGLV1-39(allele 1) | IGKJ2(allele 1) |
| AB4 | IGHV1-46(allele 1) | IGHJ4(allele 1) | IGLV2-30(allele 1) | IGKJ2(allele 1) |
| AB9 | IGHV1-46(allele 1) | IGHJ4(allele 1) | IGLV1-39(allele 1) | IGKJ2(allele 1) |
| AB11 | IGHV3-48(allele 1) | IGHJ4(allele 1) | IGLV4-1(allele 1) | IGKJ2(allele 1) |
| AB18 | IGHV1-46(allele 1) | IGHJ4(allele 1) | IGLV4-1(allele 1) | IGKJ2(allele 1) |

CDRs were defined according to the AbM definition (see the website of Dr. Andrew C. R. Martin www.bioinf.org.uk/abs/ for a table comparing CDR definitions).

Alteration of human germline framework (i.e., non-CDR residues in VH and VL) positions to corresponding parental murine sequence might be required to optimize binding of the humanized antibody. The sequences for versions of humanized antibodies are provided as SEQ ID NOs: 376-421.

For AB1, each of Asn52 (sequential numbering) of CDR2 of the HC and Asn54 of CDR2 in the LC was determined to have a low potential for deamidation based on sequence and conformation.

For AB3, each of Asn31 (sequential numbering) of CDR1 of the HC, Asn57 of CDR2 of the HC, Asn 28 of CDR1 of LC, and Asn50 of CDR2 of the LC was determined to have a low potential for deamidation based on sequence and conformation. Trp33 of CDR1 of the HC was determined as likely solvent-exposed and to have potential for oxidation, especially under stress conditions. In CDR3 of the HC it was determined that there is a free Cys106 within the CDR that could be problematic when manufacturing the antibody, as it is likely solvent-exposed. This Cys residue was recommended for alteration to Tyr, Ser or Ala. The maintenance of binding of these altered antibodies are tested. Ile53 of CDR2 of the LC was determined to be solvent-exposed and could lead to non-specific binding. It was suggested that this Ile residue be altered to Ser. The maintenance of binding of this altered antibody is tested.

For AB4, each of Asn52 (sequential numbering) of the CDR2 of HC and Asn58 of CDR2 of the LC was determined to have a low potential for deamidation based on sequence and conformation. The sequence DGNT in the CDR1 of the LC was determined as problematic, as it was determined to have a high potential for isoaspartate formation (at the sequence DG) as well as a potential for deamidation (at the sequence NT). This sequence was recommended for alteration.

For AB9, Asn33 (sequential numbering) in CDR1 of HC, and Asn52 and Asn59 in CDR2 of HC were determined to have a low potential for deamidation based on sequence and conformation. Asn54 was determined to have a medium potential for deamidation based on sequence and conformation. The NGG sequence in CDR2 of the HC was determined to have a high/ medium potential for deamidation followed by isoaspartate formation. Thus, it was recommended that this amino acid sequence is altered. A free Cys106 in CDR3 of the HC may be problematic when manufacturing the antibody, as it is determined as likely solvent-exposed. This Cys residue is suggested for alteration to Tyr, Ser or Ala. The maintenance of binding of these altered antibodies is tested. Arg28 in CDR1 of HC is not often found in human antibodies. This residue is altered to Thr and the maintenance of binding tested. For AB 9, Trp32 (sequential numbering) in CDR1 of the LC was determined as likely solvent-exposed and could undergo oxidation, especially under stress conditions. Leu24 of the same CDR is not often found in human antibodies. This residue is altered to Arg and maintenance of binding is tested.

For AB11, Asp54-Ser55 (sequential numbering) in CDR2 of the HC was determined as having a low potential for isoaspartate formation. Asn57 in CDR2 of the LC was determined as having a low potential for deamidation based on sequence and conformation.

For AB18, Asn33 and CDR-H2 Asn50 (sequential numbering) in CDR1 of the HC was determined as having a low potential for deamidation based on sequence and conformation. In CDR2 of the HC, the Asp-Pro (DP) sequence was determined as having a potential for undergoing fragmentation under acidic conditions. In the VL domain, Asn34 and Asn37 in the CDR1 of the LC was determined as having a low potential for deamidation based on sequence and conformation. In CDR3 of the LC, Trp56 was determined as likely solvent-exposed and could undergo oxidation, especially under stress conditions.

Table 8 shows a scheme for combining the humanized VH and VL. If none of the humanized versions is equivalent to the chimeric mAb. Preferred pairs are shown in bold underlined text.

**TABLE 8**

| Parental | Humanized Ab # | VH (SEQ ID NO:) | VL (SEQ ID NO:) |
|---|---|---|---|
| AB1 | AB1-1 | 376 | 380 |
| | AB1-2 | 377 | 380 |
| | **AB1-3** | 377 | **381** |
| | **AB1-4** | 377 | **382** |
| | **AB1-5** | 377 | **383** |
| | **AB1-6** | **378** | **381** |
| AB3 | AB3-1 | 384 | h21G5-L1 (optional) |
| | **AB3-2** | **385** | **388** |
| | **AB3-3** | **385** | **389** |
| | **AB3-4** | **386** | **388** |
| | **AB3-5** | **386** | **389** |
| | AB3-6 | 387 | 388 |
| | AB3-7 | 387 | 389 |
| AB4 | AB4-1 | 391 | 397 |
| | AB4-2 | 392 | 397 |
| | **AB4-3** | **392** | **398** |
| | **AB4-4** | **393** | **398** |
| | **AB4-5** | **394** | **398** |
| | AB4-6 | 395 | **398** |
| | AB4-7 | 396 | **398** |
| AB9 | AB9-1 | 403 | 409 |
| | AB9-2 | 404 | 409 |
| | **AB9-3** | **405** | **410** |
| | **AB9-4** | **405** | **411** |
| | AB9-5 | 406 | 410 |
| | AB9-6 | 406 | 411 |
| | AB9-7 | 407 | 410 |
| | AB9-8 | 407 | 411 |
| | AB9-9 | 408 | 410 |
| | AB9-10 | 408 | 411 |
| AB11 | AB11-1 | 412 | 414 |
| | **AB11-2** | **413** | **414** |
| | **AB11-3** | **413** | **415** |
| AB18 | AB18-1 | 416 | 420 |
| | AB18-2 | 417 | 420 |
| | AB18-3 | 417 | 420 |
| | AB18-4 | 417 | 421 |
| | AB18-5 | 418 | 420 |
| | AB18-6 | 418 | 421 |
| | AB18-7 | 419 | 420 |

Humanized antibodies described in Table 8 were constructed and expressed as essentially described in Example 5. FACS assays were carried out as essentially described in Example 5 to determine relative antigen binding strengths of the humanized antibodies. Two doses (1.5 µg or 0.3 µg) of the humanized antibodies were tested for binding to either human CLDN6 or murine CLDN6 which proteins were expressed by engineered 293T clones. The results of the assays are provided in Table 9.

**TABLE 9**

| **Humanized Ab Designation** | **Human CLDN6 (1.5µg Ab)** | **Human CLDN6 (0.3µg Ab)** | **Mouse CLDN6** |
|---|---|---|---|
| 2nd Ab only | 768.07 | 768.07 | 1061.72 |
| 64A-chim | 224297.77 | 124463.44 | 170906.13 |
| h64A | 233932.53 | 93415.06 | 188577.77 |
| SC27-108-chim | 320381.33 | 150854.98 | 284326.77 |
| AB1-Chim | 364416.49 | 311923.02 | 513665.18 |
| AB1-3 | 142182.2 | 141773.57 | 89292.21 |
| AB1-4 | 197142.08 | 101763.71 | 75860.6 |
| AB1-5 | 213233.57 | 137828.05 | 128498.45 |
| AB1-6 | 227152.34 | 119699.97 | 77561.34 |
| AB1-7 | 207009.91 | 79207.94 | 99740.72 |
| AB1-8 | 209971.67 | 98785.97 | 121717.49 |
| AB1-11 | 112923.25 | 64892.04 | 101386.62 |
| AB3-chim | 459373.03 | 267327.83 | 67927.47 |
| AB3-2 | 395813.97 | 309318.89 | 31741.02 |
| AB3-3 | 339510.79 | 250519.45 | 23038.44 |
| AB3-4 | 55845.14 | 11641.7 | 1521.79 |
| AB3-5 | 48550.83 | 13335.93 | 783.28 |
| AB3-7 | 169209.42 | 105881.93 | 56071.82 |
| AB3-8 | 151519.51 | 108419.64 | 21509.63 |
| AB4-chim | 326603.58 | 176289.63 | 3639.29 |
| AB4-3 | 47760.08 | 14309.78 | 671.08 |
| AB4-4 | 48975.26 | 14081.47 | 741.03 |
| AB4-5 | 43385.34 | 16765.44 | 2003.95 |
| AB4-6 | 29243.78 | 10841.68 | 14205.6 |
| AB4-7 | 33342.17 | 19215.28 | 6635.91 |
| AB4-8 | 77642.77 | 49310.15 | 4703.98 |
| AB4-9 | 56854.11 | 37231.13 | 1778.41 |
| AB4-10 | 77159.9 | 50809.2 | 2647.29 |
| AB4-11 | 61204.92 | 37798.69 | 1227.86 |
| AB4-12 | 86189.87 | 71121.12 | 1133.16 |
| AB18-chim | 135142.76 | 73775.39 | 57373.94 |
| AB18-3 | 58948.59 | 30357.27 | 2512.77 |
| AB18-4 | 54378.24 | 32424.7 | 4294.94 |
| AB18-5 | 51871.17 | 29448.66 | 8191.13 |
| AB18-6 | 58464.54 | 26883.47 | 3680.42 |
| AB9-chim | 72036.29 | 46694.71 | 6745.58 |
| AB9-3 | 24649.43 | 9504.6 | 3175.17 |
| AB9-4 | 34309.83 | 15331.51 | 1709.25 |
| AB11-chim | 3491.4 | 1671.35 | 5490.52 |
| AB11-2 | 2557.3 | 1289.17 | 7160.15 |
| AB11-3 | 2364.44 | 1009.7 | 5521.52 |

FACS assays were also carried out to determine relative antigen binding strengths of the humanized antibodies (at either 1.5 µg or 0.3 µg) for binding to CLDN6 as expressed by the indicated cancer cell lines. The results of the assays are provided in Table 10. 2nd Ab only was used as a negative control. 64A-chim, h64A, and SC27-108-chim were used as reference antibodies Corresponding Parental antibodies (antibodies prior to humanization) were used as controls and designated with "chim".

**TABLE 10**

| **Humanized Ab Designation** | **OVCA429 1.5µg** | **ARK2 1.5µg** | **ARK2 0.3µg** | **M202 1.5µg** | **M202 0.3µg** |
|---|---|---|---|---|---|
| 2nd Ab only | 885.77 | 1351.21 | 1351.21 | 638.83 | 638.83 |
| 64A-chim | 140378.55 | 80449.93 | 66518.52 | 1456.2 | 628.72 |
| h64A | 161168.87 | 69118.48 | 90346.66 | 3093.69 | 2467.63 |
| SC27-108-chim | 54987.78 | 25522.86 | 62410.85 | 2293.1 | 742.74 |
| AB1-Chim | 176836.16 | 51913.52 | 58762.01 | 23377.33 | 9426.64 |
| AB1-3 | 27535.69 | 10776.6 | 2829.25 | 1225.78 | 694.08 |
| AB1-4 | 34551.99 | 10039.46 | 3641.11 | 970.7 | 697.98 |
| AB1-5 | 54331.07 | 22353.67 | 7494.86 | 869.06 | 684.3 |
| AB1-6 | 29949.91 | 7743.19 | 2816.15 | 880.4 | 1423.53 |
| AB1-7 | 44711.89 | 11224.28 | 4443.54 | 876.24 | 611.15 |
| AB1-8 | 65974.83 | 17642.9 | 5805.35 | 583.54 | 1886.84 |
| AB1-11 | untested | 97659.62 | 53926.71 | 5079.26 | 978.76 |
| AB3-chim | 138190.1 | 29114.68 | 17055.33 | 1120.39 | 843.5 |
| AB3-2 | 85941.69 | 33618.84 | 14656.49 | 1483.17 | 727.98 |
| AB3-3 | 88679.61 | 54901.39 | 1156.25 | 801.56 | 749.95 |
| AB3-4 | 2955.8 | 1459.46 | 1315.36 | 839.75 | 563.41 |
| AB3-5 | 2951.18 | 2909.74 | 1275.51 | 713.46 | 611.66 |
| AB3-7 | untested | 90420.04 | 35995.73 | 7841 | 1742.34 |
| AB3-8 | untested | 23259.52 | 11856.48 | 1903.05 | 952.71 |
| AB4-chim | 64796.15 | 38447.83 | 19142.38 | 1343.31 | 1007.71 |
| AB4-3 | 1155.95 | 1522.98 | 1192.96 | 708.25 | 984.95 |
| AB4-4 | 1203.16 | 1398.82 | 1048.05 | 899.25 | 618.43 |
| AB4-5 | 1351.27 | 1198.66 | 966.62 | 1203.98 | 653.41 |
| AB4-6 | untested | 9842.52 | 3303.34 | 2520.88 | 1281.68 |
| AB4-7 | untested | 7563.59 | 2765.93 | 3322.65 | 1444.19 |
| AB4-8 | untested | 7313.46 | 3118.74 | 1495.74 | 974.83 |
| AB4-9 | untested | 7787.35 | 3058.96 | 1179.5 | 825.33 |
| AB4-10 | untested | 20227.28 | 7212.1 | 1392.45 | 546.63 |
| AB4-11 | untested | 9167.61 | 6392.04 | 895.77 | 549.19 |
| AB4-12 | untested | 24221.65 | 9690.02 | 2015.4 | 1506.93 |
| AB18-chim | 25521.37 | 9149.19 | 3442.7 | 1048.6 | 895.4 |
| AB18-3 | 2703.41 | 1210.4 | 1079.32 | 684.33 | 592.8 |
| AB18-4 | 3220.08 | 1379.11 | 1134.22 | 1231.31 | 762.51 |
| AB18-5 | 4273.83 | 1330.44 | 1313.83 | 1001.99 | 594.43 |
| AB18-6 | 6753.92 | 1936.48 | 1393.53 | 914.2 | 865.43 |
| AB9-chim | 3569.69 | 4487.55 | 3508.75 | 1331.44 | 680.03 |
| AB9-3 | 1257.3 | 1471.97 | 1139.54 | 1659.76 | 1111.31 |
| AB9-4 | 1110.93 | 1553.6 | 1116.78 | 1149.55 | 625.38 |
| AB11-chim | 2440.63 | 1260.97 | 1315.28 | 973.48 | 610.05 |
| AB11-2 | 2694.81 | 1937.51 | 1400.43 | 1766.07 | 872.68 |
| AB11-3 | 2201.32 | 1541.47 | 1320.38 | 1134.3 | 940.92 |

| | | | | | |
|---|---|---|---|---|---|
| "chim" is pre-humanized form of antibody. | | | | | |

Based on the *in vitro* antigen binding data, three humanized antibodies were selected for further testing and development. The antibodies were derived from AB1, AB3, and AB4.

*In vivo* binding studies of the humanized versions of AB1, AB3, and AB4 were carried out in xenograft mice injected with bladder cancer cell lines UMUC4, as essentially described in Example 6. Briefly, xenograft models of UMUC4 were established in six-week-old CD-1 athymic nude mice (Charles River Laboratories). After tumors reached an average size of 150 to 300 mm³, mice were randomized into treatment groups. Humanized antibodies were diluted in sterile saline to a working concentration of 1mg/ml for intravenous tail vein (IV) injection. Tumor xenografts were measured with calipers thrice a week, and tumor volume in mm³ was determined by multiplying height × width × length. Mice were treated for 2-7 weeks. At the end of study, animals were euthanized and tumor tissue was excised and divided to be stored as snap-frozen or formalin fixed paraffin embedded (FFPE) tissue for biomarker analysis.

The results of the xenograft assays are shown in Figures 15-21. Figure 15 shows the xenograft assay results for two versions of humanized AB3 (AB3-2 and AB3-4), wherein treatment involved 10 mg/kg administered Q4D. Controls included vehicle control (PBS), human IgG (10 mg/kg Q4D), and the murine version of AB3 (10 mg/kg Q4D). As shown in this figure, humanized AB3-4 demonstrated a decrease in tumor volume over the 35 day treatment period. Figure 16 shows the xenograft assay results for the same treatments as the experiments shown in Figure 15, except that two additional controls (64A MSE and a chimeric version thereof (64A-CHIM)) were carried out. As in Figure 15, Figure 16 shows a marked decrease in tumor volume upon treatment with humanized AB3-4.

Figure 17 shows the xenograft assay results for humanized AB1-5. Controls included vehicle control (PBS), human IgG (10 mg/kg Q4D), and the murine version of AB1 (10 mg/kg Q4D). As shown in Figure 17, mice treated with humanized AB1-5 demonstrated a marked decrease in tumor volume.

Figure 18 shows the xenograft assay results for humanized AB4-3. Controls included vehicle control (PBS), human IgG (10 mg/kg Q4D), and the murine version of AB4 (10 mg/kg Q4D). Mice treated with humanized AB4-3 did not demonstrate a marked decrease in tumor volume.

Figures 19-21 demonstrate the results of a xenograft assay wherein all 4 humanized antibodies of Figures 15-18 were tested. Mouse and Chimeric versions of reference CLDN6 antibodies were used as controls. As shown in Figure 19, mice treated with humanized AB3-4 and AB1-5 demonstrated decreases in tumor volume. Figure 20 demonstrates tumor volume over the course of time to Day 55. The body weights of mice in the assay are shown in Figure 21.

### EXAMPLE 10

An *in silico* analysis was carried out with different sequences of AB1, AB3, and AB4. In particular, the sequences for (a) the original parental clone, (b) the closest mouse germline sequence, (c) the closest human germline sequence, and (d) the humanized sequence, for each antibody, were aligned. Amino acids believed to have undergone affinity maturation are marked with an asterisk while amino acids that differ from amino acids at that position according to antibody database information are marked with a hashtag. CDRs for each sequence are boxed. Based on this analysis, several humanized antibodies will be made having a sequence listed in TABLE 10.

**TABLE 10**

| Parent Clone | Consensus Sequence HC | Consensus Sequence LC |
|---|---|---|
| AB1 | 422 | 423 |
| AB3 | 424 | 425 |
| AB4 | 426 | 427 |

Multiples antibodies having a sequence as defined by these consensus sequences are made as essentially described in Example 5 and tested *in vitro* for antigen binding via FACS (as essentially described in Example 5) and *in vivo* for the ability to decrease tumor volume in mice (as essentially described in Example 6).

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the disclosure (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted.

Recitation of ranges of values herein are merely intended to serve as a shorthand method of referring individually to each separate value falling within the range and each endpoint, unless otherwise indicated herein, and each separate value and endpoint is incorporated into the specification as if it were individually recited herein.

All methods described herein can be performed in any suitable order unless otherwise indicated herein or otherwise clearly contradicted by context. The use of any and all examples, or various language (e.g., "such as") provided herein, is intended merely to better illuminate the disclosure and does not pose a limitation on the scope of the disclosure unless otherwise claimed. No language in the specification should be construed as indicating any non-claimed element as essential to the practice of the disclosure.

Preferred embodiments of this disclosure are described herein, including the best mode known to the inventors for carrying out the disclosure. Variations of those preferred embodiments may become apparent to those of ordinary skill in the art upon reading the foregoing description. The inventors expect skilled artisans to employ such variations as appropriate, and the inventors intend for the disclosure to be practiced otherwise than as specifically described herein. Accordingly, this disclosure includes all modifications and equivalents of the subject matter recited in the claims appended hereto as permitted by applicable law. Moreover, any combination of the above-described elements in all possible variations thereof is encompassed by the disclosure unless otherwise indicated herein or otherwise clearly contradicted by context.

### The invention furthermore comprises the following items:

1. An antigen-binding protein that binds to a human Claudin6 (CLDN6) protein(SEQ ID NO: 200), wherein:
   a. the antigen-binding protein binds to Extracellular Loop 2 (EL2) of an extracellular domain (ECD) of CLDN6 and does not bind to Extracellular Loop 1 (EL1) of the ECD of CLDN6; or
   b. does not bind to any of Claudin3 (CLDN3), Claudin4 (CLDN4), and Claudin9 (CLDN9) and inhibits binding of a reference antibody to CLDN6 endogenously expressed by OVCA429 cells with less than about 1200 nM; or
   c. a combination thereof.
2. The antigen- binding protein of item 1, which binds to an epitope within the amino acid sequence of WTAHAIIRDFYNPLVAEAQKREL (SEQ ID NO: 2).
3. The antigen-binding protein of item 2, which binds to the amino acid sequence of TAHAIIRDFYNPL (SEQ ID NO: 3) or LVAEAQKREL (SEQ ID NO: 4) of CLDN 6.
4. The antigen-binding protein of any one of items 1 to 3, which does not bind to any one or more of Claudin3 (CLDN3), Claudin4 (CLDN4), and Claudin9 (CLDN9).
5. The antigen-binding protein of item 4, which does not bind to CLDN3.
6. The antigen-binding protein of item 5, which binds to CLDN6, CLDN4, and CLDN9.
7. The antigen-binding protein of item 5, which does not bind to CLDN9.
8. The antigen-binding protein of item 7, which binds to CLDN6 and CLDN4.
9. The antigen-binding protein of item 5, which does not bind to CLDN4.
10. The antigen-binding protein of item 9, which binds to CLDN6 and CLDN9.
11. The antigen-binding protein of any one of the previous items wherein the reference antibody comprises a light chain variable sequence of SEQ ID NO: 181 and a heavy chain variable sequence of SEQ ID NO: 182 or a light chain variable sequence of SEQ ID NO: 185 and a heavy chain variable sequence of SEQ ID NO: 186.
12. The antigen-binding protein of any one of the previous items comprising:
   a. a heavy chain CDR1 amino acid sequence set forth in Table A or a sequence selected from the group consisting of: SEQ ID NOs: 11, 17, 23, 29, 35, 41, 47, 53, 59, 65, 71, 77, 83, 89, 95, 101, 107, 113, 119, 125, and 131, or a variant sequence thereof which differs by only one or two amino acids or which has at least or about 70% sequence identity;
   b. a heavy chain CDR2 amino acid sequence set forth in Table A or a sequence selected from the group consisting of: SEQ ID NOs: 12, 18, 24, 30, 36, 42, 48, 54, 60, 66, 72, 78, 84, 90, 86, 102, 108, 114, 120, 126, and 132, or a variant sequence thereof which differs by only one or two amino acids or which has at least or about 70% sequence identity;
   c. a heavy chain CDR3 amino acid sequence set forth in Table A or a sequence selected from the group consisting of: SEQ ID NOs: 13, 19, 25, 31, 37, 43, 49, 55, 61, 67, 73, 79, 85, 91, 97, 103, 109, 115, 121, 127, and 133, or a variant sequence thereof which differs by only one or two amino acids or which has at least or about 70% sequence identity;
   d. a light chain CDR1 amino acid sequence set forth in Table A or a sequence selected from the group consisting of: SEQ ID NOs: 8, 14, 20, 32, 38, 44, 50, 56, 62, 68, 74, 80, 86, 92, 98, 104, 110, 116, 122, and 128, or a variant sequence thereof which differs by only one or two amino acids or which has at least or about 70% sequence identity;
   e. a light chain CDR2 amino acid sequence set forth in Table A or a sequence selected from the group consisting of: SEQ ID NOs: 9, 15, 21, 27, 33, 39, 45, 51, 57, 63, 69, 75, 81, 87, 93, 99, 105, 111, 117, 123, and 129, or a variant sequence thereof which differs by only one or two amino acids or which has at least or about 70% sequence identity;
   f. a light chain CDR3 amino acid sequence set forth in Table A or a sequence selected from the group consisting of: SEQ ID NOs: 10, 16, 22, 28, 34, 40, 46, 52, 58, 64, 70, 76, 82, 88, 94, 100, 106, 112, 118, 124, and 130, or a variant sequence thereof which differs by only one or two amino acids or which has at least or about 70% sequence identity;
   g. a combination of any two or more of (a)-(f).
13. The antigen-binding protein of item 12, wherein the variant sequence has at least about 80% or at least or about 85% sequence identity.
14. The antigen-binding protein of item 13, wherein the variant sequence has at least about 90% sequence identity or at least or about 95% sequence identity.
15. The antigen-binding protein of item 12, comprising a light chain CDR1 amino acid sequence, a light chain CDR2 amino acid sequence, and a light chain CDR3 amino acid sequence set forth in Table A and 1 or 2 of the heavy chain CDR amino acid sequences set forth in Table A.
16. The antigen-binding protein of item 12, comprising a heavy chain CDR1 amino acid sequence, a heavy chain CDR2 amino acid sequence, and a heavy chain CDR3 amino acid sequence set forth in Table A and 1 or 2 of the light chain CDR amino acid sequences set forth in Table A.
17. The antigen-binding protein of any one of items 12-16, comprising six CDR amino acid sequences selected from the group consisting of:
   a. SEQ ID NOs: 74-79;
   b. SEQ ID NOs: 50-55;
   c. SEQ ID NOs: 122-127;
   d. SEQ ID NOs: 26-31;
   e. SEQ ID NOs: 128-133;
   f. SEQ ID NOs: 38-43;
   g. SEQ ID NOs: 62-67;
   h. SEQ ID NOs: 80-85;
   i. SEQ ID NOs: 44-49;
   j. SEQ ID NOs: 86-91;
   k. SEQ ID NOs: 104-109;
   l. SEQ ID NOs: 56-61;
   m. SEQ ID NOs: 32-37;
   n. SEQ ID NOs: 110-115;
   o. SEQ ID NOs: 98-103;
   p. SEQ ID NOs: 92-97;
   q. SEQ ID NOs: 116-121;
   r. SEQ ID NOs: 8-13;
   s. SEQ ID NOs: 68-73;
   t. SEQ ID NOs: 14-19; and
   u. SEQ ID NOs: 20-25.
18. The antigen-binding protein of any one of items 12-19, comprising:
   a. a heavy chain variable region amino acid sequence set forth in in Table B or a sequence selected from the group consisting of: 135, 137, 139, 141, 143, 145, 147, 149, 151, 153, 155, 157, 159, 161, 163, 165, 167, 169, 171, 173, and 175, or a variant sequence thereof which differs by only one or two amino acids or which has at least or about 70% sequence identity; or
   b. a light chain variable region amino acid sequence set forth in in Table B or a sequence selected from the group consisting of: 134, 136, 138, 140, 142, 144, 146, 148, 150, 152, 154, 156, 158, 160, 162, 164, 166, 168, 170, 172, 174, and 176, or a variant sequence thereof which differs by only one or two amino acids or which has at least or about 70% sequence identity; or
   c. both (a) and (b).
19. The antigen-binding protein of item 18, wherein the variant sequence has at least about 80% or at least about 85% sequence identity.
20. The antigen-binding protein of item 19, wherein the variant sequence has at least about 90% or at least about 95% sequence identity.
21. The antigen-binding protein of item 18, comprising a pair of amino acid sequences selected from the group consisting of:
   a. SEQ ID NOs: 156 and 157;
   b. SEQ ID NOs: 148 and 149;
   c. SEQ ID NOs: 172 and 173;
   d. SEQ ID NOs: 140 and 141;
   e. SEQ ID NOs: 174 and 175;
   f. SEQ ID NOs: 144 and 145;
   g. SEQ ID NOs: 152 and 153;
   h. SEQ ID NOs: 158 and 159;
   i. SEQ ID NOs: 146 and 147;
   j. SEQ ID NOs: 160 and 161;
   k. SEQ ID NOs: 166 and 167;
   l. SEQ ID NOs: 150 and 151;
   m. SEQ ID NOs: 142 and 143;
   n. SEQ ID NOs: 168 and 169;
   o. SEQ ID NOs: 164 and 165;
   p. SEQ ID NOs: 162 and 163;
   q. SEQ ID NOs: 170 and 171;
   r. SEQ ID NOs: 134 and 135;
   s. SEQ ID NOs: 154 and 155;
   t. SEQ ID NOs: 136 and 137; and
   u. SEQ ID NOs: 138 and 139.
22. The antigen-binding protein of any one of the previous items which is an antibody.
23. The antigen-binding protein of item 22, which is a monoclonal antibody.
24. The antigen-binding protein of item 22 or 23, which is an IgG.
25. The antigen-binding protein of any one of the previous items , which inhibits at least about 50% colony growth in a soft agar 3D proliferation assays.
26. The antigen-binding protein of any one of the previous items , which inhibits tumor growth in xenograft mice injected with human cancer cells.
27. The antigen-binding protein of item 26, which inhibits tumor growth of in xenograft mice injected with ovarian cancer cells, melanoma cancer cells, bladder cancer cells, or endometrial cancer cells.
28. The antigen-binding protein of item 27, which inhibits at least 50% tumor growth in xenograft mice injected with ovarian cancer cells, bladder cancer cells, or endometrial cancer cells.
29. An antigen-binding protein comprising (a) an HC CDR1 amino acid sequence set forth in Table A or A1 or a sequence selected from the group consisting of: SEQ ID NOs: 11, 17, 23, 29, 35, 41, 47, 53, 59, 65, 71, 77, 83, 89, 95, 101, 107, 113, 119, 125, 131, 452, 455, 461, 465, and 472, or a variant sequence thereof which differs by only one or two amino acids or which has at least or about 70% sequence identity; (b) an HC CDR2 amino acid sequence set forth in Table A or A1 or a sequence selected from the group consisting of: SEQ ID NOs: 12, 18, 24, 30, 36, 42, 48, 54, 60, 66, 72, 78, 84, 90, 86, 102, 108, 114, 120, 126, 132, 475, 456, 462, 466, 468, and 473; or a variant sequence thereof which differs by only one or two amino acids or which has at least or about 70% sequence identity; (c) an HC CDR3 amino acid sequence set forth in Table A or A1 or a sequence selected from the group consisting of: SEQ ID NOs: 13, 19, 25, 31, 37, 43, 49, 55, 61, 67, 73, 79, 85, 91, 97, 103, 109, 115, 121, 127, 133, 453, 457, 463, 467, 469, and 474; or a variant sequence thereof which differs by only one or two amino acids or which has at least or about 70% sequence identity; (d) a LC CDR1 amino acid sequence set forth in Table A or A1 or a sequence selected from the group consisting of: SEQ ID NOs: 8, 14, 20, 32, 38, 44, 50, 56, 62, 68, 74, 80, 86, 92, 98, 104, 110, 116, 122, 128, 449, 476, 458, 464, and 470; or a variant sequence thereof which differs by only one or two amino acids or which has at least or about 70% sequence identity; (e) an LC CDR2 amino acid sequence set forth in Table A or A1 or a sequence selected from the group consisting of: SEQ ID NOs: 9, 15, 21, 27, 33, 39, 45, 51, 57, 63, 69, 75, 81, 87, 93, 99, 105, 111, 117, 123, 129, 450, 477, 459, and 471; or a variant sequence thereof which differs by only one or two amino acids or which has at least or about 70% sequence identity; (f) an LC CDR3 amino acid sequence set forth in Table A or A1 or a sequence selected from the group consisting of: SEQ ID NOs: 10, 16, 22, 28, 34, 40, 46, 52, 58, 64, 70, 76, 82, 88, 94, 100, 106, 112, 118, 124, 130, 451, 454, and 460, or a variant sequence thereof which differs by only one or two amino acids or which has at least or about 70% sequence identity; or (g) a combination of any two or more of (a)-(f).
30. An antigen-binding protein comprising six CDR amino acid sequences selected from the group consisting of:
   a. SEQ ID NOs: 74-79;
   b. SEQ ID NOs: 50-55;
   c. SEQ ID NOs: 122-127;
   d. SEQ ID NOs: 26-31;
   e. SEQ ID NOs: 128-133;
   f. SEQ ID NOs: 38-43;
   g. SEQ ID NOs: 62-67;
   h. SEQ ID NOs: 80-85;
   i. SEQ ID NOs: 44-49;
   j. SEQ ID NOs: 86-91;
   k. SEQ ID NOs: 104-109;
   l. SEQ ID NOs: 56-61;
   m. SEQ ID NOs: 32-37;
   n. SEQ ID NOs: 110-115;
   o. SEQ ID NOs: 98-103;
   p. SEQ ID NOs: 92-97;
   q. SEQ ID NOs: 116-121;
   r. SEQ ID NOs: 8-13;
   s. SEQ ID NOs: 68-73;
   t. SEQ ID NOs: 14-19;
   u. SEQ ID NOs: 20-25;
   v. SEQ ID NOs: 449-453 and 475;
   w. SEQ ID NOs: 476-477, 454-457;
   x. SEQ ID NOs: 458-463;
   y. SEQ ID NOs: 57, 58, 464-467;
   z. SEQ ID NOs: 68-71 and 468-469; and
   aa. SEQ ID NOs: 112, and 470-474.
31. An antigen-binding protein comprising:
   a. a heavy chain variable region amino acid sequence set forth in in Table B or a sequence selected from the group consisting of: SEQ ID NOs: 135, 137, 139, 141, 143, 145, 147, 149, 151, 153, 155, 157, 159, 161, 163, 165, 167, 169, 171, 173, and 175, or a variant sequence thereof which differs by only one or two amino acids or which has at least or about 70% sequence identity; or
   b. a light chain variable region amino acid sequence set forth in in Table B or a sequence selected from the group consisting of: SEQ ID NOs: 134, 136, 138, 140, 142, 144, 146, 148, 150, 152, 154, 156, 158, 160, 162, 164, 166, 168, 170, 172, 174, and 176, or a variant sequence thereof which differs by only one or two amino acids or which has at least or about 70% sequence identity; or
   c. both (a) and (b).
32. The antigen-binding protein of item 31, wherein the variant sequence has at least about 85% sequence identity or about 90% or about 95% sequence identity.
33. An antigen-binding protein comprising a pair of amino acid sequences selected from the group consisting of:
   a. SEQ ID NOs: 156 and 157;
   b. SEQ ID NOs: 148 and 149;
   c. SEQ ID NOs: 172 and 173;
   d. SEQ ID NOs: 140 and 141;
   e. SEQ ID NOs: 174 and 175;
   f. SEQ ID NOs: 144 and 145;
   g. SEQ ID NOs: 152 and 153;
   h. SEQ ID NOs: 158 and 159;
   i. SEQ ID NOs: 146 and 147;
   j. SEQ ID NOs: 160 and 161;
   k. SEQ ID NOs: 166 and 167;
   l. SEQ ID NOs: 150 and 151;
   m. SEQ ID NOs: 142 and 143;
   n. SEQ ID NOs: 168 and 169;
   o. SEQ ID NOs: 164 and 165;
   p. SEQ ID NOs: 162 and 163;
   q. SEQ ID NOs: 170 and 171;
   r. SEQ ID NOs: 134 and 135;
   s. SEQ ID NOs: 154 and 155;
   t. SEQ ID NOs: 136 and 137; and
   u. SEQ ID NOs: 138 and 139.
34. An antigen-binding protein comprising:
   a. a heavy chain variable region amino acid sequence set forth in Table C or a sequence selected from the group consisting of: SEQ ID NOs: 376-379, 384-387, 391-396, 403-408, 412-413, 416-419, and 422-427, or a variant sequence thereof which differs by only one or two amino acids or which has at least or about 70% sequence identity; or
   b. a light chain variable region amino acid sequence set forth in in Table C or a sequence selected from the group consisting of: SEQ ID NOs: 380-383, 388-390, 397-402, 409-411, 414-415, and 420-421, or a variant sequence thereof which differs by only one or two amino acids or which has at least or about 70% sequence identity; or
   c. both (a) and (b).
35. The antigen-binding protein of item 6, wherein the variant sequence has at least about 85% sequence identity.
36. The antigen-binding protein of item 7, wherein the variant sequence has at least about 90% or about 95% sequence identity.
37. An antigen-binding protein comprising a pair of amino acid sequences selected from the group consisting of:
   a. SEQ ID NOs: 376 and 380;
   b. SEQ ID NOs: 377 and 380;
   c. SEQ ID NOs: 377 and 381;
   d. SEQ ID NOs: 377 and 382;
   e. SEQ ID NOs: 377 and 383;
   f. SEQ ID NOs: 378 and 381;
   g. SEQ ID NOs: 378 and 382;
   h. SEQ ID NOs: 378 and 383;
   i. SEQ ID NOs: 379 and 381;
   j. SEQ ID NOs: 379 and 382;
   k. SEQ ID NOs: 379 and 383;
   l. SEQ ID NOs: 384 and 388;
   m. SEQ ID NOs: 385 and 388;
   n. SEQ ID NOs: 385 and 389;
   o. SEQ ID NOs: 386 and 388;
   p. SEQ ID NOs: 386 and 389;
   q. SEQ ID NOs: 387 and 389;
   r. SEQ ID NOs: 422 and 389;
   s. SEQ ID NOs: 391 and 397;
   t. SEQ ID NOs: 392 and 397;
   u. SEQ ID NO: 393 and 398;
   v. SEQ ID NO: 394 and 398;
   w. SEQ ID NO: 395 and 398;
   x. SEQ ID NO: 396 and 398;
   y. SEQ ID NO: 423 and 398;
   z. SEQ ID NO: 424 and 398;
   aa. SEQ ID NO: 425 and 398;
   bb. SEQ ID NO: 426 and 398
   cc. SEQ ID NO: 427 and 398;
   dd. SEQ ID NO: 403 and 409;
   ee. SEQ ID NO: 404 and 409;
   ff. SEQ ID NO: 405 and 410;
   gg. SEQ ID NO: 405 and 411;
   hh. SEQ ID NO: 406 and 410;
   ii. SEQ ID NO: 406 and 411;
   jj. SEQ ID NO: 407 and 410;
   kk. SEQ ID NO: 407 and 411;
   II. SEQ ID NO: 408 and 410;
   mm. SEQ ID NO: 408 and 411;
   nn. SEQ ID NO: 412 and 414;
   oo. SEQ ID NO: 413 and 414;
   pp. SEQ ID NO: 416 and 420;
   qq. SEQ ID NO: 417 and 420;
   rr. SEQ ID NO: 417 and 421;
   ss. SEQ ID NO: 418 and 420;
   tt. SEQ ID NO: 418 and 421;
   uu. SEQ ID NO: 419 and 420; and
   vv. SEQ ID NO: 419 and 421;
38. An antigen-binding protein comprising:
   (A) an HC CDR1 comprising the amino acid sequence of YTFTXYT, wherein X is T, V, D, or S (SEQ ID NO: 452), optionally, comprising the amino acid sequence of YTFTTYT (SEQ ID NO: 11);
   (B) an HC CDR2 comprising the amino acid sequence of IXPSSGYT, wherein X is Q, S, A, or N (SEQ ID NO: 475), optionally, comprising the amino acid sequence of INPSSGYT (SEQ ID NO: 12);
   (C) an HC CDR3 comprising the amino acid sequence of AXGDYYVAY, wherein X is N, Q, H, or D (SEQ ID NO: 453), optionally, comprising the amino acid sequence of ANGDYYVAY (SEQ ID NO:13);
   (D) an LC CDR1 comprising the amino acid sequence of SSVSSXY, wherein X is T, V, F, or D (SEQ ID NO: 449), optionally, comprising the amino acid sequence of SSVSSTY (SEQ ID NO: 8);
   (E) an LC CDR2 comprising the amino acid sequence of XTX, wherein X at position 1 is S, T, Q, or A and X at position 3 is S, T, D, or Q (SEQ ID NO: 450), optionally, comprising the amino acid sequence of STS (SEQ ID NO: 9); and
   (F) an LC CDR3 comprising the amino acid sequence of HXYXRSPLT, wherein X at position 2 is Q, H, or S and X at position 4 is H, Y, Q, or S (SEQ ID NO: 451), optionally, comprising the amino acid sequence of HQYHRSPLT (SEQ ID NO: 10).
39. An antigen-binding protein comprising:
   (A) an HC CDR1 comprising the amino acid sequence of FTFSXYX, wherein X at position 5 is N, S, R, Q, or A and X at position 7 is W, H, Y, F (SEQ ID NO: 455), optionally, comprising the amino acid sequence of FTFSNYW (SEQ ID NO: 23);
   (B) an HC CDR2 comprising the amino acid sequence of IRLKXDXYAT, wherein X at position 5 is S, N, A, or T and X at position 7 is Q, S, A, N (SEQ ID NO: 456), optionally, comprising the amino acid sequence of IRLKSDNYAT (SEQ ID NO: 24);
   (C) an HC CDR3 comprising the amino acid sequence of XDGPPSGX, wherein X at position 1 is N, D, or T and X at position 8 is S, T, A, C, or Y (SEQ ID NO: 457), optionally, comprising the amino acid sequence of NDGPPSGC (SEQ ID NO: 25);
   (D) an LC CDR1 comprising the amino acid sequence of EXIYSY, wherein X is Q, S, A, D, or N (SEQ ID NO: 476), optionally, comprising the amino acid sequence of ENIYSY (SEQ ID NO: 20);
   (E) an LC CDR2 comprising the amino acid sequence of XAK, wherein X at position 1 is Q, S, A, D, or N (SEQ ID NO: 477), optionally, comprising the amino acid sequence of NAK (SEQ ID NO: 21); and
   (F) an LC CDR3 comprising the amino acid sequence of QXHYXVPWT, wherein X at position 2 is H, Q, S, or T and X at position 5 is T, S, N, or G (SEQ ID NO: 454), optionally, comprising the amino acid sequence of QHHYTVPWT (SEQ ID NO: 22).
40. An antigen-binding protein comprising:
   (A) an HC CDR1 comprising the amino acid sequence of YTXTXYT, wherein X at position 3 is F, Y, S, or T and X at position 5 is S, T, Y, or D (SEQ ID NO: 461), optionally, comprising the amino acid sequence of YTFTSYT (SEQ ID NO: 29);
   (B) an HC CDR2 comprising the amino acid sequence of IXPSSXYT, wherein X at position 2 is Q, S, A, or N and X at position 6 is T, S, V, D, or G (SEQ ID NO: 462), optionally, comprising the amino acid sequence of INPSSTYT (SEQ ID NO: 30);
   (C) an HC CDR3 comprising the amino acid sequence of XRGEXGGFAY, wherein X at position 1 is S, A, T, or V and X at position 5 is L, V, or F (SEQ ID NO: 463), optionally, comprising the amino acid sequence of SRGELGGFAY (SEQ ID NO: 31);
   (D) an LC CDR1 comprising the amino acid sequence of QSLVHSXGXTY, wherein X at position 7 is D, N, E, Q, S, or A and X at position 9 is Q, S, A, D, or N (SEQ ID NO: 458), optionally, comprising the amino acid sequence of QSLVHSDGNTY (SEQ ID NO: 26);
   (E) an LC CDR2 comprising the amino acid sequence of XVX, wherein X at position 1 is K, Q, or R and X at position 3 is S, T, or V (SEQ ID NO: 459), optionally, comprising the amino acid sequence of KVS (SEQ ID NO: 27); and
   (F) an LC CDR3 comprising the amino acid sequence of SXXTHVPYT, wherein X at position 2 is Q, H, or T and X at position 3 is S, G, T, or D (SEQ ID NO: 460), optionally, comprising the amino acid sequence of SQSTHVPYT (SEQ ID NO: 28).
41. A conjugate comprising an antigen-binding protein of any one of the previous items .
42. A fusion protein comprising an antigen-binding protein of any one of the previous items .
43. A nucleic acid comprising a nucleotide sequence encoding an antigen binding protein of any one of the previous items , a conjugate of item 41, or a fusion protein of item 42.
44. A vector comprising the nucleic acid of item 43.
45. A host cell comprising the nucleic acid of item 43 or the vector of item 44.
46. A method of producing an antigen-binding protein that binds to a Claudin6 (CLDN6) protein, comprising (i) culturing the host cell of item 45 in a cell culture medium, wherein the host cell comprises a nucleic acid comprising a nucleotide sequence encoding an antigen binding protein of any one of the previous items , and (ii) harvesting the antigen-binding protein from the cell culture medium.
47. A method of producing a fusion protein comprising an antigen-binding protein that binds to a Claudin6 (CLDN6) protein, comprising (i) culturing the host cell of item 45 in a cell culture medium, wherein the host cell comprises a nucleic acid comprising a nucleotide sequence encoding a fusion protein of item 30, and (ii) harvesting the fusion protein from the cell culture medium.
48. A method of producing a pharmaceutical composition comprising combining an antigen-binding protein of any one of items 1 to 40, a conjugate of item 41, a fusion protein of item 42, a nucleic acid of item 43, a vector of item 44, a host cell of item 45, or a combination thereof, and a pharmaceutically acceptable carrier, diluent or excipient
49. A pharmaceutical composition comprising an antigen-binding protein of any one of items 1 to 40, a conjugate of item 1, a fusion protein of item 2, a nucleic acid of item 43, a vector of item 44, a host cell of item 45, or a combination thereof, and a pharmaceutically acceptable carrier, diluent or excipient.
50. A method of treating a subject with a CLDN6-expressing cancer comprising administering to the subject a pharmaceutical composition of item 49 in an amount effective to treat the cancer.
51. A method of inhibiting tumor growth in a subject, comprising administering to the subject a pharmaceutical composition of item 49 in an amount effective to inhibit tumor growth.
52. A method of reducing tumor size in a subject, comprising administering to the subject a pharmaceutical composition of item 49 in an amount effective to reduce tumor size.
53. A method of preventing the recurrence of cancer in a subject, comprising administering to the subject a pharmaceutical composition of item 49 in an amount effective to prevent the recurrence of cancer.
54. A method of treating cancer in a subject diagnosed to be a low over-expresser of CLDN6, comprising administering to the subject a pharmaceutical composition of item 49 in an amount effective to prevent the recurrence of cancer
55. The method of any one of items 50-54 wherein the administering induces apoptosis in tumor cells.
56. The method of any one of items 50-55 wherein the administering induces apoptosis in cells expressing CLDN6
57. A method of detecting Claudin6 (CLDN6) in a sample, comprising contacting the sample with an antigen-binding protein of any one of items 1 to 40, a conjugate of item 41, or a fusion protein of item 42, and assaying for an immunocomplex comprising the antigen-binding protein, conjugate or fusion protein bound to CLDN6.
58. A method of diagnosing a Claudin6 (CLDN6)-positive cancer in a subject, comprising contacting a biological sample comprising cells or tissue obtained from the subject with an antigen-binding protein of any one of items 1 to 40, a conjugate of item 41, or a fusion protein of item 42, and assaying for an immunocomplex comprising the antigen-binding protein, conjugate or fusion protein bound to CLDN6.

## Claims

1. An antigen-binding protein that binds to a human Claudin6 (CLDN6) protein (SEQ ID NO: 200) that comprises
(i) HC CDR1 comprising GFTFSNYW (SEQ ID NO: 23),
(ii) HC CDR2 comprising IRLKSDNYAT (SEQ ID NO: 24),
(iii) HC CDR3 comprising XDGPPSGX (SEQ ID NO: 457), wherein X at position 1 is N and X at position 8 is S, T, A, C, or Y,
(iv) LC CDR1 comprising ENIYSY (SEQ ID NO: 20),
(v) LC CDR2 comprising NAK (SEQ ID NO: 21), and
(vi) LC CDR3 comprising QHHYTVPWT (SEQ ID NO: 22)

2. The antigen-binding protein of claim 1, wherein X at position 8 is S.

3. The antigen-binding protein of claim 1, wherein X at position 8 is T.

4. The antigen-binding protein of claim 1, wherein X at position 8 is A.

5. The antigen-binding protein of claim 1, wherein X at position 8 is C.

6. The antigen-binding protein of claim 1, wherein X at position 8 is Y.

7. The antigen-binding protein of any one of claims 1 to 6, wherein the antigen-binding protein is an antibody-binding fragment, preferably selected from the group consisting of scFv, F(ab')₂, Fab, Fab' and Fv.

8. The antigen-binding protein of anyone of claims 1 to 6, wherein the antigen-binding protein is an antibody.

9. The antigen-binding protein of claim 8, wherein the antibody is
(i) a monoclonal antibody,
(ii) a human antibody, humanized antibody, or a chimeric antibody, or.
(iii) an IgG.

10. The antigen-binding protein of claim 9, wherein the IgG is selected from the group consisting of IgG1, IgG2, IgG3 and IgG4, and preferably is IgG1.

11. A conjugate comprising the antigen-binding protein of any one of claims 1 to 10 and a cytotoxic agent or a chemotherapeutic agent.

12. The conjugate protein of claim 11, further comprising a linker, preferably a cleavable linker.

13. The conjugate of claim 11 or 12, wherein the cytotoxic agent or a chemotherapeutic agent is conjugated to the antigen-binding protein via a linker.

14. The conjugate of claim 11 or 12, wherein the chemotherapeutic agent is an anti-mitotic agent that inhibits cell division by blocking tubulin polymerization, is preferably an auristatin, and is most preferably MMAE.

15. The conjugate of claim 11 or 12, wherein the chemotherapeutic agent is MMAE that is conjugated to the antigen-binding protein via a linker.

16. A pharmaceutical composition comprising:
(a) the antigen-binding protein of any one of claims 1 to 10 or the conjugate of any one of claims 11 to 15; and
(b) a pharmaceutically acceptable carrier, diluent, and/or excipient.

17. The antigen-binding protein of any one of claims 1 to 10 or the conjugate of any one of claims 11 to 15 for use in treating cancer.

18. An *in vitro* method for detecting Claudin 6 (CLDN6) in a sample comprising (a) contacting the antigen-binding protein of any one of claims 1 to 10 with the sample, and (b) assaying for an immunocomplex comprising the antibody or antigen-binding fragment thereof bound to CLDN6.

19. The antigen-binding protein of any one of claims 1 to 10 for use in a method of diagnosing *in vivo* a Claudin 6 (CLDN6)-positive cancer in a subject.
